# FASCICULE DE BREVET EUROPEEN

(11) **EP 1 383 925 B1**
(45) Date de publication et mention de la délivrance du brevet: **20.09.2006**
(21) Numéro de dépôt: 02738211.8
(22) Date de dépôt: 03.05.2002
(51) Int. Cl.: C12Q 1/68

(54) **PROCEDE DE MARQUAGE ET DE FRAGMENTATION D'ADN**
VERFAHREN ZUR MARKIERUNG UND FRAGMENTIERUNG VON DNS
METHOD FOR LABELLING AND FRAGMENTING DNA

(30) Priorité: 04.05.2001 FR 0106039
(43) Date de publication de la demande: 28.01.2004
(73) Titulaire: BIO MERIEUX, 69280 Marcy l'Etoile (FR); UNIVERSITE JOSEPH FOURIER (GRENOBLE 1), F-38041 Grenoble Cédex (FR); CENTRE NATIONAL DE LA RECHERCHE SCIENTIFIQUE, 75794 Paris Cedex 16 (FR)
(72) Inventeur: BOURGET, Cécile, F-74940 ANNECY LE VIEUX (FR); KOTERA, Mitsuharu, F-38240 MEYLAN (FR); LHOMME, Jean, F-38240 MEYLAN (FR); TREVISIOL, Emmanuelle, F-81500 MONTCABRIER (FR); LAAYOUN, Ali, F-69780 TOUSSIEU (FR); TORA, Christelle, F-38780 Oytier Saint Oblas (FR); SOTHIER, Isabelle, F-69730 GENAY (FR)
(74) Mandataire: Guerre, Dominique
(86) Numéro de dépôt international: PCT/FR2002/001542
(87) Numéro de publication internationale: WO 2002/090584

(56) Documents cités:
- WO-A-90/08838
- WO-A-99/65926
- SHIGA M ET AL: "FLUORESCENCE DETECTION OF DNA USING A NOVEL PEROXIDASE SUBSTRATE, 4-(4-HYDROXYPHENYLCARBAMOYL)BUTANOIC ACID" ANALYTICAL SCIENCES, JAPAN SOCIETY FOR ANALYTICAL CHEMISTRY, TOKYO,, JP, vol. 11, no. 4, août 1995 (1995-08), pages 591-595, XP008005960 ISSN: 0910-6340
- MAKRIGIORGOS G M ET AL: "FLUORESCENT LABELLING OF ABASIC SITES: A NOVEL METHODOLOGY TO DETECT CLOSELY-SPACED DAMAGE SITES IN DNA" INTERNATIONAL JOURNAL OF RADIATION BIOLOGY, TAYLOR AND FRANCIS, LONDON, GB, vol. 74, no. 1, 1998, pages 99-109, XP002925462 ISSN: 0955-3002
- SHIGA M ET AL: "SYNTHESIS OF A NOVEL BIOTIN DERIVATIVE THAT BEARS A DIAZO GROUP AS THE REACTIVE SITE" ANALYTICAL SCIENCES, JAPAN SOCIETY FOR ANALYTICAL CHEMISTRY, TOKYO,, JP, vol. 9, no. 4, août 1993 (1993-08), pages 553-556, XP008005959 ISSN: 0910-6340
- BIOPOLYMERS (NUCLEIC ACID SCIENCES), vol. 52, 1999, pages 65-83,
- JOURNAL OF BIOLOGICAL CHEMISTRY, vol. 266, no. 22, 5 août 1991 (1991-08-05), pages 14627-14635,

## Description

La présente invention concerne un procédé de fragmentation et de marquage d'ADN et un procédé pour la mise à disposition de sondes de détection d'un acide nucléique cible ainsi que les applications de ce procédé en particulier dans le domaine du diagnostic.

L'état de la technique montre que de nombreuses méthodes existent pour marquer des acides nucléiques.

Une première méthode consiste à fixer le marqueur sur la base, que celle-ci soit naturelle ou modifiée. Une deuxième méthode propose de fixer le marqueur sur le sucre, là encore qu'il soit naturel ou modifié. Une troisième méthode a pour objet la fixation du marqueur sur le phosphate.

Le marquage sur la base a été notamment utilisé dans l'approche de marquage des acides nucléiques par incorporation de nucléotides directement marqués.

Le marquage sur le sucre est souvent utilisé dans le cas des oligonucléotides préparées par synthèse chimique.

Le marquage sur le phosphate a été aussi utilisé pour introduire des bras fonctionnalisés et des marqueurs lors de la synthèse chimique des oligonucléotides.

En fait l'homme du métier, qui doit effectuer le marquage d'un nucléotide, ou d'un analogue de nucléotide ou d'un acide nucléique, est enclin à effectuer cette fixation sur la base ou sur le sucre qui lui offre plus de commodité et d'alternatives. C'est d'ailleurs ce qui ressort de l'étude de nombreux documents, tels que EP-A-0.329.198, EP-A-0.302.175, EP-A-0.097.373, EP-A-0.063.879, US-A-5,449,767, US-A-5,328,824, WO-A-93/16094, DE-A-39 10 151, EP-A-0.567.841 pour la base ou EP-A-0.286.898 pour le sucre.

La fixation du marqueur sur le phosphate est une technique plus complexe que la technique consistant à fonctionnaliser la base ou le sucre et a été bien moins utilisée notamment à cause de la faible réactivité du phosphate (voir par exemple Jencks W.P. et al J. Amer. Chem. Soc., 82, 1778-1785, 1960). De même dans la revue de O'Donnel et Mc Laughlin (« Reporter groups for the analysis of nucleic acid structure », p 216-243, dans « Bioorganic Chemistry: Nucleic Acids », Ed Hecht S.M., Oxford University Press, 1996) portant sur les méthodes d'introduction de sondes dans les fragments d'oligonucléotides, l'alkylation efficace du phosphodiester internucléotidique est considérée comme étant impossible.

Un deuxième problème se pose pour le marquage d'acides nucléiques spécialement pour les acides nucléiques de grande taille, c'est-à-dire de plus de cent (100) nucléotides, qui doivent s'hybrider avec des sondes nucléiques. Ce problème est lié à la gêne stérique ou au manque de spécificité entre l'acide nucléique et la sonde nucléique. Cela se traduit pas une perte de sensibilité en détection.

La gêne stérique peut être le fait, non seulement, de la longueur de l'acide nucléique, mais également de l'existence ou de la conservation de structures secondaires. La fragmentation permet de détruire ces structures et ainsi d'optimiser l'hybridation. Cette gêne stérique joue un rôle particulièrement important dans le cas de l'hybridation sur des surfaces contenant des sondes de capture à forte densité, par exemple les puces à ADN mises au point par la société Affymetrix ("Accessing Genetic Information with High-Density DNA arrays", M. Shee et al., Science, 274, 610-614. "Light-generated oligonucleotide arrays for rapide DNA sequence analysis", A. Caviani Pease et al., Proc. Natl. Acad. Sci. USA, 1994, 91, 5022-5026).

En ce qui concerne la fragmentation des acides nucléiques, de nombreuses méthodes sont décrites dans l'état de la technique.

Premièrement, la fragmentation peut être enzymatique, c'est-à-dire que la fragmentation des acides nucléiques peut être réalisée par des nucléases (DNases).

Deuxièmement, la fragmentation peut être chimique. Par exemple dans le cas de l'ADN, on peut effectuer la dépurination ou la dépyrimidination de l'ADN, qui est alors fragmenté en présence d'une base par un mécanisme dit de «β-élimination». La fragmentation de l'ADN peut être réalisé par des mécanismes d'oxydation, d'alkylation, d'addition de radicaux libres entre autres.

Enfin la fragmentation peut être physique, par exemple par sonication ou par voie photochimique.

La difficulté consiste en fait à associer ces deux étapes de fragmentation et de marquage.

La demande de brevet WO-A-99/65926 décrit un procédé de marquage d'un acide ribonucléique (ARN) de synthèse ou naturel qui consiste à fragmenter l'ARN et à marquer au niveau du phosphate terminal. Ce document décrit un certain nombre de fonctions réactives pouvant être utilisées pour le marquage en liaison avec la fragmentation. Ces fonctions permettent de marquer les ARN mais il faut associer une étape de fragmentation pour avoir un marquage efficace car ce marquage se produit sur le phosphate libéré lors de la fragmentation. De plus, il faut ajouter un excès important de réactif de marquage par rapport à l'ARN pour obtenir un marquage efficace, ce qui induit des problèmes de bruit de fond généré par le marqueur en excès. Enfin cette méthode n'est pas applicable à l'ADN double brin.

Il existe donc un besoin pour une technique de marquage de l'ADN simple et efficace qui permette un bon rendement de marquage et donc une bonne sensibilité, qui soit spécifique au niveau de la position de marquage, et en particulier qui n'affecte pas les propriétés d'hybridation des bases impliquées dans la formation de la double hélice par l'intermédiaire des liaisons hydrogènes, et enfin qui permette la fragmentation de l'ADN dans le but d'hybrider ces fragments d'ADN marqués sur des sondes nucléiques, et en particulier sur des sondes nucléiques fixés sur un support solide.

La présente invention décrit un procédé de marquage et de fragmentation d'un acide désoxyribonucléique (ADN) simple ou double brin comprenant :
- fragmenter l'ADN en créant au moins un site abasique sur ledit ADN,
- attacher un marqueur sur au moins un des fragments par l'intermédiaire d'un réactif de marquage, ledit réactif se couplant de manière covalente et majoritaire sur au moins un phosphate dudit fragment,
la fragmentation et le marquage étant réalisés en une étape.

La présente invention concerne également un procédé pour la mise à disposition de sondes de détection d'un acide nucléique cible, comprenant le marquage et la fragmentation d'un acide désoxyribonucléique (ADN) simple ou double brin comprenant les étapes suivantes :
- fragmenter l'ADN par l'intermédiaire de la création d'au moins un site abasique sur ledit ADN,
- attacher un marqueur sur au moins un des fragments par l'intermédiaire d'un réactif de marquage, ledit réactif se couplant de manière covalente et majoritaire sur au moins un phosphate dudit fragment.

La fragmentation et le marquage s'effectue en une étape ou en deux étapes et le marquage peut s'effectuer indifféremment avant, après ou simultanément avec la fragmentation.

De préférence, la marquage et/ou la fragmentation s'effectue(nt) en solution homogène sensiblement aqueuse. De préférence, le marquage et la fragmentation s'effectuent simultanément, c'est-à-dire que les réactifs nécessaires à ces deux étapes sont mis ensemble dans une solution sensiblement aqueuse avec l'acide nucléique par exemple. C'est notamment le cas pour la fragmentation chimique ou enzymatique. Dans le cas de la fragmentation mécanique par un moyen physique, marquage et fragmentation s'effectuant simultanément signifie que le moyen physique est appliqué à une solution contenant au moins les acides nucléiques et le réactif de marquage.

Par solution sensiblement aqueuse, on entend une solution contenant au moins 50% d'eau. Cette solution contient de préférence des sels comme une solution tampon. Par solution homogène, on entend une solution monophasique telle qu'une solution eau/DMSO, par opposition à une solution biphasique telle qu'une solution eau/chloroforme.

La fragmentation de l'ADN par l'intermédiaire de la création d'un site abasique s'effectue par voie enzymatique, chimique ou physique.

La fragmentation par voie chimique de l'ADN est réalisée en mettant en présence l'acide nucléique avec un moyen chimique de création de site abasique.

Des exemples de conditions de fragmentation chimique par l'intermédiaire d'un site abasique de l'ADN sont donnés dans G. Pratviel et al., Angew. Chem. Int. Ed. Engl., 34, p746-769, 1995; G. Pratviel et al., Adv. Inorg. Chem.,45, p251-312, 1998; D. S. Sigman et al. Chem. Rev., 93, p2295-2316, 1993 ; J. Lhomme et al., Biopolymers (Nucleic Acid Sciences), 52, p65-83, 1999.

Un site abasique est créé quand la liaison N-glycosidique reliant la base modifiée au désoxyribose est hydrolysée, laissant la liaison phosphodiester intacte. Ce phénomène est appelé dépurination (dans le cas des purines) ou dépyrimidination (dans le cas des pyrimidines). La dépurination est beaucoup plus fréquente que la dépyrimidination.

Des agents chimiques, tels que le pH acide, les agents oxydants ou les agents alkylants, peuvent induire le phénomène de dépurination et donc la formation de sites abasiques. Ces agents alkylants sont constitués d'espèces électrophiles qui réagissent avec les sites nucléophiles d'un fragment d'ADN. L'atome d'azote en position 7 de la guanine est le site principal d'alkylation de l'ADN. En plus de la protonation des purines, l'alkylation est l'une des modifications chimiques susceptibles de rendre la liaison N-glycosidique plus labile.

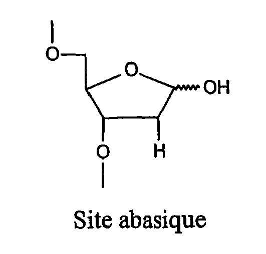

L'oxydation de l'ADN peut également générer des lésions abasiques dites « alcalilabiles », qui conduisent à la fragmentation de l'ADN en présence d'une base. Par exemple, le résidu ribonolactone peut être généré par réaction du radical hydroxyle (OH) avec le carbone C1'.

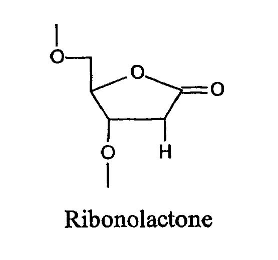

Le site abasique est très instable. Sous sa forme aldéhydique, il peut subir facilement une β-élimination du phosphodiester fixé en position 3', ce qui conduit à la fragmentation du brin de l'ADN.

La dépurination est spontanée dans des conditions physiologiques (pH 7,4 à 37°C) mais la vitesse de la réaction est très faible de l'ordre de 3.10⁻¹¹ dépurination par seconde, c'est-à-dire inutilisable pour une fragmentation efficace. Pour augmenter la vitesse de réaction, on utilise des agents alkylants qui fragilisent la liaison N-glycosidique ou des enzymes, comme des ADN glycosylases.

Un mode préféré de réalisation de la fragmentation est obtenu par l'utilisation d'un pH acide, c'est-à-dire un pH inférieur à 5, de préférence inférieur à 4. Avantageusement le pH est d'environ 3.

Un tampon formiate de sodium à pH 3 permet de fragmenter de manière efficace les acides nucléiques selon la présente invention. Ce tampon est compatible avec les conditions de marquage en une étape comme cela sera démontré dans les exemples. Encore plus avantageusement, un milieu acide (HCl, carbonate, H₂SO₄) est utilisé.

Dans un mode particulier de la présente invention et dans le but d'augmenter encore la fragmentation, l'acide désoxyribonucléique contient au moins une base modifiée susceptible de générer un site abasique plus facilement.

Diverses bases modifiées sont utilisables comme les N7-alkylpurines, les N3-alkylpurines, les O6-alkylpurines, les 8-bromopurines, les 8-thiopurines, les 8-alkylthiopurines, les 8 azidopurines ou les 8-alkylsulfonylpurines.

Dans le cas où l'acide nucléique à marquer est généré par une technique d'amplification enzymatique comme la PCR, l'utilisation d'une 8-bromopurine permet d'avoir une incorporation efficace dudit nucléotide pendant l'amplification ce qui facilite d'autant le procédé de fragmentation et de marquage selon l'invention, tout en conservant une sensibilité excellente pour l'étape d'amplification enzymatique. Une 8-méthylthiopurine est également incorporée sans gêner l'efficacité d'amplification et d'incorporation par PCR. Une fois incorporée, la base thioéther est oxydée par un peracide ou un dérivé de monopersulfate comme le peroxymonosulfate de potassium par exemple, en sulphonyle correspondant qui est très labile. Il a été démontré que le temps de demi-vie correspondant à l'hydrolyse d'une 8-méthylsulfonylguanosine est inférieur à 2 minutes alors que le temps de demi-vie pour son homologue naturel est de 1000 heures.

Par marqueur, on entend au moins un marqueur capable de générer directement ou indirectement un signal détectable. Une liste non limitative de ces marqueurs suit :
- les enzymes qui produisent un signal détectable par exemple par colorimétrie, fluorescence, luminescence, comme la péroxydase de raifort, la phosphatase alcaline, la β-galactosidase, la glucose-6-phosphate déshydrogénase,
- les chromophores comme les composés fluorescents, luminescents, colorants,
- les groupements à densité électronique détectable par microscopie électronique ou par leur propriété électrique comme la conductivité, l'ampérométrie, la voltamétrie, l'impédance,
- les groupements détectables, par exemple dont les molécules sont de tailles suffisantes pour induire des modifications détectables de leurs caractéristiques physiques et/ou chimiques, cette détection peut être réalisée par des méthodes optiques comme la diffraction, la résonance plasmon de surface, la variation de surface, la variation d'angle de contact ou des méthodes physiques comme la spectroscopie de force atomique, l'effet tunnel, et
- les molécules'radioactives comme le ³²P, le ³⁵S ou le ¹²⁵I.

De préférence, le marqueur n'est pas un marqueur radioactif pour éviter les problèmes de sécurité liés à ces marqueurs.

Dans un mode de réalisation particulier de la présente invention, le marqueur est détectable électrochimiquement et en particulier le marqueur est un dérivé d'un complexe de Fer comme un ferrocène.

Des systèmes indirects peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte la fonction réactive. L'anti-ligand peut être détectable directement par les marqueurs décrits au paragraphe précédent ou être lui-même détectable par un autre couple ligand/anti-ligand.

Un autre exemple de systèmes indirects utilise une liaison covalente spécifique entre le ligand et l'anti-ligand, par exemple méthylcétone et alcoxyamine. Des exemples de ce système sont décrits dans les demandes de brevet WO-A-00/40590 et WO-A-98/05766.

Dans le procédé de la présente invention, la liaison entre le réactif de marquage et l'acide nucléique est covalente, mais il est décrit ci-dessus que des interactions non covalentes peuvent être utilisées notamment dans les systèmes d'empilement ou dans le cas où le marqueur est détectable indirectement. Le terme « attacher » couvre donc ces différentes possibilités.

Ces systèmes de détection indirects peuvent conduire, dans certaines conditions, à une amplification du signal et l'on pourra se reporter aux demandes de brevet antérieures WO-A-00/07982, WO-A-01/92361 et WO-A-95/08000 de la demanderesse pour des exemples d'amplification chimique en utilisant des polymères ou à la demande WO-A-01/44506, toujours de la demanderesse, pour les systèmes d'amplification chimique par empilement.

Dans un mode particulier d'amplification de signal, au moins deux marqueurs sont présents sur le réactif de marquage.

Dans un mode préféré de l'invention, le traceur est un composé fluorescent de faible encombrement stérique comme la fluorescéine, le dansyl, les chromophores du type IR (Li-COR Inc, Lincoln NE, USA), les dérivés cyanines comme le Cy5 et Cy3 (Randolph J.B. and al, Nucleic Acids Res., 25(14), p2923-2929, 1997) et en particulier les dérivés du Cy5 ou bien le traceur est un haptène de faible encombrement stérique comme la biotine ou un dérivé de l'abiétane (voir la demande WO-A-00/07982). Par faible encombrement stérique, on entend un poids moléculaire inférieur à 1000 g/mole.

Dans le cas d'un fluorophore, il est préférable de travailler avec des fluorophores dont la longueur d'onde d'excitation est supérieure à 450 nm, de préférence supérieure à 600 nm.

Dans le cas où le traceur est un haptène qui ne produit pas de signal par lui-même, comme par exemple la biotine, la détection est réalisée par la réaction d'un anti-ligand comme décrit plus haut. Dans le cas de la biotine, on utilise de préférence de la streptavidine ou un anticorps anti-biotine couplé à un composé fluorescent comme la fluorescéine, Cy5 ou la Phycoérythrine. Dans le cas de l'abiétane, on utilise un anticorps monoclonal comme décrit dans WO-A-00/07982.

Le terme « acide désoxyribonucléique» ou ADN signifie un enchaînement d'au moins deux désoxyribonucléotides comprenant éventuellement au moins un nucléotide modifié, par exemple au moins un nucléotide comportant une base modifiée tel que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant l'hybridation.

Cet ADN peut aussi être modifié au niveau de la liaison internucléotidique comme par exemple les phosphorothioates, les H-phosphonates, les alkyl-phosphonates, au niveau du squelette comme par exemple les alpha-oligonucléotides (FR-A-2.607.507) ou les PNA (M. Egholm et al., J. Am. Chem. Soc., 114, 1895-1897, 1992). L'ADN est naturel ou synthétique, et/ ou sous la forme de fragment. L'ADN est simple ou double brin.

En particulier l'ADN est obtenu par une technique d'amplification enzymatique telle que:
- la PCR (Polymerase Chain Reaction), décrite dans les brevets US-A-4,683,195, US-A-4,683,202 et US-A-4,800,159, et sa dérivée RT-PCR (Reverse Transcription-PCR), notamment dans un format en une étape tel que décrit dans la demande de brevet EP-A-0.569.272,
- la LCR (Ligase Chain Reaction), exposée par exemple dans la demande de brevet EP-A-0.201.184,
- la RCR (Repair Chain Reaction), décrite dans la demande de brevet WO-A-90/01069.

On parle alors d'amplicons pour désigner l'ADN généré par une technique d'amplification enzymatique. L'ADN peut aussi comprendre des ribonucléotides en faible proportion, par exemple inférieur à 10%.

Chacune de ces modifications peut être prise en combinaison pour peu qu'au moins un phosphate soit présent dans l'ADN.

Le réactif de marquage comprend, comme fonction réactive, un motif choisi parmi les composés suivants : diazométhyle ; halogénure d'alkyle ; nitrosourée ; spirocyclopropane ; aziridine ; époxyde ; trifluorosulfonates.

Les fonctions réactives sont décrites ci-dessous :

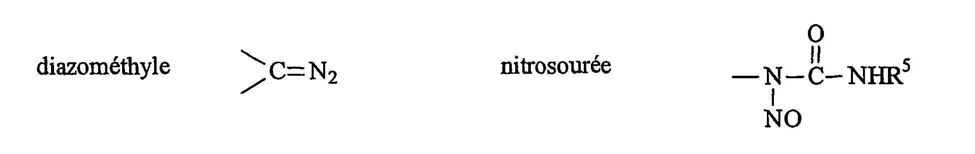

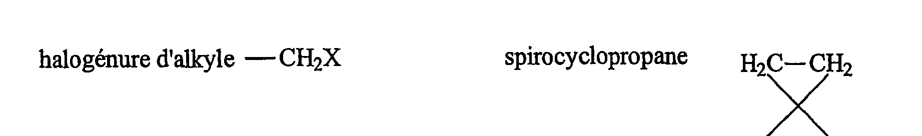

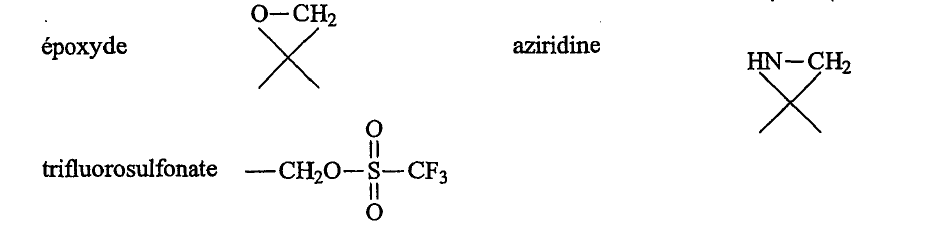

Pour la fonction réactive halogénure d'alkyle, X signifie Br, Cl ou I. Avantageusement le réactif de marquage est la 5-(bromométhyl) fluorescéine.

Pour la fonction réactive nitrosourée, R⁵ est un alkyle ou H.

La fonction diazométhyle a déjà été utilisée pour l'alkylation des groupements phosphates, mais un certain nombre de problèmes se pose. D'une part, les dérivés diazo en général sont instables eux-mêmes, ce qui génère des problèmes pour l'utilisation de ces réactifs de marquage dans un kit de marquage, et d'autre part, le produit de couplage est instable ce qui est rédhibitoire si le produit marqué a pour fonction de mettre en évidence la présence d'une molécule cible biologique dans un échantillon quelconque, ou si c'est la cible marquée que l'on souhaite détecter.

Enfin les dérivés portant la fonction diazométhyle sont insolubles dans l'eau ce qui conduit à utiliser des conditions biphasiques pour le couplage avec des acides nucléiques, qui ne sont solubles et stables que dans l'eau ou des tampons aqueux, mais ces conditions ralentissent la vitesse de réaction et donc nuisent à l'efficacité du couplage.

Dans un mode préféré du procédé selon l'invention, le réactif de marquage est choisi parmi les composés de formule (1) : 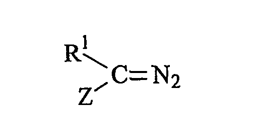 dans laquelle :
- R¹ représente H ou un groupe alkyle, alkyle substitué, aryle ou aryle substitué,
- Z comprend un marqueur détectable.
Z et/ou R¹ sont choisis pour stabiliser la fonction diazométhyle, c'est-à-dire au moins un des deux groupes Z ou R¹a un noyau phényle.

De préférence, le réactif de marquage est choisi parmi les composés de formule (2) : 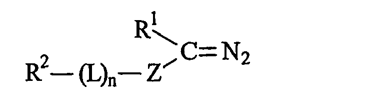 dans laquelle :
- R¹ représente H ou un groupe alkyle, aryle ou aryle substitué,
- R² est un marqueur détectable,
- L est un bras de liaison comportant un enchaînement linéaire d'au moins deux liaisons covalentes et n est égal à 0 ou 1,et
- Z est choisi parmi : 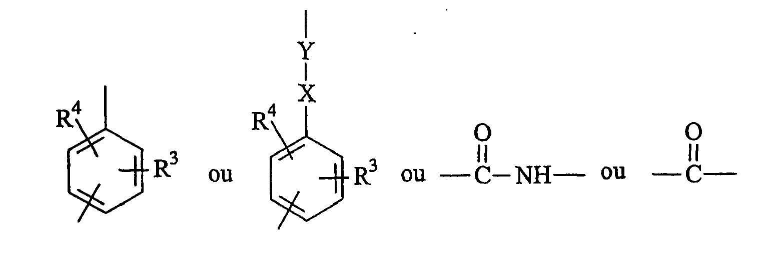 dans lequel :
   ■ R³ et R⁴ représentent indépendamment l'un de l'autre : H, NO₂, Cl, Br, F, I, OR, SR, NR₂, R, NHCOR, CONHR, COOR avec R = alkyle ou aryle,
   ■ -Y-X- représente -CONH-, -NHCO-, -CH₂O-, -CH₂S-.

Dans un mode particulier selon la formule (1), Z a la structure suivante : 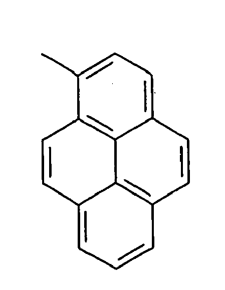 Dans ce cas et si R¹ est égal à H, le réactif de marquage est le 1-Pyrényldiazométhane (PDAM).

Bien que ce marqueur soit fluorescent, la longueur d'onde d'excitation est trop proche de celles des acides nucléiques. Un détection indirecte par l'utilisation d'un anticorps monoclonal dirigé contre le motif pyrène est préférée. Le mode d'obtention de cet anticorps est bien connu de l'homme du métier (voir par exemple la demande de brevet WO-A-00/07982).

Dans un mode préféré de réalisation du procédé, le réactif de marquage est de formule (3) : 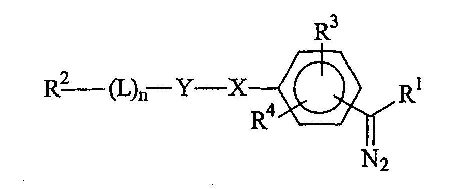 dans laquelle :
- R¹ représente H ou un groupe alkyle, aryle ou aryle substitué,
- R² représente un marqueur détectable,
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO₂, Cl, Br, F, I, OR, SR, NR₂, R, NHCOR, CONHR, COOR avec R = alkyle ou aryle,
- -Y-X- représente -CONH-, -NHCO-, -CH₂O-, -CH₂S-.

Avantageusement le réactif est de formule (4) : 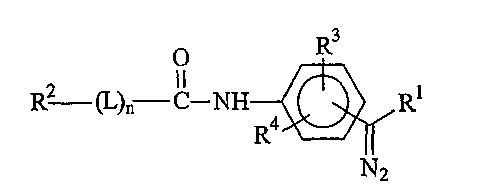 dans laquelle :
- R¹ représente H ou un groupe alkyle, aryle ou aryle substitué,
- R² représente un marqueur détectable,
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO₂, Cl, Br, F, I, OR, SR, NR₂, R, NHCOR, CONHR, COOR avec R = alkyle ou aryle.

Avantageusement le réactif est de formule (5) : 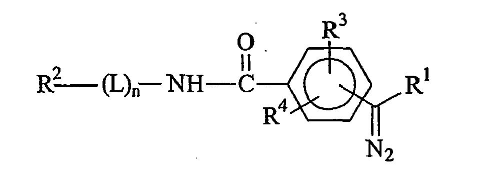 dans laquelle :
- R¹ représente H ou un groupe alkyle, aryle ou aryle substitué,
- R² représente un marqueur détectable,
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO₂, Cl, Br, F, I, OR, SR, NR₂, R, NHCOR, CONHR, COOR avec R = alkyle ou aryle.

Avantageusement le réactif est de formule (6) : 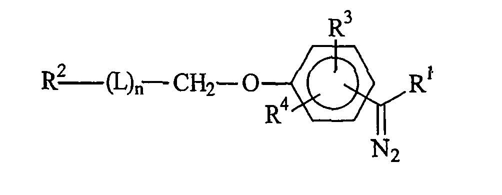 dans laquelle :
- R¹ représente H ou un groupe alkyle, aryle ou aryle substitué,
- R² représente un marqueur détectable,
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO₂, Cl, Br, F, I, OR, SR, NR₂, R, NHCOR, CONHR, COOR avec R = alkyle ou aryle.

Dans les formules ci-dessus (3) à (6), avantageusement R³ et R⁴ représentent indépendamment l'un de l'autre : H, NO₂, OCH₃.

Ainsi, un composé préféré selon la formule (6) est de formule (6') : 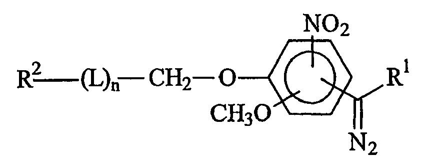 dans laquelle :
- R¹ représente H ou un groupe alkyle, aryle ou aryle substitué,
- R² représente un marqueur détectable,
- L est un bras de liaison comportant un enchaînement linéaire d'au moins deux liaisons covalentes et n un nombre entier égal à 0 ou 1.

Un composé préféré selon la formule (4) est de formule (4') : 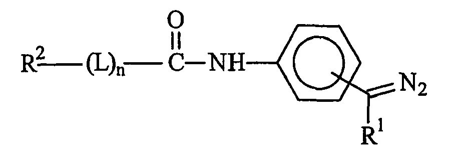 dans laquelle :
- R¹ représente H ou un groupe alkyle, aryle ou aryle substitué,
- R² représente un marqueur détectable,
- L est un bras de liaison comportant un enchaînement linéaire d'au moins deux liaisons covalentes et n un nombre entier égal à 0 ou 1.

Dans un mode particulier du procédé où l'on veut amplifier le signal, au moins deux marqueurs sont présents sur le réactif de marquage. En particulier un réactif qui permet de mettre en oeuvre l'amplification de signal selon la présente invention possède une structure R²-(L)ₙ- de formule (7) ci dessous : 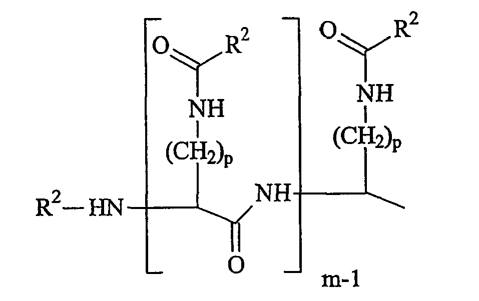 dans laquelle :
- R² représente un marqueur détectable,
- m est un nombre entier compris entre 1 et 100, de préférence compris entre 1 et 20,
- p est un nombre entier compris entre 1 et 10, avantageusement 2 à 6, de préférence 4.

Cette structure de R²-(L)ₙ s'applique indifféremment aux formules (2) à (6) précédentes.

Un autre réactif de marquage préféré pour l'amplification de signal est le réactif de formule (8) : 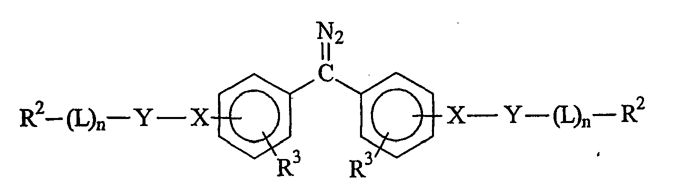 dans laquelle :
- R² représente un marqueur détectable,
- R³ représente H, NO₂, Cl, Br, F, I, OR, SR, NR₂, R, NHCOR, CONHR, COOR avec R = alkyle ou aryle,
- L est un bras de liaison comportant un enchaînement linéaire d'au moins deux liaisons covalentes et n un nombre entier égal à 0 ou 1,
- -Y-X- représente -CONH-, -NHCO-, -CH₂O-, -CH₂S-.

Avantageusement le réactif pour l'amplification de signal a la formule (9) 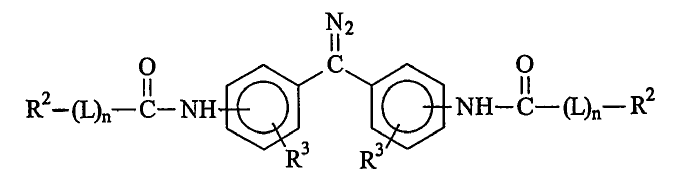 dans laquelle :
- R² représente un marqueur détectable,
- R³ représente H, NO₂, Cl, Br, F, I, OR, SR, NR₂, R, NHCOR, CONHR, COOR avec R = alkyle ou aryle, de préférence R³ représente H, NO₂ ou OCH_{3.},
- L est un bras de liaison comportant un enchaînement linéaire d'au moins deux liaisons covalentes et n un nombre entier égal à 0 ou 1.

Certains réactifs avantageux de l'invention sont :
a) de formule (10) : 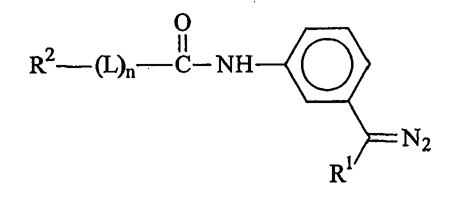 dans laquelle :
   - R¹ représente H ou un groupe alkyle ou aryle ou aryle substitué,
   - R² représente un marqueur détectable,
   - L est un bras de liaison comportant un enchaînement linéaire d'au moins deux liaisons covalentes et n un nombre entier égal à 0 ou 1,
b) de formule (11) : 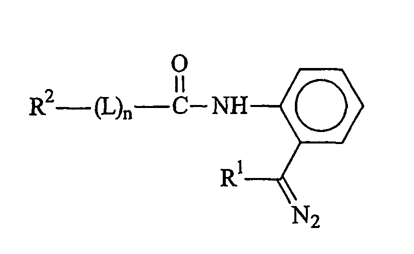 dans laquelle:
   - R¹ représente H ou un groupe alkyle ou aryle ou aryle substitué,
   - R² représente un marqueur détectable,
   - L est un bras de liaison comportant un enchaînement linéaire d'au moins deux liaisons covalentes et n un nombre entier égal à 0 ou 1,
c) de formule (12) : 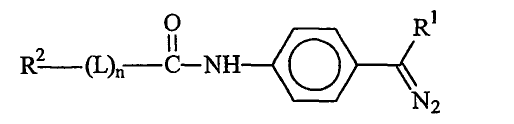 dans laquelle :

- R¹ représente H ou un groupe alkyle ou aryle ou aryle substitué,
- R² représente un marqueur détectable,
- L est un bras de liaison comportant un enchaînement linéaire d'au moins deux liaisons covalentes et n un nombre entier égal à 0 ou 1.

De préférence, le réactif de marquage a :
a) la structure 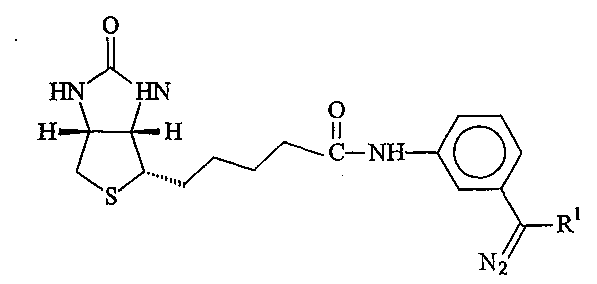 dans laquelle R¹ représente un groupe méthyle ou un phényle, ou
b) la structure : 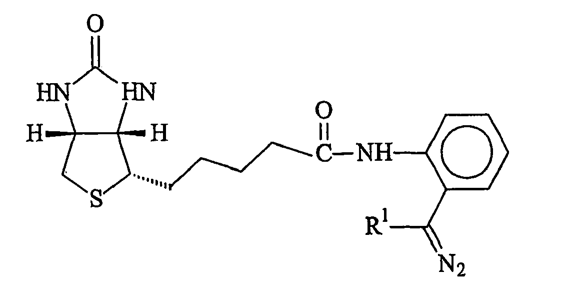 dans laquelle R¹ représente un groupe méthyle ou phényle, ou
c) la structure : 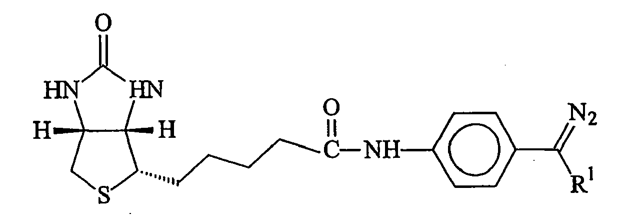
dans laquelle R¹ représente un groupe méthyle ou phényle.

D'autres réactifs préférés selon l'invention ont la formule (13) : 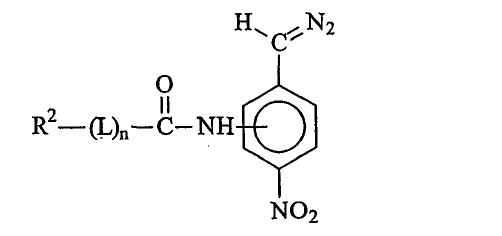 dans laquelle :
- R² représente un marqueur détectable,
- L est un bras de liaison comportant un enchaînement linéaire d'au moins deux liaisons covalentes et n un nombre entier égal à 0 ou 1.

En particulier les réactifs de marquage selon le procédé de l'invention sont solubles dans des solvants polaires miscibles à l'eau comme le DMF, le DMSO, CH₃CN, THF, DMA (diméthylacétamide), NMP (N-méthylpyrrolidone), DME (diméthoxyéthane).

De préférence, les réactifs de marquage sont solubles dans le DMSO ou l'eau.

Par solvant miscible à l'eau, on entend un solvant qui est miscible dans une proportion d'au moins 5% en volume avec de l'eau ou un tampon aqueux contenant des sels.

Avantageusement dans les formules précédentes, le bras L comprend un motif éthylène glycol ou polyéthylène glycol pour augmenter la solubilité du réactif dans l'eau.

Ces réactifs peuvent ainsi se fixer en phase homogène sur les acides nucléiques, la phase homogène étant constituée d'une solution sensiblement aqueuse, c'est-à-dire contenant au moins 50% d'eau.

Un objet de la présente invention est de décrire un procédé de détection d'un acide désoxyribonucléique cible (ADN) simple ou double brin comportant les étapes suivantes :
a) fragmenter et marquer lesdits ADN selon l'un quelconque des procédés décrits précédemment,
b) hybrider les fragments marqués sur au moins une sonde nucléique suffisamment spécifique de l'acide nucléique cible,
c) détecter l'hybride formé par l'intermédiaire du marqueur.

Comme cela sera démontré dans les exemples, la dénaturation de l'ADN avant hybridation permet d'augmenter la sensibilité. Cette étape de dénaturation a lieu après la fragmentation et le marquage.

Dans un mode particulier du procédé, une étape d'amplification enzymatique a lieu avant la fragmentation et le marquage. L'étape d'amplification enzymatique génère des amplicons ADN à partir d'un acide nucléique cible ADN, mais aussi à partir d'un acide nucléique cible ARN comme une ARN messager, un ARN de transfert ou un ARN ribosomal. La technique de RT-PCR par exemple est un moyen connu pour amplifier l'ARN.

De préférence, les étapes de fragmentation, marquage et dénaturation ont lieu en même temps, aussi bien dans le cas d'un acide nucléique cible naturel que dans le cas d'un acide nucléique cible obtenu par une technique d'amplification enzymatique.

La fragmentation par création d'un site abasique implique la perte d'une base. Dans les procédés de détection d'acide nucléique cible et en particulier dans les procédés de génotypage c'est-à-dire dans les procédés où l'acide nucléique cible présente un polymorphisme réparti sur sa séquence, il est nécessaire d'identifier une pluralité de modifications de séquences dont certaines représentent la modification d'une seule base entre la cible et les sondes nucléiques dont la fonction est d'identifier cette modification (sondes de capture). La spécificité est donc essentielle dans ce contexte. De manière surprenante, la présente invention démontre que cette perte d'une base n'affecte en rien la spécificité de l'hybridation pour un acide nucléique marqué et fragmenté selon l'invention.

La présente invention a aussi pour objet un procédé de mise en évidence d'un polymorphisme, réparti en des positions prédéterminées ou non d'un acide nucléique cible par la présence d'une pluralité de délétions et/ou insertions et/ou mutations dans la séquence dudit acide nucléique cible par rapport à une séquence, dite de référence, comportant les étapes suivantes :
a) disposer d'un ADN cible comportant l'ensemble du polymorphisme à étudier, ledit ADN étant éventuellement généré par une technique d'amplification enzymatique,
b) fragmenter et marquer ledit ADN par un des procédés décrits précédemment,
c) hybrider lesdits fragments sur une pluralité de sondes nucléiques dites sondes de capture, la pluralité de sondes de capture étant fixé sur un support solide et la pluralité de sondes de capture couvrant dans son ensemble au moins le polymorphisme à étudier,
d) détecter les hybrides formés entre les fragments marqués et au moins une partie des sondes nucléiques par l'intermédiaire du marqueur et en déduire le polymorphisme de l'ADN cible.

Comme indiqué précédemment une étape de dénaturation après l'étape de fragmentation et marquage permet d'améliorer la sensibilité du procédé, et cette étape est réalisée de préférence simultanément avec l'étape de marquage et de fragmentation.

Le procédé de fragmentation et de marquage selon l'invention est particulièrement utile dans le cas où l'ADN marqué et fragmenté doit s'hybrider avec une multitude d'acides nucléiques, notamment oligonucléotides, fixés sur le support solide à une position prédéterminée pour former une puce à ADN. Par "puce à ADN", on entend un support solide de dimension réduite où sont fixés une multitude de sondes de capture à des positions prédéterminées. Par multitude, on entend un support solide comportant au moins dix (10) sondes nucléiques de séquences différentes, avantageusement au moins quatre cents (400), de préférence au moins mille (1000).

En effet, la densité des acides nucléiques fixés sur le support solide impose des contraintes stériques importantes lors de l'hybridation et la fragmentation permet d'améliorer cette étape d'hybridation. Des exemples de ces puces à ADN sont donnés par exemple dans les publications de G. Ramsay, Nature Biotechnology, 16, p40-44, 1998; F. Ginot, Human Mutation, 10, p1-10, 1997 ; J. Cheng et al, Molecular diagnosis, 1(3), p183-200, 1996 ; T. Livache et al, Nucleic Acids Research, 22(15), p2915-2921, 1994 ; J. Cheng et al, Nature Biotechnology, 16, p541-546, 1998. Le terme "support solide" tel qu'utilisé ici inclut tous les matériaux sur lesquels peut être fixé un acide nucléique. Le support solide peut être sous la forme d'une plaque de microtitration, d'une membrane, d'une particule ou d'une plaque sensiblement plane.

L'invention permet l'utilisation d'un ADN marqué, tel que défini ci-dessus, comme sonde de détection d'une acide nucléique cible.

Pour permettre la détection et/ou la quantification et/ou la purification de l'acide nucléique cible, l'ADN marqué est capable de former un complexe d'hybridation avec une sonde nucléique. A titre d'exemple, l'acide nucléique marqué est suffisamment complémentaire de la cible pour s'hybrider spécifiquement en fonction des conditions de réaction et notamment de la température ou de la salinité du milieu réactionnel.

Le procédé de détection est applicable pour le séquençage, le profil d'expression des ARN messagers ou le criblage de mutations à des fins de recherche ainsi que le criblage de drogues dans l'industrie pharmaceutique, le diagnostic de maladies infectieuses (aussi bien en bactériologie, virologie, parasitologie) ou génétiques, le contrôle alimentaire ou industriel.

De nombreuses publications décrivent ce type d'applications : Antoine de Saizieu et al, Nature Biotechnology, 16, p44-48, 1998 ;Thomas R. Gingeras et al Genome Research, 8, p435-448, 1998 ; David J. Lockhart et al, Nature Biotechnology, 14, p1675-1680, 1996 ; Daniel D. Shoemaker et al, Nature Genetics, vol 14, p450-456, 1996 ; R.J. Lipshutz et al; BioTechniques, 19(3), p442-447,1995; David G. Wang et al, Science, 280, p1077-1082, 1998 ; Kevin L. Gunderson et al; Genome Research, 8, p1142-1152, 1998 ; Joseph G. Hacia et al; Nature Genetics, vol 14, p441-447, 1996.

La tendance en matière de diagnostic et notamment pour les maladies infectieuses (SIDA ou Tuberculose par exemple) est de baisser le niveau de sensibilité jusqu'à la détection d'une molécule unique dans un échantillon, qui peut représenter plusieurs millilitres dans le cas d'un prélèvement liquide type sang ou urine ou liquide céphalo-rachidien. Ce niveau de sensibilité ne peut être obtenu que si toutes les étapes depuis le prélèvement de l'échantillon jusqu'au rendu de résultat sont optimisées. En particulier dans le cas où une étape d'amplification enzymatique est nécessaire pour obtenir la sensibilité nécessaire (infection virale ou bactérienne comme VIH, VHC ou Tuberculose), un procédé de marquage et/ou fragmentation comme décrit dans la présente invention permet de ne pas affecter la sensibilité de la technique d'amplification, soit parce qu'il n'est pas nécessaire de remplacer les désoxyribonucléotides utilisés dans la technique d'amplification enzymatique, soit parce ce que les désoxyribonucléotides incorporés n'altèrent pas la sensibilité.

Des informations complémentaires peuvent être trouvées dans une autre demande de brevet de la Demanderesse FR-2 824 323 déposée le 4 mai 2001 sous le numéro d'enregistrement FR01/06040, ainsi que de son extension internationale déposée le même jour que la présente invention.

Les figures et exemples ci-joints représentent des modes particuliers de réalisation et ne peuvent pas être considérés comme limitant la portée de la présente invention.

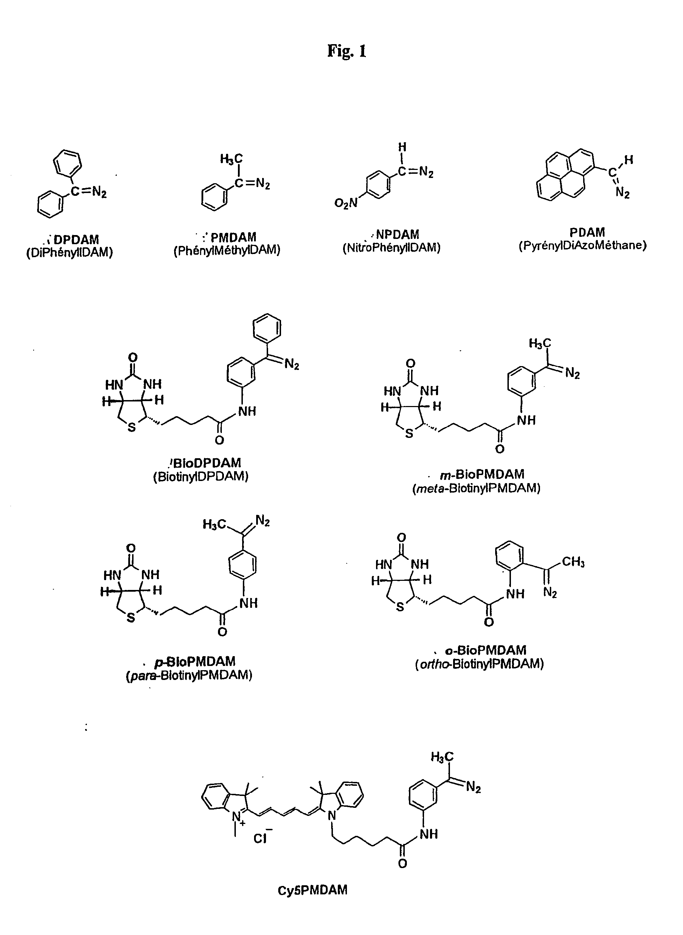

La figure 1 représente les formules développées de différents réactifs utilisés dans la présente invention ainsi que l'abréviation les désignant (o- signifie *ortho*, *m- méta* et p- *para*).
Les figures 2A à 2I représentent les profils, en fonction du temps, analysés par électrophorèse capillaire du couplage covalent de différents réactifs portant une fonction diazométhyle sur l'uridine 3' monophosphate (3'-UMP) selon l'exemple 6.1. Les molécules sont les suivantes :
   - PDAM en figure 2A,
   - DPDAM à 2 mM (millimole(s) par litre) en figure 2B,
   - DPDAM à 20 mM en figure 2C,
   - PMDAM en figure 2D,
   - NPDAM en figure 2E,
   - BioDPDAM en figure 2F,
   - *méta*-BioPMDAM en figure 2G,
   - *para*-BioPMDAM en figure 2H, et
   - *ortho*-BioPMDAM en figure 21.
Les figure 3A à 3D représentent les profils en fonction du temps analysée par électrophorèse capillaire de la réaction du *méta*-BioPMDAM sur quatre (4) nucléotides 3'-monophosphate selon l'exemple 6.2. Les molécules sont les suivantes :
   - 3'-CMP en série ribonucléotide selon la figure 3A,
   - 3'-AMP en série ribonucléotide selon la figure 3B,
   - 3'-GMP en série ribonucléotide selon la figure 3C, et
   - 3'-TMP en série désoxyribonucléotide selon la figure 3D.
La figure 4 représente les profils en fonction du temps analysée par électrophorèse capillaire de la réaction du *méta*-BioPMDAM sur un dinucléotide 5'-ApUp selon l'exemple 6.3.
Les figures 5A à 5D représentent le spectre RMN du proton dans D₂O des différents conjugués entre le réactif *méta*-BioPMDAM et quatre (4) ribonucléotides 3'-monophosphate selon l'exemple 6.4. Les molécules sont les suivantes :
   - 3'-GMP en figure 5A,
   - 3'-AMP en figure 5B,
   - 3' CMP en figure 5C, et
   - 3'UMP en figure 5D.

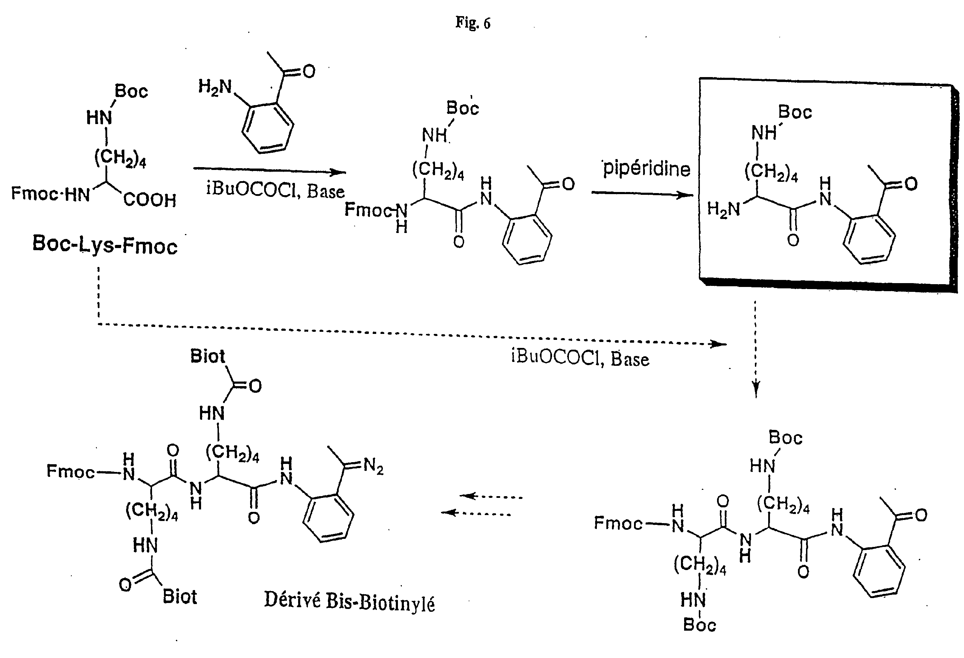

La figure 6 représente un schéma de synthèse d'un réactif de marquage portant deux biotines pour l'amplification chimique du signal.

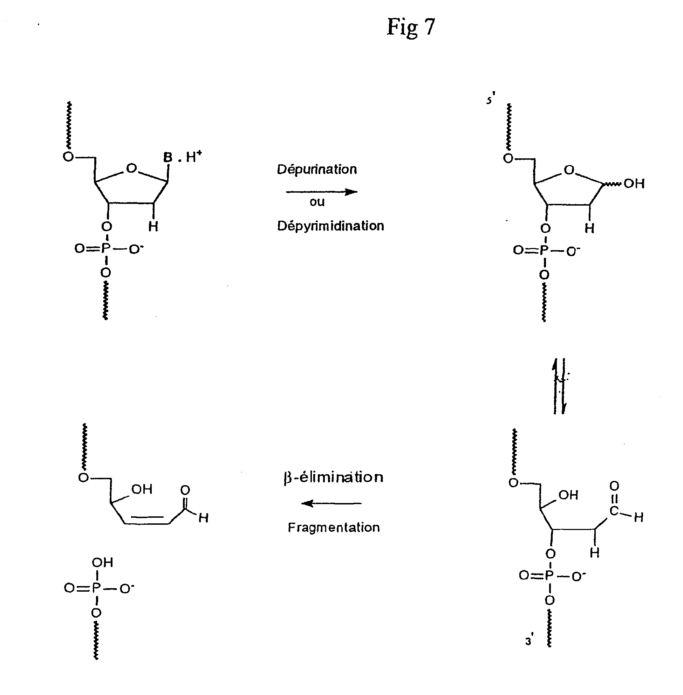

La figure 7 représente le mécanisme de fragmentation en milieu acide par la formation de site abasique.
La figure 8 montre, selon l'exemple 8.1, la cinétique de dégradation à pH acide pour différents nucléosides modifiés (8-bromo-2'-désoxyadénosine (8-BrdA) et la 5-bromo-2'-désoxycytidine (5-BrdC)) ainsi que les quatre nucléosides naturels (dA, dC, dG et dT). Les résultats sont représentés sous forme de pourcentage d'hydrolyse du nucléoside de départ (en ordonnée) par rapport au temps de réaction en minutes (en abscisse).
La figure 9 représente, selon l'exemple 11.2, la cinétique de marquage en fonction du temps à une température de 60°C avec le réactif PDAM sur un ODN synthétique 5'-phosphate. Les résultats sont représentés sous forme de pourcentage de marquage par rapport au temps de réaction exprimé en minutes (en abscisse).
La figure 10 représente, selon l'exemple 11.3, le pourcentage de marquage en fonction de la température de réaction. Les résultats sont présentés sur la figure 10 avec en ordonnée le pourcentage de marquage et en abscisse la température de réaction en °C.

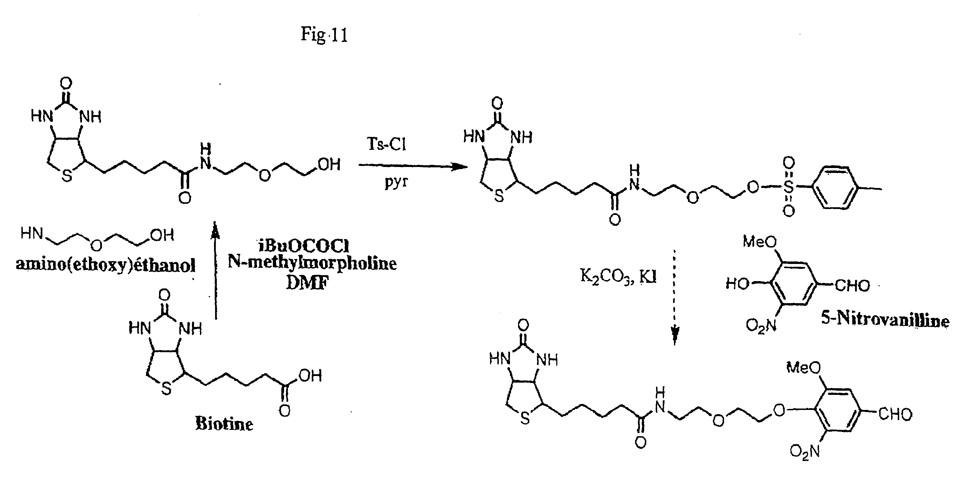

La figure 11 représente une voie de synthèse pour un réactif selon la formule 4', en utilisant le réactif commercial 5-Nitrovanilline. La fonction aldéhyde est le précurseur de la fonction diazométhyle.

### Exemple 1 : Synthèse de réactifs avec la biotine :

### Schéma général de synthèse :

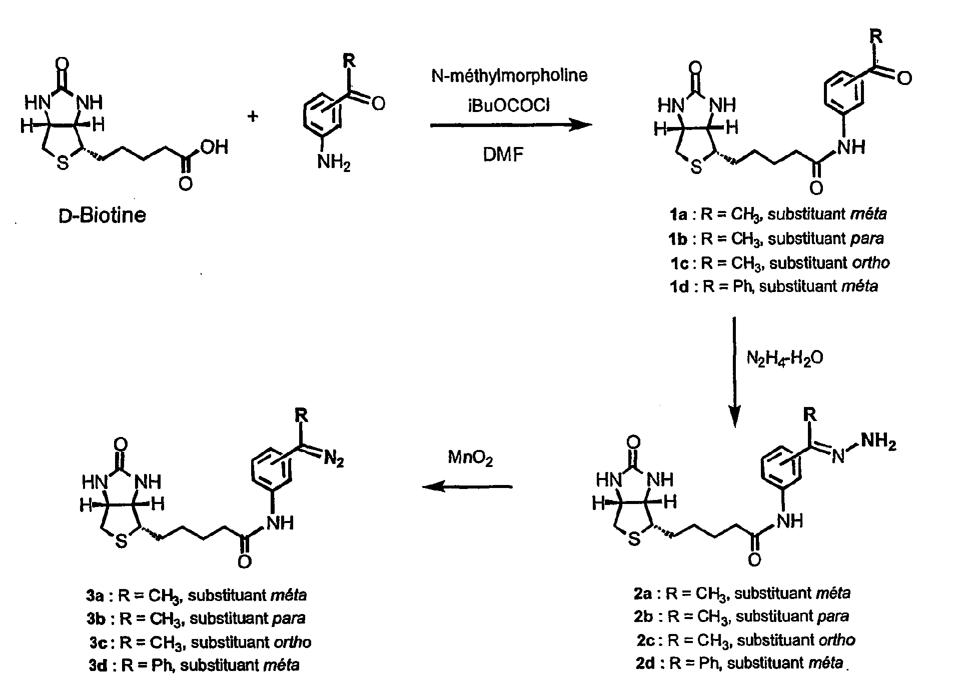

### Exemple 1.1 : Synthèse du méta-BioPMDAM :

### • Composé biotine méta-acétophénone 1a :

On solubilise la D-biotine (1,0 gramme (g), 4,1 millimoles (mmol)) dans 45 millilitres (mL) de DMF anhydre à chaud. On refroidit à 0°C sous argon, puis on ajoute successivement la *N-*méthylmorpholine (590 microlitres (µL), 5,33 mmol) et le chloroformiate d'isobutyle (840 µL, 6,60 mmol). On laisse sous agitation pendant 30 minutes (mn), puis on ajoute la 3-aminoacétophénone (824 mg, 6,10 mmol) et la *N*-méthylmorpholine (480 µL, 4,35 mmol) dans 10 mL de DMF. La solution est maintenue sous agitation à 0°C pendant 2 heures (h), puis on évapore à sec. On reprend le résidu dans 3 mL de MeOH, puis on ajoute 50 mL d'eau. Le précipité obtenu est filtré, lavé avec de l'eau, du CH₂Cl₂ et de l'éther pour donner 1,2 g (80 %) de produit **1a** brut. Une recristallisation dans le couple MeOH-H₂O donne **1a** (1,01 g, 70 %) sous forme d'une poudre blanche.

F 145°C. - IR (KBr) : 3280, 2931, 2857, 1691, 1590,1540, 1487, 1434, 1298, 1266 cm⁻¹. - RMN ¹H (300 MHz, DMSO-d₆) δ = 1,3-1,7 (m, 6 H) ; 2,33 (t, *J*= 8 Hz, 2 H) ; 2,55 (s, 3 H) ; 2,58 ; (d, *J*= 12 Hz, 1 H) ; 2,83 (dd, *J*= 12 et 5 Hz, 1 H) ; 3,13 (m, 1 H) ; 4,15 (m, 1 H) ; 4,31 (m, 1 H) ; 6,34 (s, 1 H) ; 6,41 (s, 1 H) ; 7,44 (t, *J*= 8 Hz, 1 H) ; 7,64 (d, *J*= 8 Hz, 1 H) ; 7,85 (d, *J*= 8 Hz, 1 H) ; 8,17 (s, 1 H) ; 10,05 (s, 1 H). - MS (FAB/glycérol), *m*/*z*: 362 [M+H]⁺.

### • Composé méta-hydrazone 2a :

Une solution de **1a** (500 mg, 1,38 mmol) et d'hydrazine monohydrate (200 µL, 4,15 mmol) dans de l'éthanol absolu (8 mL) est chauffée à reflux pendant 2 h. Après refroidissement à température ambiante, le précipité blanc est filtré, lavé avec de l'eau, puis avec de l'éther et séché. On obtient ainsi 385 mg (74 %) de produit **2a** sous forme d'une poudre blanche.

F 185°C. - IR (KBr) : 3298, 2931, 2857, 1698, 1665, 1626, 1541, 1494, 1470, 1446, 1330, 1265 cm⁻¹. - RMN ¹H (300 MHz, DMSO-d₆) δ = 1,3-1,7 (m, 6 H) ; 1,98 (s, 3 H) ; 2,26 (t, *J*= 8 Hz, 2 H) ; 2,56 ; (d, *J* = 12 Hz, 1 H) ; 2,81 (dd, *J* = 12 et 5 Hz, 1 H) ; 3,11 (m, 1 H) ; 4,13 (m, 1 H) ; 4,29 (m, 1 H) ; 6,39 (s, 3 H) ; 6,42 (s, 1 H) ; 7,22 (m, 2 H) ; 7,50 (d, *J*= 8 Hz, 1 H) ; 7,84 (s, 1 H) ; 9,82 (s, 1 H). - MS (FAB/glycérol), *m*/*z*: 376 [M+H]⁺.

### • Composé méta-diazométhane 3a :

On solubilise **2a** (180 mg, 0,48 mmol) dans 2 mL de DMF. On ajoute alors MnO₂ (340 mg, 3,9 mmol). Après 30 minutes d'agitation à température ambiante, le mélange est filtré à travers un entonnoir fritté contenant de la célite (épaisseur : 0,5 cm) et des tamis moléculaires en poudre 3 Å (0,5cm). Le mélange réactionnel est concentré jusqu'à un volume d'environ 0,5 mL, puis 5 mL d'éther sont ajoutés. Le précipité résultant est filtré, lavé à l'éther puis séché. Le composé **3a** (170 mg, 95 %) est obtenu sous forme d'une poudre rose.

F 160°C. - IR (KBr) : 3278, 2935, 2859, 2038, 1704, 1666, 1605, 1577, 1536, 1458, 1430, 1263 cm⁻¹. - RMN ¹H (300 MHz) δ = 1,3-1,7 (m, 6 H) ; 2,11 (s, 3 H); 2,28 (t, *J*= 8 Hz, 2 H); 2,57 ; (d, *J*= 12 Hz, 1 H) ; 2,81 (dd, *J*= 12 et 5 Hz, 1 H) ; 3,11 (m, 1 H) ; 4,13 (m, 1 H) ; 4,29 (m, 1 H) ; 6,33 (s, 1 H) ; 6,41 (s, 1 H) ; 6,60 (m, 1 H) ; 7,25 (m, 3 H) ; 9,84 (s, 1 H). ).

### Exemple 1.2 : Synthèse du para-BioPMDAM :

### • Composé biotine para-acétophénone 1b :

On solubilise la D-biotine (1 g, 4,1 mmol) dans 45 mL de DMF anhydre à chaud. On refroidit à 0°C sous argon puis on ajoute successivement la *N*-méthylmorpholine (590 µL, 5,33 mmol) et chloroformiate d'isobutyle (840 µL, 6,60 mmol). On laisse sous agitation pendant 30 min, puis on ajoute la 4-aminoacétophénone (824 mg, 6,10 mmol). La solution est maintenue sous agitation à 0°C pendant 2 h puis on évapore à sec. On reprend le résidu dans 50 mL d'eau. Le précipité obtenu est filtré, lavé avec de l'eau puis avec 50mL de MeOH à chaud. Le précipité blanc est dissous dans du DMF en chauffant puis la solution obtenue est filtrée et lavée au MeOH. Le filtrat est récupéré et évaporé à sec pour donner 888 mg de **1b** (2,46 mmol, 60 %).

F 260°C. - IR (KBr) : 3260, 2930, 2358, 1706, 1673, 1610, 1526, 1401, 1380, 1322, 1257, 1150 cm⁻¹.- RMN ¹H (200 MHz, DMSO-d₆) δ = 8,82 (s, 1H, NH-CO) ; 7,57 (d, 2H, *J*= 9 Hz, Ar-H); 6,83 (d, 2H, *J*= 9 Hz, Ar-H); 6,40 (s large, 1H, NH-CO-NH) ; 6,32 (s large, 1H, NH-CO-NH) ; 4,28 (m, 1H, CH₂-CH-NH) ; 4,12 (m, 1H, CH-CH-NH) ; 3,11 (m, 1H, CH-S) ; 2,80 et 2,55 (système ABX, 2H, ²*J*_{AB}= 5 Hz, ³*J*_{AX} = 12 Hz, ³*J*_{BX} = 0 Hz, CH₂-S); 2,35 (t, 2H, *J*= 8 Hz, CH₂-CO) ; 2,10 (s, 3H, CH₃) ; 1,60-1,34 (m, 6H, (CH₂)₃).

### • Composé para-hydrazone 2b :

Le composé **1b** (870 mg, 2,4 mmol) est dissous à chaud dans l'éthanol (99 %, 8 mL) puis l'hydrazine monohydrate (995 µL, 19,5 mmol) est ajoutée. La solution est chauffée à reflux pendant 3 h. Le précipité blanc obtenu est filtré, lavé avec de l'eau glacée. On obtient ainsi 820 mg (90 %) de produit **2b** sous forme d'une poudre blanche.

F 305°C. - IR (KBr) : 3281, 3183, 2930, 2857, 1698, 1658, 1593, 1521, 1459, 1401, 1325, 1263, 1187 cm⁻¹. - RMN ¹H (200 MHz, DMSO-d₆) δ = 9,68 (s, 1H, NH-CO) ; 7,52 (s, 4H, *J* = 9 Hz, Ar-H) ; 6,43 (s large, 1H, NH-CO-NH) ; 6,35 (s large, 1H, NH-CO-NH) ; 6,21 (s, 2H, NH₂) 4,29 (m, 1H, CH₂-CH-NH) ; 4,12 (m, 1H, CH-CH-NH) ; 3,12 (m, 1H, CH-S) ; 2,81 et 2,56 (système ABX, 2H, ²*J*_{AB}= 5 Hz, ³*J*_{AX} = 12 Hz, ³*J*_{BX} = 0 Hz, CH₂-S) ; 2,32 (t, 2H, J= 8 Hz, CH₂-CO) ; 1,97 (s, 3H, CH₃) ; 1,63-1,36 (m, 6H, (CH₂)₃).

### • Composé para-diazométhane 3b :

On solubilise **2b** (200 mg, 0,53 mmol) dans 10 mL de DMF. On ajoute alors 800 mg de MnO₂. Après 10 minutes d'agitation, le mélange est filtré à travers une couche mixte Célite (0,5 cm) - tamis moléculaire (0,5 cm en poudre). Le mélange réactionnel est évaporé à sec puis lavé à l'éther et séché. Le composé **3b** (190 mg, 96 %) est obtenu sous forme d'une poudre rose.

F 180°C (dec). - IR (KBr) : 3257, 2930, 2857, 2032, 1698, 1597, 1524, 1510, 1455, 1404, 1307, 1259, 1180 cm⁻¹. - RMN ¹H (200 MHz, DMSO-d₆) δ = 10,18 (s, 1H, NH-CO) ; 7,88 (d, 2H, *J*= 6 Hz, Ax-H) ; 7,7 (d, 2H, *J*= 6 Hz, Ar-H) ; 6,41 (s large, 1H, NH-CO-NH) ; 6,34 (s large, 1 H, NH-CO-NH) ; 4,28 (m, 1H, CH₂-CH-NH) ; 4,12 (m, 1H, CH-CH-NH) ; 3,11 (m, 1H, CH-S) ; 2,80 et 2,55 (système ABX, 2H, ²*J*_{AB}= 5 Hz, ³*J*_{AX} = 12 Hz, ³*J*_{BX} = 0 Hz, CH₂-S) ; 2,35 (t, 2H, *J*= 8 Hz, CH₂-CO) ; 2,10 (s, 3H, CH₃) ; 1,60-1,34 (m, 6H, (CH₂)₃).

### Exemple 1.3 : Synthèse de l'ortho-BioPMDAM :

### • Composé biotine ortho-acétophénone 1c :

On solubilise la D-biotine (1 g, 4,1 mmol) dans 45 mL de DMF anhydre à chaud. On refroidit à 0°C sous argon puis on ajoute successivement la *N*-méthylmorpholine (590 µL, 5,33 mmol) et chloroformiate d'isobutyle (840 µL, 6,60 mmol). On laisse sous agitation pendant 30 min, puis on ajoute la 2-aminoacétophénone (824 mg, 6,10 mmol). La solution est maintenue sous agitation à température ambiante pendant 3 h 30 puis on évapore à sec. On reprend le résidu dans 50 mL d'eau. Le précipité obtenu est filtré, lavé avec de l'eau puis avec 50mL de MeOH à chaud. Le précipité obtenu est filtré, lavé avec de l'eau. Une recristallisation est faite en dissolvant le produit dans du MeOH à chaud et en reprécipitant par addition d'eau. Le précipité est filtré, lavé à l'eau, puis à l'éther pour donner 1,1 g (2,95 mmol, 72 %) de produit **1c** brut.

F 150°C. - IR (KBr) : 3248, 2930, 2857, 2359, 1691, 1669, 1651, 1582, 1528, 1448, 1354, 1310, 1245, 1161 cm⁻¹. - RMN ¹H (200 MHz, DMSO-d₆) δ = 11,24 (s, 1H, NH-CO) ; 8,33 (d, 1H, *J =* 8,5 Hz, Ar-H) ; 7,97 (d, 2H, *J =* 8 Hz, Ar-H) ; 7,57 (t, 1H, *J=* 7 Hz, Ar-H) ; 7,18 (t, 1H, *J =* 7 Hz, Ar-H) ; 6,44 (s large, 1H, NH-CO-NH) ; 6,35 (s large, 1H, NH-CO-NH) ; 4,30 (m, 1H, CH₂-CH-NH) ; 4,14 (m, 1H, CH-CH-NH) ; 3,12 (m, 1H, CH-S) ; 2,80 et 2,55 (système ABX, 2H, ²*J*_{AB}= 5 Hz, ³*J*_{AX} = 12 Hz, ³*J*_{BX} = 0 Hz, CH₂-S) ; 2,61 (s, 3H, CH₃) ; 2,37 (t, 2H, *J=* 8 Hz, CH₂-CO) ; 1,62-1,38 (m, 6H, (CH₂)₃).

### • Composé ortho-hydrazone 2c :

Le composé **1c** (500 mg, 1,38 mmol) est dissous à chaud dans l'éthanol (99 %, 8 mL) puis l'hydrazine monohydrate (572 µL, 11,1 mmol) est ajoutée. La solution est chauffée à reflux pendant 50 minutes. La solution est évaporée à sec. Le précipité blanc obtenu est filtré, lavé avec de l'eau puis séché à l'éther. On obtient ainsi 416 mg (11,1 mmol, 80 %) de produit **2c** sous forme d'une poudre blanche.

F 161°C. - IR (KBr) : 3412, 3240, 2930, 2857, 2351, 1706, 1677, 1604, 1590, 1531, 1463, 1444, 1372, 1303, 1270, 1169 cm⁻¹.- RMN ¹H (200 MHz, DMSO-d₆) δ =11,97 (s, 1H, NH-CO) ; 8,35 (d, 1H, *J* = 8 Hz, Ar-H) ; 7,45 (d; 1H, J= 7 Hz, Ar-H) ; 7,19 (t, 1H, *J* = 7,5 Hz, Ar-H) ; 7,04 (t, 1H, *J*= 7 Hz, Ar-H) ; 6,61 (s, 2H, NH₂) ; 6,42 (s large, 1H, NH-CO-NH) ; 6,35 (s large, 1H, NH-CO-NH) ; 4,32 (m, 1H, CH₂-CH-NH) ; 4,14 (m, 1H, CH-CH-NH) ; 3,12 (m, 1H, CH-S) ; 2,81 et 2,56 (système ABX, 2H, ²*J*_{AB}= 5 Hz, ³*J*_{AX} = 12 Hz, ³*J*_{BX} = 0 Hz, CH₂-S) ; 2,31 (t, 2H, *J*= 8 Hz, CH₂-CO) ; 2,09 (s, 3H, CH₃) ; 1,63-1,36 (m, 6H, (CH₂)₃).

### • Composé ortho-diazométhane 3c :

On solubilise **2c** (200 mg, 0,53 mmol) dans 10 mL de DMF. On ajoute alors 800 mg de MnO₂. Après 15 minutes d'agitation, le mélange est filtré à travers une couche mixte Célite(0,5cm)-tamis moléculaire(0,5cm en poudre). Le mélange réactionnel est évaporé à sec puis lavé à l'éther et séché. Le composé **3c** (130 mg, 65 %) est obtenu sous forme d'une poudre rose.

F 110°C. - IR (KBr) : 3248, 2930, 2857, 2367, 2342, 2038, 1699, 1521, 1456 cm⁻¹. - RMN ¹H (200 MHz, DMSO-d₆) δ = 9,37 (s, 1H, NH-CO) ; 7,26-7,00 (m, 4H, Ar-H) ; 6,43 (s large, 1H, NH-CO-NH) ; 6,35 (s large, 1H, NH-CO-NH) ; 4,30 (m, 1H, CH₂-CH-NH) ; 4,15 (m, 1H, CH-CH-NH) ; 3,12 (m, 1H, CH-S) ; 2,82 et 2,54 (système ABX, 2H, ²*J*_{AB}= 5 Hz, ³*J*_{AX} = 12 Hz, ³*J*_{BX} = 0 Hz, CH₂-S) ; 2,24 (t, 2H, *J*= 8 Hz, CH₂-CO) ; 2,12 (s, 3H, CH₃) ; 1,63-1,37 (m, 6H, (CH₂)₃).

### Exemple 1.4 : Synthèse du méta-BioDPDAM :

### • Composé méta-benzophénone 1d :

On solubilise la D-biotine (500 mg, 2,05 mmol) dans 23 mL de DMF anhydre à chaud. On refroidit à 0°C sous argon, puis on ajoute successivement la *N*-méthylmorpholine (295 µL, 2,67 mmol) et le chloroformiate d'isobutyle (420 µL, 3,28 mmol). On laisse sous agitation pendant 30 min, puis on ajoute la 3-aminobenzophénone (605 mg, 3,07 mmol) et la *N-*méthylmorpholine (240 µL, 2,17 mmol) dans 7 mL de DMF. La solution est maintenue sous agitation à 0°C pendant 2 h, puis on évapore à sec. On reprend le résidu dans 1 mL de MeOH, puis on ajoute 25 mL d'eau. Le précipité obtenu est filtré, lavé avec de l'eau, puis avec de l'éther pour donner 810 mg (93 %) de produit **1d** brut. Une recristallisation dans le couple MeOH-H₂O donne **1d** (630 mg, 72 %) sous forme d'une poudre blanche.

RMN ¹H (200MHz, DMSO-d₆) δ =10,10 (s, 1H, NH-CO) ; 8-7,39 (m, 9H, Ar-H) ; 6,43 (s large, 1H, NH-CO-NH) ; 6,35 (s large, 1H, NH-CO-NH) ; 4,27 (m, 1H, CH₂-CH-NH) ; 4,13 (m, 1H, CH-CH-NH) ; 3,12 (m, 1H, CH-S) ; 2,84 et 2,55 (système ABX, 2H, ²*J*_{AB}= 5 Hz, ³*J*_{AX} =12 Hz, ³*J*_{BX} = 0 Hz, CH₂-S) ; 2, 31 (t, 2H, *J*= 8 Hz, CH₂-CO) ; 1,59-1,36 (m, 6H, (CH₂)₃).

### • Composé méta-hydrazone 2d :

On solubilise **1d** (350 mg, 0,83 mmol) dans 5,5 mL d'éthanol absolu puis on ajoute l'hydrazine monohydrate (140 µL, 2,48 mmol). La solution est chauffée à reflux pendant une nuit. Après évaporation, le produit est repris dans 1 mL d'éthanol et de l'eau. Le précipité blanc est recristallisé : on le dissout dans un minimum d'éthanol à chaud et l'on ajoute de l'eau jusqu'à apparition d'un léger trouble. Après refroidissement, le précipité obtenu est lavé à l'eau puis séché à l'éther. On obtient ainsi 264 mg (73 %) de produit **2d** sous forme d'une poudre blanche.

RMN ¹H (200 MHz, DMSO-d₆) δ = 9,99 (s, 1H, NH-CO) ; 9,80 (s, 2H, NH₂) ; 7,54-6,88 (m, 9H, Ar-H) ; 6,26 (s large, 1H, NH-CO-NH) ; 6,21 (s large, 1H, NH-CO-NH) ; 4,28 (m, 1H, CH₂-CH-NH) ; 4,13 (m, 1H, CH-CH-NH) ; 3,12 (m, 1H, CH-S) ; 2,78 et 2,59 (système ABX, 2H, ²*J*_{AB}= 5 Hz, ³*J*_{AX} = 12 Hz, ³*J*_{BX} = 0 Hz, CH₂-S) ; 2,27 (t, 2H, *J*= 8 Hz, CH₂-CO) ; 1,57-1,36 (m, 6H, (CH₂)₃).

### • Composé méta-diazodiphényl 3d :

On solubilise **3d** (500 mg, 0,53 mmol) dans 1 mL de THF. On ajoute alors 80 mg de MnO₂ activé. Après 5 minutes d'agitation à température ambiante, le mélange est filtré à travers une couche mixte Célite (0,5 cm) -tamis moléculaire (0,5 cm en poudre). Le mélange réactionnel est évaporé à sec. Le composé **3d** (47 mg, 100 %) est obtenu sous forme d'une huile violette.

RMN ¹H (200 MHz, DMSO-d₆) δ = 9,95 (s, 1H, NH-CO) ; 7,60-6,9 (m, 9H, Ar-H) ; 6,42 (s large, 1H, NH-CO-NH) ; 6,35 (s large, 1H, NH-CO-NH) ; 4,28 (m, 1H, CH₂-CH-NH) ; 4,14 (m, 1H, CH-CH-NH) ; 3,12 (m, 1H, CH-S) ; 2,83 et 2,59 (système ABX, 2H, ²*J*_{AB}= 5 Hz, ³*J*_{AX} = 12 Hz, ³*J*_{BX} = 0 Hz, CH₂-S) ; 2,27 (t, 2H, *J* = 8 Hz, CH₂-CO) ; 1,58-1,35 (m, 6H, (CH₂)₃).

### Exemple 2 : Réactif de marquage avec le Cy5 : Cy5-PMDAM :

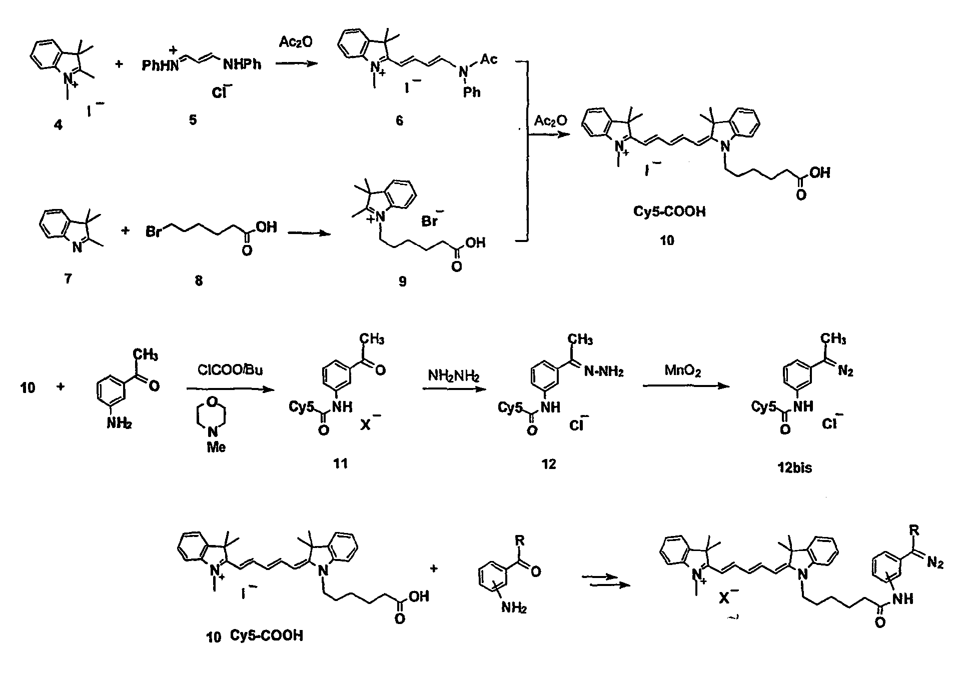

### • Composé Iodure de 2-[4-(N-acétyl-N-phénylamino)buta-1,3-diényl]-1,2,3,3-tétraméthyl[3H] indolium 6 :

Le mélange du monochlorhydrate de malonaldehyde-bis(phénylimine) **5** (18,3 g; 70,0 mmole), de NaOAc (9,0 g ; 110 mmole) et de l'iodure de 1,2,3,3- tétraméthyl[3H]indolium 4 (4,25 g ; 14,1 mmole) dans de l'anhydride acétique (75 mL) est chauffé à 110°C pendant 20 min. Après refroidissement, on ajoute de l'éther (350 mL) et le solide brun précipité est filtré, lavé avec de l'éther (3 x 100 mL). Le solide est redissout dans 150 mL de CH₂Cl₂, filtré (élimination des sels minéraux) puis évaporé pour donner un solide brun (6,0 g, 90 %).

RMN ¹H (CDCl₃) : δ = 8,64 (d ; 1 H ; *J* = 12 Hz ; 1-H) ; 8,14 (t ; 1 H ; *J* = 16 ; 12 Hz ; 3-H) ; 7,63-7,19 (m ; 9H) ; 6,90 (d ; 1H ; *J* = 15 Hz ; 4-H) ; 5,82 (t ; 1H ; *J=* 12 ; 13 Hz ; 2-H) ; 4,06 (s ; 3H ; NCH₃) ; (2,16 (s ; 3H ; -COCH₃) ; 1,74 (s ; 6H ; CH₃).

### • Composé Bromure de 1-(5-carboxypentyl)-2,3,3-triméthyl[3H]indolium 9 :

On mélange le 2,3,3-triméthylindole **7** (10,0 g ; 62,8 mmole) et l'acide 6-bromohexanoïque **8** (12,3 g ; 62,8 mmole) sans solvant et chauffe à 110°C pendant 12 h sous argon. Le mélange réactionnel pâteux rouge-violet est lavé avec de l'acétate d'éthyle (2 x 60 mL, on triture la pâte avec la spatule et on décante le surnageant), puis avec de l'acétone (50 mL, la pâte solidifie). Le solide rose est filtré puis séché sous vide (16,0 g ; 73 %).

### • Composé Cy5COOH 10 :

Le mélange de l'iodure **6** (6,0 g ; 12,7 mmole), du bromure **9** (4,5 g ; 12,7 mmole) et de NaOAc (2,6 g ; 32 mmole) dans de l'anhydride acétique (35 mL) est chauffé à 110 °C pendant 20 min. Après refroidissement, on ajoute de l'éther (150 mL) et le précipité est filtré et lavé avec de l'éther (3 × 50 mL). Le solide est dissous dans 100 mL de CH₂Cl₂, filtré et purifié par chromatographie sur colonne de SiO₂ (éluant : MeOH 5-10 %/CH₂Cl₂). On obtient 3,4 g (44 %).

RMN ¹H (CDCl₃) : δ = 8,03 (t ; 2H ; *J* = 10 ; 11 Hz, 2-H, 4-H) ; 7,38-6,91 (m ; 9H ; Ar-H, 3-H) ; 6,41 (d ; 1H ; *J* = 14 Hz ; 1-H) ; 6,31 (d ; 1H ; *J* = 13 Hz ; 5-H) ; 4,07 (t ; 2H, *J* = 7 ; 7 Hz ; α-CH₂) ; 3,68 (s ; 3H ; NCH₃) ; 2,47 (t ; 2H, *J* = 7 ; 7 Hz ; ε-CH₂) ; 1,71 (m ; 18H ; CH₃, β,γ et δ-CH₂).

### • Composé de couplage de la 3-aminoacétophénone avec le Cy5COOH 10 (produit 11) :

À une solution de Cy5COOH **10** (1,19 g ; 1,9 mmole) dans 12 mL de CH₂Cl₂, on ajoute *N-*méthylmorpholine (360 µL; 3,2 mmole). La solution est refroidie avec un bain de glace, puis on ajoute le chloroformiate d'isobutyle (480 µL; 3,7 mmole). Après 5 minutes d'agitation, on additionne la 3-aminoacétophénone (488 mg; 3,6 mmole). On agite le mélange à température ambiante pendant 3 h. En ajoutant 250 mL d'éther, on obtient un solide pâteux. Après agitation, on laisse reposer le solide au fond de ballon et on décante le surnageant. On ajoute de nouveau 50 mL d'éther et on triture avec une spatule pour obtenir un solide. Celui-ci est filtré, lavé avec de l'eau, de l'éther, puis séché sous vide. Le produit (iodure) est ensuite dissous dans de l'éthanol, passé sur colonne d'amberlite IRA900 (Cl⁻ ; 15 g). La solution éthanolique recueillie est évaporée à sec puis passée sur colonne de SiO₂. On obtient 0,93 g (77 %) de solide bleu.

### • Composé Cy5-hydrazone 12 :

On ajoute à une solution de l'acétophénone **11** (0,93 g ; 1,46 mmole) dans 5 mL d'éthanol absolu l'hydrazine monohydraté (180 µL ; 3,1 mmole) que l'on agite à température ambiante pendant 7 h. On ajoute 50 mL d'éther et le précipité est filtré et lavé avec de l'éther. On dissout le produit brut dans 50 mL de CH₂Cl₂, la solution est filtrée puis concentrée à 10 mL. Le produit est précipité par ajout de 100 mL d'éther, filtré, lavé à l'éther et séché sous vide. On obtient 540 mg de produit **12** (57 %).

### • Composé Cy5PMDAM 12bis :

A une solution de 100 mg d'hydrazone **12** dans 2 mL de DMF, on ajoute 300 mg de MnO₂ et l'on agite vigoureusement pendant 10 min. On filtre la suspension à travers une couche de célite et lavée avec DMF (3×500 µL). On ajoute 50 mL d'éther et l'huile déposée est triturée avec une spatule et le surnageant est décanté. On répète l'opération de lavage trois fois avec 25 mL d'éther et le solide ainsi obtenu est filtré, séché. On obtient 65 mg (65 %) de produit **12bis.** La pureté du produit est d'environ 80-85 % (RMN ¹H).

### Exemple 3 : Autres réactifs synthétisés :

### Exemple 3.1 : Synthèse de para-nitrophényldiazométhane (NPDAM) :

Le 4-nitrobenzaldéhyde hydrazone est commercialement disponible (référence 28,118-2, Aldrich, France).
On travaille donc sur 600 mg (3,64 mmole) de ce produit que l'on dissout dans 9 mL de THF. La solution est laissée sous agitation pendant 5 minutes puis 1,26 g (4 équivalent, 14,56 mmole) de MnO₂ sont additionnés avec précaution. Le mélange est laissé sous agitation pendant 10 minutes, puis filtré. Le filtrat récupéré est évaporé à sec. Après lavage au pentane, le composé *para*-nitrophényldiazométhane est obtenu sous forme d'une poudre orange vif avec un rendement de 79 % (468 mg, 2,87 mmole).

F 80-82°C. - RMN ¹H (300 MHz, DMSO-d₆) δ = 8,11 (d, 2H, *J*= 9 Hz, Ar-H₃); 7,18 (d, 2H, *J*= 9 Hz, Ar-H₂); 6,06 (s, 1H, CH₁-N₂).

### Exemple 3.2 : Synthèse du phénylméthyldiazométhane (PMDAM):

Hydrazone de l'acétophénone : On dilue l'acétophénone (2,0 g, 16 mmole) dans 16 mL d'éthanol absolu puis on ajoute l'hydrazine (2,3 mL, 48 mmole). On porte au reflux. Après 2 h, on évapore le solvant et on reprend le résidu dans de l'éther (150 mL). On lave avec de l'eau (100mL). Après séchage sur Na₂SO₄, on évapore l'éther. On obtient l'huile jaune pâle (1,5 g, 11 mmole, 69 %).
Phénylméthyldiazométhane (PMDAM) : On dissout l'hydrazone (150 mg, 1,1 mmole) dans 3 mL de THF. On ajoute le MnO₂ (480 mg, 5,5 mmole). On agite pendant 30 min à température ambiante. Le milieu se colore en rouge. On filtre et on évapore le solvant. On obtient l'huile rouge (145 mg, 100 %). Ce réactif est utilisé sans purification.

### Exemple 3.3 : Synthèse du diphényldiazométhane (DPDAM) :

La Benzophénone hydrazone est commerciale (référence B 960-2 Aldrich, France).
On travaille donc sur 196 mg (1,0 mmole) que l'on dissout dans 5 mL de THF. 435 mg (5 éq, 5,0 mmole) de MnO₂ sont additionnés, le mélange est laissé sous agitation pendant 10 minutes, puis filtré. Le filtrat récupéré est évaporé à sec. On obtient 193 mg (0,99 mmole). Ce réactif est utilisé sans purification.

### Exemple 3.4 : Synthèse du NVDAM :

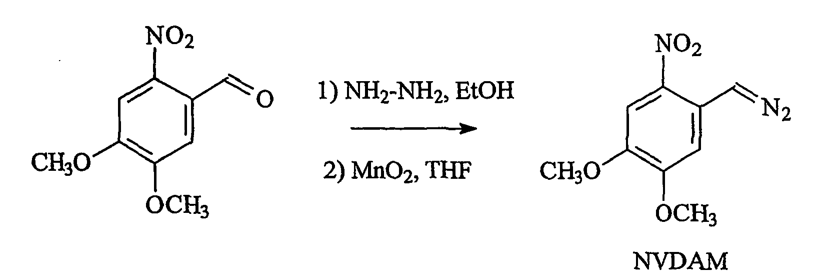

La synthèse est réalisée selon le protocole décrit précédemment à partir de 6-nitrovératraldéhyde (Aldrich, référence 27,960-9).

### Exemple 4 : Synthèse du dérivé biotinylé à partir du NPDAM :

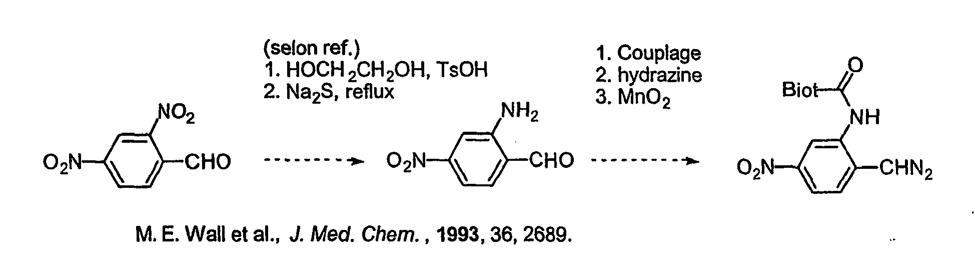

Le dérivé 2-amino-4-nitro benzaldéhyde est préparé selon la méthode de ME Wall et al référencé ci-dessus.

La préparation du diazométhane NPDAM est identique à celle décrite dans l'exemple 3.1 ci-dessus.

### Exemple 5 : Préparation des acides nucléiques ADN :

Les amplicons ADN sont générés par PCR à partir de cibles d'ADN génomique *Mycobacterium tuberculosis* 16S (10⁺⁴ copies comme cibles de départ) en utilisant le kit Fast Start de Roche, 0,2 mM de chaque désoxyribonucléotide (d-ATP, d-CTP, d-GTP, d-TTP), 0.3 µM d'amorces et 0,4 µL d'enzyme.
Les paramètres de la PCR sont les suivants :
- 95°C : 4 min puis 35 cycles (95°C : 30 sec ; 55°C : 30 sec ; 72°C : 30 sec) puis 4°C. Les amplicons sont analysés qualitativement par électrophorèse sur gel d'agarose (1,5%, TBE 0,5X). Le volume déposé est de 5 µL et la migration s'effectue durant 20 min à 100 Volts (V). La visualisation des produits PCR est réalisée sous lampe UV après coloration au bromure d'éthidium.

Les conditions pour la culture, l'extraction des Mycobactéries ainsi que les amorces d'amplification sont données dans la demande de brevet WO-A-99/65926.

### Exemple 6 : Réactivité des réactifs de marquage sur des nucléotides modèles

La synthèse des réactifs de marquage est décrite dans les exemples 1 à 4 ci-dessus. Le PDAM décrit dans la présente invention est commercialement disponible. (référence P1405, Molecular Probes, Eugene, OR)

### Exemple 6.1 : Marquage des monomères UMP 3'-phosphate :

La réactivité des réactifs de marquage portant une fonction diazométhyle a été étudiée pour contrôler la spécificité de la réaction.

Un protocole a été mis au point qui consiste à étudier cette réactivité par électrophorèse capillaire sur un composé modèle le 3'-UMP (Uridine 3'monophosphate, référence U1126 Sigma) dans les conditions standards suivantes :
3'-UMP 0,04 mM ; H₃BO₃ 2,0 mM ; Marqueur 2,0 mM [ajouté avec un solvant organique approprié (THF, AcOEt ou DMSO)] ; Solvants H₂O - CH₃CN -Solvant organique (rapport : 1/3/1).

On répartit cette solution en dix fractions de 250 µL que l'on chauffe à 60 °C. Après une durée définie, chaque fraction est traitée avec 250 µL de dichlorométhane. Après agitation, on élimine la phase organique (phase inférieure). On répète cette opération encore deux fois. Après centrifugation (5 min, 5000 tours par minutes (rpm)), la phase aqueuse est analysée par électrophorèse capillaire. Les conditions de l'électrophorèse capillaire (EC) sont les suivantes : l'analyse EC a été effectuée par l'appareil Beckman P/ACE 5000. Un capillaire de silice fondue non traité (75 µm × 50 cm) a été utilisé. Le voltage appliqué est de 30 kV (polarité normale) et la température du capillaire a été maintenue à 23 °C. Les électrophorégrammes ont été enregistrés à 254 nm. La solution tampon borate (0,1 M, pH 8,3) a été préparée à partir de l'acide borique en ajustant le pH avec une solution NaOH et filtrée à travers un filtre 0,2 µm. Les échantillons ont été injectés sous pression (0,5 psi, 5 secondes). Chaque analyse est précédée de la régénération du capillaire par passages successifs d'une solution de NaOH (0,1 M, 2 min), eau (2 min) et de tampon borate (2 min) sous pression (20 psi).

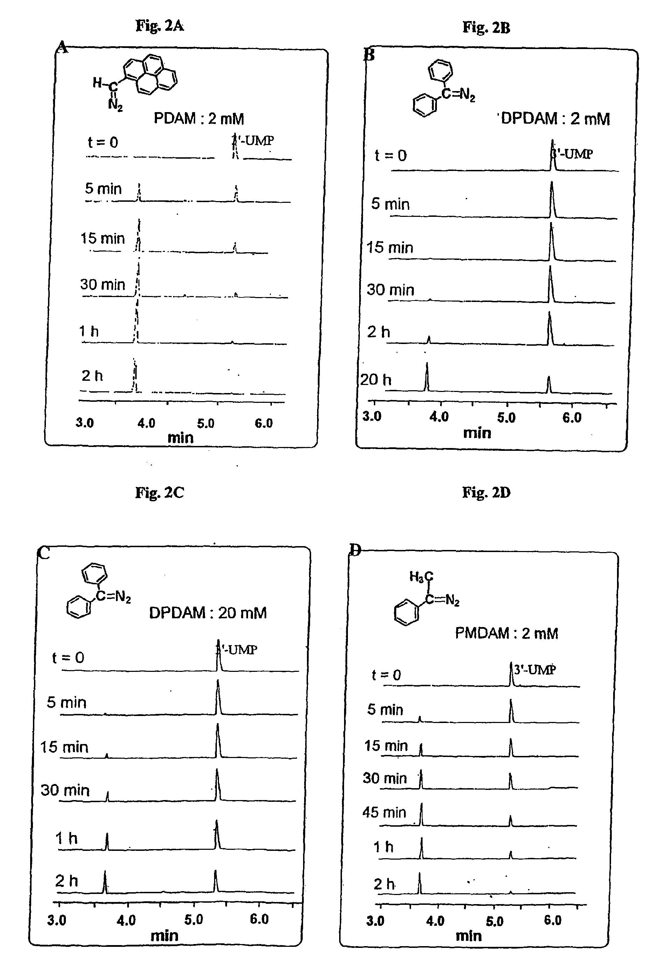

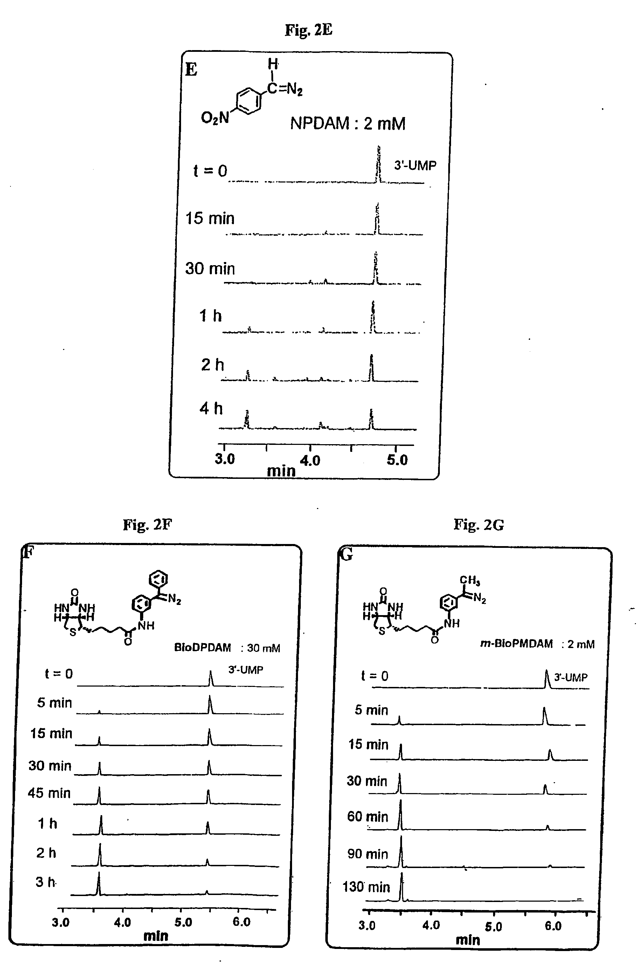

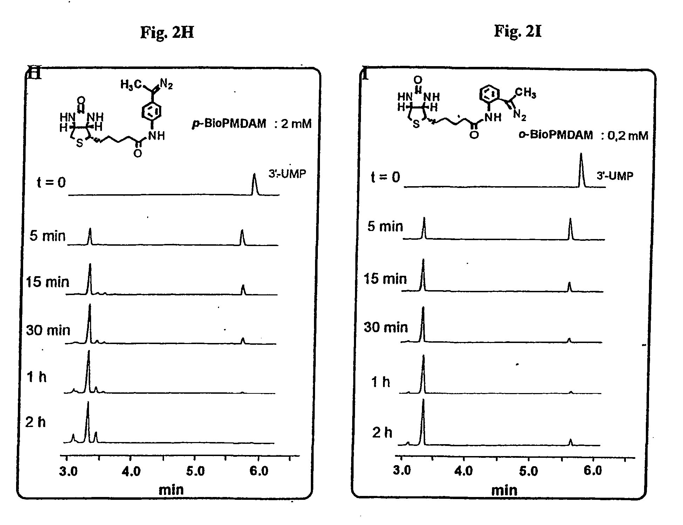

La réaction est effectuée en faisant varier le temps de réaction entre 0 et 4 heures, comme indiqué sur chaque figure, et les résultats sont présentés pour chaque réactif testé sur les figures 2A à 2I avec la concentration en réactif utilisé.
Le temps de réaction est indiqué sur chaque électrophorégramme.
Avec tous les réactifs testés, on obtient la formation exclusive d'un produit monoalkylé, ce qui prouve la spécificité de la réaction.
La réactivité, c'est-à-dire le temps de demi réaction du réactif sur le 3'-UMP, peut être ainsi calculée, par comparaison de la hauteur des pics, et les résultats sont présentés dans le tableau 1 ci-dessous (conditions standards décrites ci-dessus) :

**Tableau 1 : Réactivité (temps de demi réaction) de réactifs**

| | | | | |
|---|---|---|---|---|
| Formules chimiques et noms | 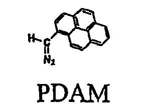 | 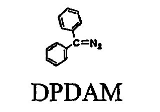 | 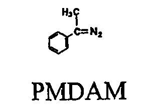 | 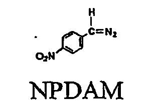 |
| Réactivité | 5 min | 20 h | 30 min | 4h |
| Formules chimiques et noms | 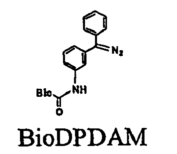 | 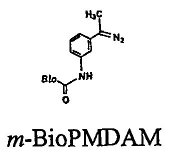 | 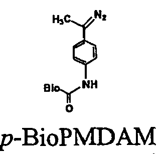 | 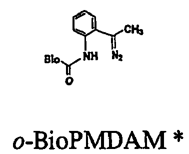 |
| Réactivité | 10 h | 15 min | 5 min | 1 min |

| | | | | |
|---|---|---|---|---|
| * Avec le *o*-BioPMDAM, la réactivité est estimée car dans les conditions de concentration de 2 mM en réactif de marquage, la réaction est complète en moins de 5 min. La figure 2I utilise donc une concentration de 0,2 mM. | | | | |

On peut néanmoins noter que, la réaction étant très spécifique et ne conduisant pas à des sous produits pour tous les réactifs testés, il est possible d'augmenter la concentration du réactif de marquage sans conséquence du point de vue de la sélectivité sur le marquage.
Ainsi pour le réactif DPDAM, si l'on augmente la concentration à 20 mM (voir figure 2C), la réactivité (temps de demi réaction) est de 2 heures. Le même résultat est obtenu avec le BioDPDAM (figure 2F) où la réactivité est de 45 min à une concentration de 30 mM.

### Exemple 6.2 : Test de différents nucléotides 3'-monophosphate :

De manière à éviter toute erreur d'interprétation, une étude complémentaire sur le marqueur *méta*-BioPMDAM, pris à titre d'exemple significatif, a été effectuée avec les autres nucléotides 3'-monophosphate.

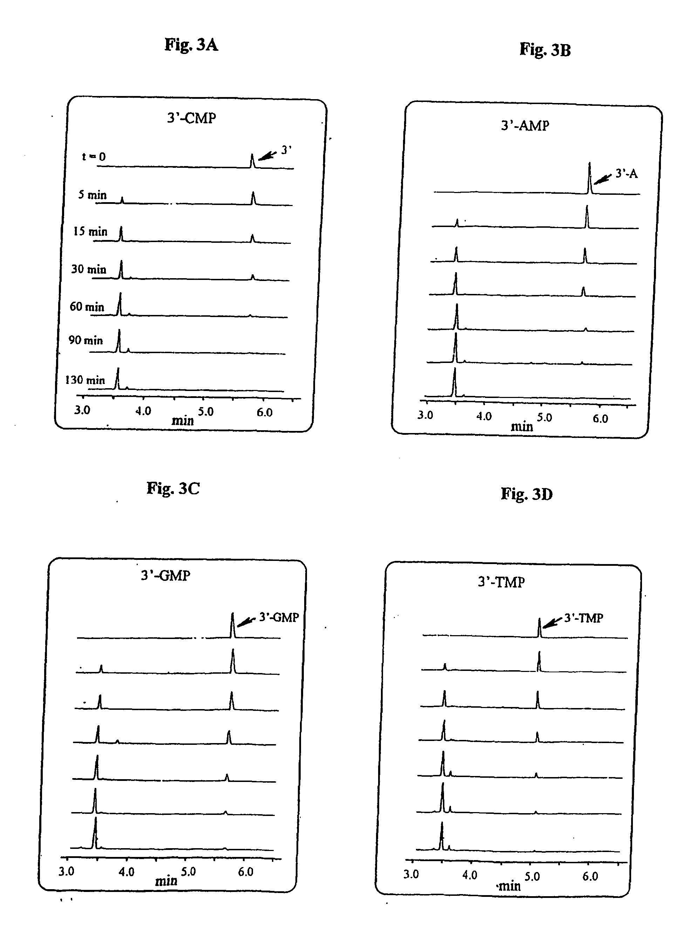

Les nucléotides testés sont les suivants : 3'-AMP (référence 85,194-9, Aldrich), 3'-GMP (référence 151214, ICN), 3'-CMP (référence C1133, Sigma), 3'-TMP (série désoxyribo) (référence T1008, Sigma). Les électrophorégrammes obtenus avec les différents nucléotides sont représentés sur les figures 3A à 3D. Les temps de réaction indiqués sur la figure 3A sont identiques pour les figures 3B à 3D.

Quel que soit le nucléotide de départ (série ribo ou désoxyribo), nous observons la formation exclusive et complète du produit alkylé en 130 min à 60°C. Il est important de noter que dans le cas de la guanine (base la plus réactive avec les réactifs alkylants usuels), seul le produit alkylé au phosphate est observé prouvant la très grande sélectivité de la réaction.
Cette étude permet également de vérifier que la vitesse de la réaction ne dépend pas de la nature de nucléotide en tant que substrat.

### Exemple 6.3 : Etude d'un dinucléotide :

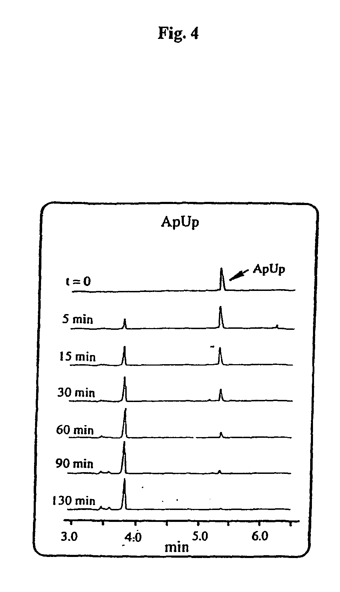

L'alkylation du dinucléotide ApUp (référence A4298, Sigma) a été effectuée avec le *méta-*BioPMDAM afin de vérifier la sélectivité de la réaction vis-à-vis du phosphate terminal par rapport au phosphate internucléotidique. Le suivi de la réaction par électrophorèse est représenté sur la figure 4. Les conditions sont les conditions standards de l'exemple 6.1 déjà décrit.

La formation exclusive d'un seul produit est observée montrant une bonne sélectivité du réactif *méta*-BioPMDAM vis-à-vis du phosphate terminal par rapport au phosphate internucléotidique.

### Exmple 6.4: Caractérisation des adduits avec les quatre (4) nucléotides 3'-monophosphates :

Pour s'assurer que les produits obtenus provenaient bien d'une alkylation au phosphate, la synthèse des adduits des monophosphates 3'-UMP, 3'-CMP, 3'-GMP et 3'-AMP avec le réactif *méta*-BioPMDAM a été effectuée. La réaction d'alkylation est réalisée à l'échelle préparative comme indiqué ci-dessous. Les adduits, obtenus avec des rendements de l'ordre de 70 % sont purifiés puis étudiés par RMN du proton et du phosphore.

### Protocole de préparation :

On dissout le 3'-UMP (sous forme de sel disodique ; 9,3 mg ; 21,1 µmol) dans 2 mL d'une solution aqueuse 0,1 M de H₃BO₃, puis on ajoute successivement 2 mL de CH₃CN, 6 mL de MeOH puis le réactif *méta*-BioPMDAM (75 mg ; 0,20 mmol). La réaction est réalisée pendant 2,5 h à température ambiante. Elle est suivie par électrophorèse capillaire. On ajoute 3 mL d'eau, puis l'excès du réactif est éliminé par extraction avec CH₂Cl₂. La phase aqueuse est évaporée. Le résidu est dissous dans une petite quantité d'eau et purifié par passage sur une colonne de gel de silice en phase inverse (Lichroprep RP-18, Merck ; élution MeOH/H₂O (20/80)). On obtient 10 mg (69 %) de l'adduit du 3'-UMP.

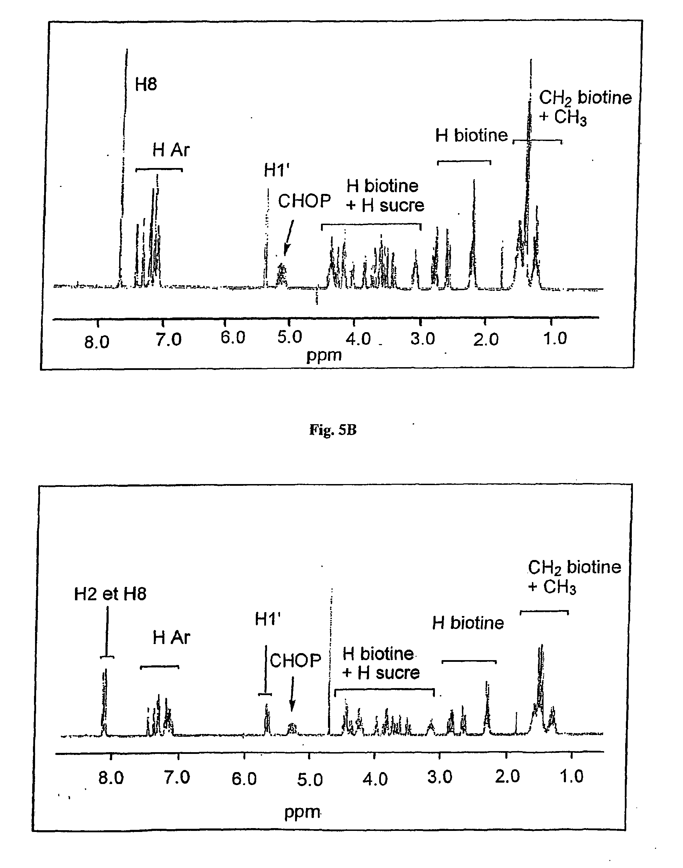

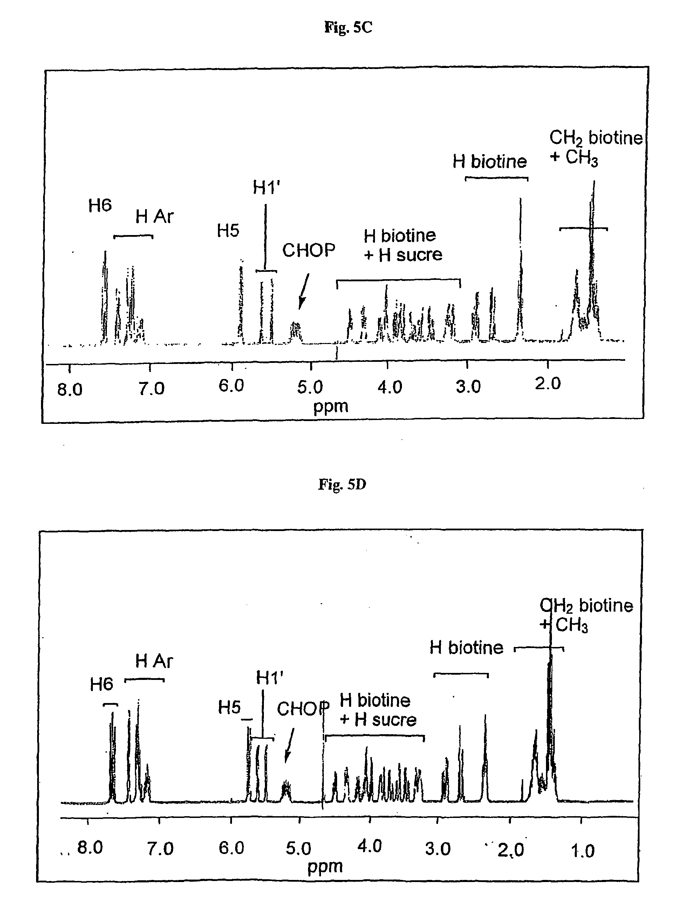

Les spectres de RMN du proton obtenus pour les adduits du 3'-NMP (N= G, U, C, A) sont présentés sur les figures 5A à 5D. L'identification des adduits a été réalisé par des expériences de RMN à deux dimensions ¹H/¹H (COSY). Deux diastéréoisomères pour chacun de ces adduits dans un rapport 1/1 sont présents.
Un seul pic est présent pour la RMN du phosphore vers 0 ppm (300 MHz, D₂O).
Ces expériences démontrent que la réaction est bien spécifique, qu'un seul adduit est observé et que le marquage a bien lieu sur le phosphore. Il n'y a pas de réaction secondaire d'alkylation sur les bases. Les produits de marquage sont donc particulièrement adaptés pour une étape d'hybridation.

### Exemple 7 : Etude de stabilité à la température :

Tous les dérivés du diazométhane, décrit dans le tableau 1 de l'exemple 6.1 ci-dessus, sont conservé à l'état solide dans le congélateur à -20 °C pendant au moins trois (3) mois et aucune perte de réactivité n'est observée.
La stabilité à température ambiante sur la paillasse a été déterminée par RMN ¹H pour les deux réactifs NPDAM et *méta*-BioPMDAM. Nous n'avons observé aucune décomposition en laissant le NPDAM pendant un mois sur la paillasse sans précaution particulière. Nous observons environ 50% de décomposition en laissant le *méta*-BioPMDAM sur la paillasse pendant vingt-cinq (25) jours.
La stabilité à la température du réactif de marquage est une caractéristique essentielle. En effet, la destination finale d'un tel réactif pour une application industrielle est un kit de marquage. Un réactif qui n'est pas stable au moins pendant quinze (15) jours à -20°C, de préférence un (1) mois est invendable. Même s'il existe des moyens de stockage et d'envoi jusqu'à -110°C, il existe une relation entre la stabilité à -110°C et à -20°C et c'est pourquoi la valeur de quinze (15) jours à -20°C, de préférence un (1) mois à -20°C est un minimum industriel. Au delà de -110°C, les laboratoires ne disposent pas des équipements nécessaires (congélateur) pour stocker ces réactifs du point de vue de l'utilisateur et du fabricant, et il n'y a pas de moyen simple type carboglace pour les expédier du point de vue du fabricant.
En ce qui concerne la stabilité à température ambiante, une stabilité de quelques heures, de préférence de un (1) jour, est suffisante pour permettre à l'utilisateur d'effectuer le marquage.

### Exemple 8 : Etude de la fragmentation de l'ADN :

### Exemple 8.1 : Hydrolyse de différents nucléosides en milieu acide :

Le but de cette étude est de démontrer la différence en terme de stabilité à pH acide entre les nucléosides naturels, les nucléosides modifiés ainsi que les nucléosides de type puriniques et pyrimidiniques. Cette étude permet également de mieux contrôler la fragmentation de l'ADN en tenant compte de sa composition en bases.

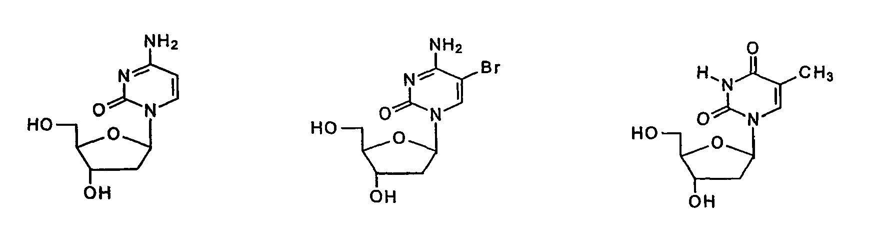

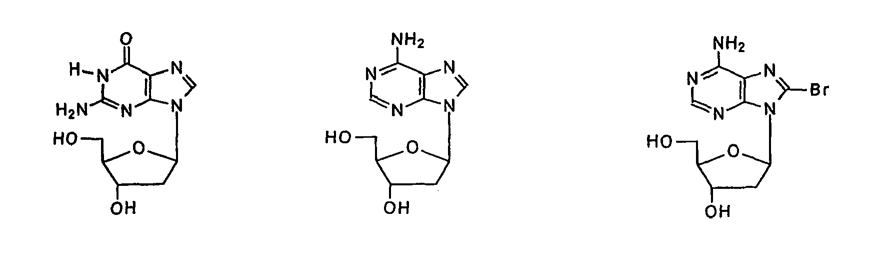

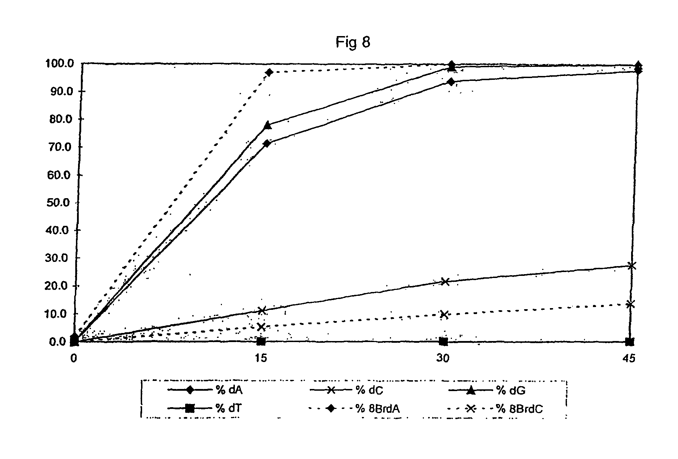

Deux nucléosides modifiés, la 8-bromo-2'-désoxyadénosine (8-BrdA) et la 5-bromo-2'-désoxycytidine (5-BrdC) ainsi que les quatre (4) nucléosides naturels (dA, dC, dG et dT) ont été utilisés dans cette étude.
50 nanomoles (nmol) de chaque nucléoside sont incubées dans 50 mM de formiate de sodium pH 3 à 95°C. Les temps d'incubation varient de 0 à 45 min. Après séchage sous vide et reprise par 20 µl d'eau pure, les échantillons (10 nmol) sont alors analysés par HPLC en phase inverse. Les résultats sont donnés sous forme de pourcentage d'hydrolyse du nucléoside de départ (en ordonnée) par rapport au temps d'incubation en minutes (en abscisse) voir figure 8.

Les courbes de la figure 8 montrent que la modification de l'adénine en position 8 par un atome de brome rend ce nucléoside moins stable que le nucléoside naturel. D'autre part, les résultats montrent que, dans les conditions utilisées, la dépurination est beaucoup plus importante que la dépyrimidination.
Cette étude montre que la fragmentation de l'ADN par la dépurination ou la dépyrimidination peut être contrôlée en optimisant les conditions d'hydrolyse, ou en incorporant soit des bases modifiées moins stables que les bases naturelles soit des bases pouvant être modifiées et hydrolysées après leur incorporation.

### Exemple 8.2 : Fragmentation de l'ADN double brin incorporant ou non un nucléotide modifié :

Trois amplifications PCR ont été réalisées en parallèle à partir de cible ADN génomique *Mycobacterium tuberculosis* 16S (10⁺⁴ copies de départ) en utilisant le kit *Fast Start* de Roche, 0,2 mM de chaque désoxyribonucléotide (d-ATP, d-CTP, d-GTP, d-TTP), 0,3 µM d'amorces et 0,4 µL d'enzyme.
Les paramètres de la PCR sont ceux de l'exemple 5.
Dans le premier cas, le protocole est utilisé tel quel : cas de la PCR dite naturelle.
Dans le deuxième cas, le protocole est modifié pour obtenir une PCR à 30 % de 8Br-dATP. Ceci est réalisé en introduisant 0,2 mM de d-CTP, d-GTP et d-TTP. On introduit également 0,14 mM de d-ATP et 0,06 mM de 8-BrdATP. (La 8-BrdATP est commerciale (référence N-2005-1, TriLink Biotechnologies, San Diego CA).

Dans le troisième cas, le protocole est modifié pour obtenir une PCR à 50 % de 8-BrdATP. Ceci est réalisé en introduisant 0,2 mM de d-CTP, d-GTP et d-TTP. On introduit également 0,1 mM de d-ATP et 0,1 mM de 8-BrdATP.

L'étude de la fragmentation seule de ces amplicons a été réalisée dans les conditions décrites ci-dessus : 50 mM de formiate de sodium pH 3 à 95°C.
L'analyse a été effectuée sur gel dénaturant de polyacrylamide (8 % polyacrylamide, 7 M urée, 1 X TBE) utilisant coloration au bromure d'éthidium.

Après 15 min d'incubation à 95 °C dans 50 mM de formiate de sodium pH 3, aucune différence n'est visible entre les trois (3) cibles. Dans tous les cas, nous avons observé une fragmentation totale des amplicons PCR.

La dépurination des amplicons PCR a été également réalisée à différents pH et à différentes températures et temps d'incubation. L'analyse sur gel, dans les conditions ci-dessus, montre qu'à pH 3, la fragmentation est totale après seulement 10 min d'incubation à 95°C. A ce pH, la fragmentation est aussi totale à 60°C après 30 min d'incubation.
A pH 4, 30 min d'incubation sont nécessaires pour l'obtention d'une fragmentation totale des amplicons ADN même à 95°C. Ce résultat est très intéressant et montre que le site abasique généré à pH acide est instable et donc conduit à la fragmentation de la chaîne ADN sans aucun autre traitement particulier.

### Exemple 9 : Marquage et fragmentation de l'ADN par le dérivé méta-bioPMDAM (3a) en deux étapes :

Le dérivé *méta*-bioPMDAM (3a) a été obtenu selon le schéma réactionnel décrit dans l'exemple 1.1.
Les amplicons ADN ont été préparés par amplification PCR selon le protocole décrit dans l'exemple 5.

### Marquage

A 10 µl de PCR, 38 µL d'eau pure (sigma), 50 µL de formiate de sodium à pH 3 (100 mM dans l'eau pure) sont ajoutés et le mélange est incubé pendant 30 minutes à 60°C. Puis 2 µL de *méta*-bioPMDAM (100 mM dans DMSO) sont ajoutés ensuite. La solution a été mélangée par vortex, puis incubée 15 minutes supplémentaires à 60°C.
Les essais sont réalisés en duplicata afin de pouvoir analyser la fragmentation de l'ADN sur gel et l'efficacité de marquage par hybridation et lecture de la puce à ADN.

### Purification

La purification est réalisée sur colonnes QIAQUICK^{™} (Nucleotide Removal kit, Qiagen, Hilden, Allemagne). Le protocole de purification utilisé est celui recommandé par le fournisseur.

Après purification, l'éluat est transféré dans un tube propre contenant 400 µL de tampon d'hybridation (1,75 mL 20X SSPE ; 2,9 mL Bétaine 5M ; 290 µL DTAB 0,1M ; 10 µL Antimousse 30%) sont ajoutés. Les références de ces substances sont, pour :
- la bétaine référence B-2754 Sigma, et
- la DTAB référence D-5047 Sigma.
La solution est mélangée par vortex et incubée 10 min à 95°C, afin de séparer les brins d'ADN qui ne sont pas séparés lors de l'étape de marquage pendant la fragmentation (étape de dénaturation). Le tube est ensuite plongé dans un mélange eau-glace à 0°C avant hybridation sur puce à ADN.

### Hybridation sur puce à ADN

Après l'étape de marquage pendant la fragmentation, les fragments obtenus sont hybridés sur les puces à ADN conçues pour l'analyse de la région 213-415 de la séquence M20940 « Genbank » de l'ARN 16S de *Mycobacterium tuberculosis.* Cette puce à ADN est décrite dans A. Troesch et al, J. Clin Microbiol., 37(1), p49-55, 1999.
Les étapes d'hybridation ont été réalisées sur les stations fluidiques (Affymetrix, Santa Clara, CA) en utilisant le protocole d'hybridation et les tampons décrits dans A. Troesch et al, J. Clin Microbiol., 37(1), p49-55, 1999. Une étape supplémentaire est nécessaire pour révéler la biotine (détection indirecte).

L'hybridation est révélée par le couplage de la streptavidine marquée à la phycoérythrine (PE) qui interagit avec la biotine de la *méta*-BioPMDAM dans les conditions suivantes : 300 µL d'eau pure ; 300 µL de tampon Tris 100 mM pH 7 / NaCl 1M / tween 0,05% /Antimousse 0,005% ; 6 µL de BSA (50 mg/mL) ; 6 µL de streptavidine-PE (300 µg / mL). Les références de ces substances sont, pour :
- la Streptavidine-Phycoérythrine : référence R0438, Dako, Danemark,
- la Streptavidine-CY5 : référence C0050, Dako, Danemark,
- l'Antimousse référence M5-575, Ultra Additives Inc., et
- le Tween référence P-7949, Sigma.

### Lecture de la puce à ADN :

La lecture de la fluorescence émise à la surface de la puce à ADN après marquage et hybridation ainsi que la génération des données en termes d'intensité du signal et du pourcentage d'homologie sont réalisés par les systèmes de lecture et le logiciel fournis par Affymetrix (GeneChip® Instrument System et GeneChip® Information System, Santa Clara CA).
Le système de lecture fournit des intensités en signal et bruit de fond exprimées en rfu (« relative fluorescence unit »). Le pourcentage d'homologie est donné par rapport à une séquence référence qui dans ce cas est la séquence de *Mycobacterium tuberculosis.*
Les résultats en termes d'intensité moyenne du signal (I), du bruit de fond (B) et du pourcentage d'homologie (%) sont donnés dans le tableau 2 ci-dessous :
De manière générale, on considère qu'un pourcentage d'homologie supérieur à 90% est un résultat satisfaisant bien que l'on cherche en général un résultat supérieur à 95%. Au dessus de 95%, les valeurs ne sont plus indiquées car elles ne sont pas significatives dans le cas de la puce à ADN Mycobactéries. Une intensité élevée avec un bruit de fond faible est le deuxième résultat recherché dans les exemples qui suivent. Dans tous les résultats, le bruit de fond B est déduit de l'intensité moyenne I.

### Analyse sur gel de polyacrylamide

Les échantillons destinés à être analysés sur gel sont séchés sous vide, repris par 10 µL d'eau pure et 10 µL de bleu formamide 2X.
La migration est effectuée sur gel d'acrylamide 8%, dans du TBE 1X, une (1) heure à 150 V.

Le pH acide a été utilisé pour la fragmentation de l'ADN. En effet à ce pH, le phénomène de dépurination engendre des sites abasiques très instables conduisant à une fragmentation presque immédiate des séquences ADN à température élevée. Ce type de fragmentation produit des fragments ADN-5' phosphate.

L'analyse sur gel montre que l'incubation des amplicons PCR à 60°C pendant 30 min en solution dans le tampons formiate (50 mM, pH 3) conduit à une fragmentation totale de ces amplicons. Ceci nous a permis d'évaluer le marquage pendant la fragmentation des amplicons ADN en présence du marqueur *méta*-bioPMDAM.

Les résultats de marquage pendant la fragmentation des amplicons ADN en termes de pourcentage d'homologie, d'intensité des signaux et du bruit de fond sont donnés dans le tableau 2 ci-dessous.

**Tableau 2 : Marquage et fragmentation des amplicons ADN en termes d'homologie, d'intensité des signaux (I) et du bruit de fond (B)**

| **Conditions de marquage des amplicons PCR** | **Homologie (%)** | **I (rfu)** | **B (rfu)** | **I/B** |
|---|---|---|---|---|
| Tampon : formiate pH3, 50 mM | | | | |
| Marqueur : 2 mM *méta*-bioPMDAM | > 95 | 4456 | 593 | 7,5 |
| Incubation : 30 min à 60°C | | | | |

Cet exemple montre que les dérivés de l'invention peuvent être utilisés pour le marquage des fragments ADN produits par amplification enzymatique dans un protocole en deux étapes. Ils peuvent être également utilisés pour le marquage de l'ADN naturel non amplifié.

### Exemple 10 : Marquage de l'ADN par le dérivé Cy5-PMDAM (12bis) :

Le marquage de l'ADN par ce nouveau marqueur portant le fonction diazométhyle a été évalué en utilisant un fragment ADN synthétique.

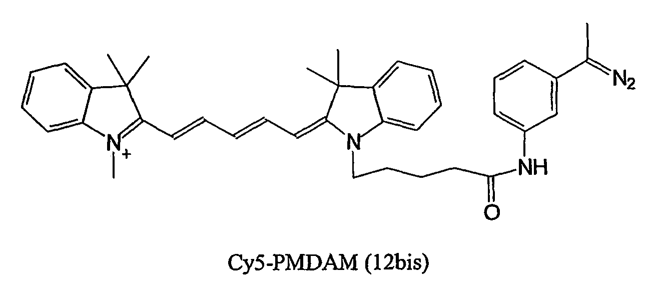

Le réactif de marquage Cy5-PMDAM (12bis) est préparé selon le protocole décrit dans l'exemple 2.
Un oligodésoxyribonucléotide (ODN) vingt (20) mers est préparé selon la méthode dite au phosphoramidite. On introduit un phosphate à l'extrémité 5' par un réactif de phosphorylation standard compatible avec la chimie au phosphoramidite. La séquence de cet ODN est constituée de toutes les bases naturelles de l'ADN (séquence de l'ODN : 5'-CTGAACGGTAGCATCTTGAC-3'). Cette séquence est complémentaire de séquences de capture d'une puce à ADN dite « modèle », synthétisée selon la technologie Affymetrix. Cette puce à ADN contient des sondes de capture identiques en séquence et qui sont réparties en damier sur sa surface. La lecture de cette puce à ADN donne des informations quant à la performance du marquage en terme d'intensité mais pas de résultat d'homologie.

Marquage : A 50 picomoles (pmoles) de cet ODN, 10 µL de Cy5-PMDAM (100 mM dans le DMSO) sont ajoutés. Le volume final est de 100 µL. Après homogénéisation, l'incubation est réalisée à 60°C pendant 30 minutes.

Purification et lecture : La purification pour éliminer l'excès de réactif de marquage est réalisée selon l'exemple 9. La lecture sur puce à ADN est effectuée selon l'exemple 17.

### Résultats :

La moyenne des intensités de marquage (I) lue sur la puce à ADN est de 16 644 rfu pour un bruit de fond (B) de 450 rfu.
Ce niveau d'intensité est très élevé et montre que le réactif de marquage Cy5-PMDAM (12bis) est tout à fait compatible avec le marquage des fragments d'ADN sur le groupement phosphate.

### Exemple 11 : Marquage et fragmentation de l'ADN par le réactif PDAM :

### Exemple 11.1 : Marquage par le 1-Pyrényldiazométhane (PDAM) :

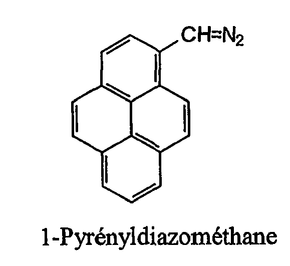

Le PDAM est obtenu chez Molecular Probes (Eugene, OR) et solubilisé dans du DMSO anhydre.
Deux ODN de vingt (20) mers ont été utilisés comme modèles ADN : un ODN de 20mer 5'-hydroxyle et le même ODN de 20mer portant un phosphate à l'extrémité 5'. La séquence de l'ODN est décrite dans l'exemple 10. La réaction de marquage est réalisée dans un mélange à 50 % en DMSO et 1,5 mM en 1-Pyrényldiazométhane (PDAM) à 60°C pendant 30 minutes ou une heure.
L'efficacité du marquage a été évaluée par chromatographie sur couche mince (en phase normale) dans un éluant isopropanol/ammoniaque/eau 60/10/30. Après 30 minutes, le couplage est total sur l'ODN 5'-phosphate. Il faut une heure pour obtenir un couplage partiel sur l'ODN 5'-hydroxyle c'est à dire environ 50%.

Les résultats de l'exemple 6 sont confirmés sur une séquence modèle de 20 bases en ce qui concerne le marquage préférentiel des réactifs portant une fonction diazométhyle sur le phosphate terminal. Le marquage sur un phosphate intranucléotidique n'est pas un inconvénient rédhibitoire puisque cela peut conduire à une augmentation de la sensibilité par introduction de plus d'un marqueur sur le fragment d'acide nucléique. Cela permet à l'acide nucléique de s'hybrider avec une bonne sensibilité sur la cible complémentaire tout en conservant une bonne spécificité d'hybridation.
L'homme du métier par des réactions d'optimisation peut ainsi contrôler la spécificité du marquage, en jouant par exemple sur le réactif de marquage, le temps de réaction et la température, pour avoir un marquage exclusif sur le phosphate terminal.

### Exemple 11.2 : Etude cinétique de la réaction de marquage par le PDAM :

Cette étude a été réalisée en utilisant l'ODN 20mer 5'-phosphate dans les conditions précédentes en faisant varier le temps de réaction. Les rendements de marquage ont été évalués par analyse chromatographie liquide haute pression (CLHP) en phase inverse dans les conditions suivantes :
Phase inverse colonne Spheri-5 RP-18 5 µm, 220x 4,6 mm (Perkin Elmer). Les tampons et le gradient utilisés sont :
   - Tampon A : 0,1 M TEAA ; Tampon B = 50% Tampon A + 50 % CH₃CN, et
   - Gradient de 10 à 50 % de B en 30 min à 1 ml/min à température ambiante.

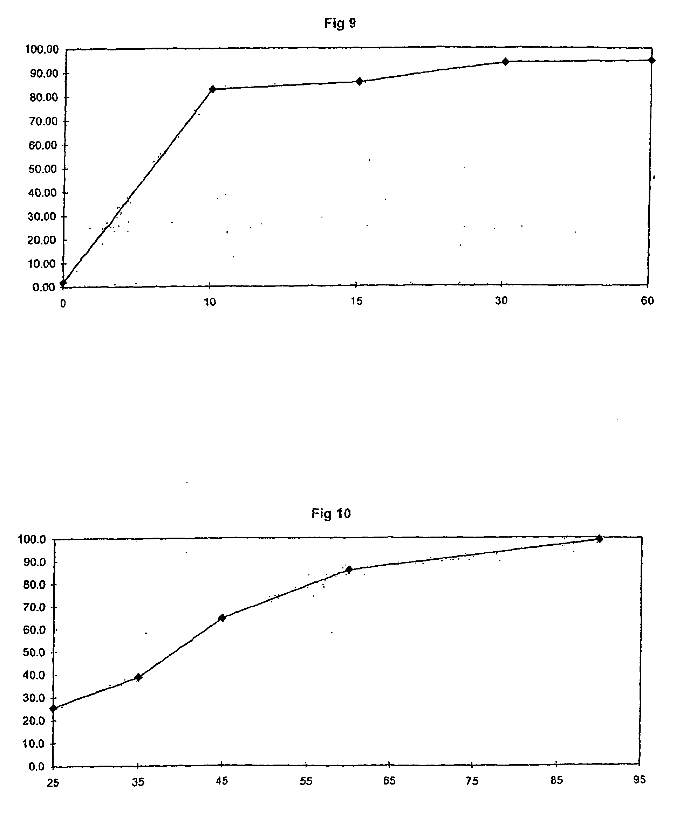

Les résultats sont représentés sur la figure 9 avec en abscisse le temps de réaction, exprimé en minutes, et en ordonnée le pourcentage de marquage.
Le rendement est proche de 90% après seulement 10 min d'incubation à 60°C.

### Exemple 11.3 : Effet de la température sur le marquage au PDAM :

Le marquage a été réalisée en utilisant l'ODN 20mer 5'-phosphate dans les conditions précédentes en faisant varier la température d'incubation et avec un temps d'incubation de 10 minutes dans chaque cas.

Les rendements de marquage ont été évalués par analyse CLHP en phase inverse.
Les résultats sont présentés sur la figure 10 avec en ordonnée le pourcentage de marquage et en abscisse la température de réaction en °C
Il est très important de noter que même à température ambiante (25°C), nous observons un marquage de l'ODN. Après 10 minutes d'incubation à 25 °C, le rendement de marquage est environ de 25%. A des températures supérieures à 50°C, des rendements supérieurs à 80% sont obtenus.
Ceci montre l'efficacité et la flexibilité de cette chimie de marquage de l'ADN par les réactifs portant une fonction diazométhyle.

### Exemple 12 : Marquage et fragmentation des amplicons ADN obtenus par amplification PCR avec le réactif de marquage méta-bioPMDAM (3a) :

Le dérivé *méta*-bioPMDAM (3a) a été obtenu selon le schéma réactionnel décrit dans l'exemple 1.1.
Les amplicons ADN ont été préparés par amplification PCR selon le protocole décrit dans l'exemple 5.

### Exemple 12.1 : Comparaison du marquage avec et sans fragmentation :

### a. Marquage dans les conditions de fragmentation :

A 10 µL de PCR sont ajoutés 50 µL de formiate de sodium pH 3 (50 mM) et 2 µL de *méta-*BioPMDAM 100 mM dans du DMSO). Le volume est ajusté à 100 µL. La solution est incubée 30 min à 60°C.

### b. Marquage sans fragmentation :

A 10 µL de PCR sont ajoutés 2 µL de *méta*-BioPMDAM (100 mM dans du DMSO). Le volume est ajusté à 100 µL. La solution est incubée 30 min à 60°C.

Le reste du protocole est identique à celui de l'exemple 9.

### Résultats

**Tableau 3 : Comparaison du marquage avec et sans fragmentation**

| **Protocole utilisé** | **Homologie (%)** | **I (rfu)** | **B (rfu)** | **I/B** |
|---|---|---|---|---|
| *a. Marquage dans les conditions de fragmentation* | >95 | 3995 | 569 | 7,0 |
| *b. Marquage sans fragmentation* | 94,1 | 500 | 542 | 0,9 |

Les résultats dans le tableau 3 ci-dessus montre que sans fragmentation, la moyenne des intensités obtenues est au même niveau que le bruit de fond (500 rfu). Le marquage pendant la fragmentation donne un niveau d'intensité bien supérieur (environ 4000 rfu) et un très bon pourcentage d'homologie. La combinaison des deux étapes représente donc bien une amélioration significative pour la détection d'un acide nucléique de longueur supérieure à cent nucléotides.

### Exemple 12.2 : Effet de la dénaturation avant hybridation sur puce à ADN :

Deux réactions de marquage ont été réalisées en parallèle dans deux tubes séparés selon le protocole suivant : A 10 µL de PCR sont ajoutés 50 µL de tampon formiate de sodium pH 3 (50 mM) et 2 µL de *méta-bio*PMDAM (100mM dans du DMSO). Le volume total est ajusté à 100 µL et incubé 30 min à 60°C.
Après purification sur colonne (exemple 9), la solution issue du premier tube est incubée 10 min à 95°C (afin de désapparier le double brin d'ADN), puis le tube est plongé dans un mélange eau-glace à 0°C jusqu'à l'hybridation sur la puce ADN.
La solution issue du deuxième tube est hybridée sur la puce à ADN sans dénaturation préalable.

Les fragments biotinylés hybridés sur les sondes de capture à la surface de la puce à ADN sont révélés par l'introduction d'une streptavidine marquée par la phycoérythrine en utilisant les conditions décrites dans l'exemple 9.

### Résultats

**Tableau 4 : Effet de la dénaturation avant hybridation sur puce à ADN**

| **Conditions utilisées** | **Homologie (%)** | **I (rfu)** | **B (rfu)** | **I/B** |
|---|---|---|---|---|
| *Avec dénaturation* | >95 | 22812 | 570 | 40,1 |
| *Sans dénaturation* | 93,5 | 4795 | 681 | 7,0 |

Les résultats obtenus, présentés dans le tableau 4, avec la dénaturation pré-hybridation sont supérieurs à ceux obtenus sans l'étape de dénaturation. Ceci montre que la dénaturation de l'ADN est nécessaire pour obtenir un bon niveau d'intensité. La fragmentation via les sites abasiques est un moyen pour faciliter la dénaturation d'un ADN double brin et renforcer l'hybridation sur les sondes de captures.
Pour tester d'autres réactifs de marquage et compte tenu des résultats obtenus avec les différentes conditions ci-dessus, nous avons défini un protocole de référence utilisant la fragmentation au tampon formiate de sodium (50 mM, pH 3) et un étape de dénaturation pré-hybridation.

### Exemple 13 : Marquage et fragmentation des amplicons PCR par les réactifs biotinylés dans un protocole en une étape :

Les dérivés *méta*-, *ortho-,* et *para*-bioPMDAM ont été préparés selon le protocole décrit dans les exemples 1.1, 1.2 et 1.3. Ils ont été solubilisés dans du DMSO anhydre à une concentration de 100 mM.

Le protocole est identique à celui de l'exemple 12.2 ci-dessus (marquage et fragmentation en une étape puis étape de dénaturation pré-hybridation).

### Résultats

**Tableau 5 : Marquage et fragmentation des amplicons PCR par les réactifs biotinylés dans un protocole en une étape**

| **Marqueur** | **Homologie (%)** | **I (rfu)** | **B (rfu)** | **I/B** |
|---|---|---|---|---|
| *ortho*-bioPMDAM | >95 | 25951 | 820 | 31,6 |
| *méta*-bioPMDAM | >95 | 22960 | 581 | 39,5 |
| *para*-bioPMDAM | 94,1 | 43785 | 1205 | 36,3 |

Le protocole optimisé avec fragmentation et marquage en une étape donne d'excellents résultats avec différents réactifs de marquage avec la fonction réactive diazométhyle, comme le montrent les résultats exposés dans le tableau 5.

### Exemple 14 : Marquage et fragmentation de l'ADN par le BioDPDAM :

Les amplicons ADN ont été préparés par amplification PCR selon le protocole décrit dans l'exemple 5.
La synthèse du réactif de marquage est décrite dans l'exemple 1.
Le protocole est identique à celui de l'exemple 12.2, y compris la dénaturation à 95 °C avant l'étape d'hybridation.

### Résultats

**Tableau 6: Marquage et fragmentation de l'ADN par le BioDPDAM**

| **Marqueur utilisé** | **Homologie (%)** | **I (rfu)** | **B (rfu)** | **I/B** |
|---|---|---|---|---|
| BioDPDAM | 93,0 | 32359 | 3610 | 9,1 |

Ce résultat, décrit dans le tableau 6, montre que des substitutions aussi importantes que le groupement phényle peuvent être utilisées pour optimiser la réactivité des réactifs de marquage portant une fonction diazométhyle.

### Exemple 15 : Marquage et fragmentation de l'ADN par la 5-(bromométhyl)fluorescéine :

Les amplicons ADN ont été préparés par amplification PCR selon le protocole décrit dans l'exemple 5.
A 10 µL de PCR sont ajoutés 50 µL de formiate de sodium pH3 (100 mM) et 2 µL de 5-(bromométhyl)fluorescéine (Molecular probes, Eugen, OR) (100 mM dans du DMSO). Le volume est ajusté à 100 µL. La solution est incubée 30 min à 60°C.

Les conditions de purification sont conformes à celles de l'exemple 9. Une étape de dénaturation est effectuée comme décrit dans l'exemple 12.2.
Les autres conditions d'hybridation et de lecture sont identiques à celles décrites dans l'article de A. Troesch et al. J. Clin. Microbiol., 37(1), p 49-55 1999. La fluorescéine est directement. détectable sur le lecteur.

**Tableau 7 : Marquage et fragmentation de l'ADN par la 5-(bromométhyl)fluorescéine**

| **Protocole utilisé** | **Homologie (%)** | **I (rfu)** | **B (rfu)** | **I/B** |
|---|---|---|---|---|
| *Marquage et fragmentation des amplicons PCR avec la 5-(bromo-méthyl)fluorescéine* | >95 | 855 | 183 | 4,7 |

Ce résultat du tableau 7 montre que la fragmentation de l'ADN par la création de sites abasiques est tout à fait compatible avec un réactif de marquage portant une fonction réactive halogénure d'alkyle. Ce protocole est réalisé en une étape (fragmentation et marquage) mais avec des intensités plus faibles qu'avec les marqueurs portant une fonction diazométhyle.

### Exemple 16 : Marquage et fragmentation des amplicons ADN en présence d'un autre agent chimique de fragmentation dérivé de la phénanthroline :

Des amplicons ADN ont été préparés par amplification PCR selon le protocole décrit dans l'exemple 5. Deux types de conditions sont utilisées :

### Conditions a :

A 10 µL de PCR sont ajoutés 20 µL de phénanthroline-FeSO₄ (25 mM) et 2 µL de *méta-*BioPMDAM (100 mM dans DMSO). Le volume total est ajusté à 100 µL. Le mélange est incubé 60 min à 95°C.

### Conditions b :

A 10 µL de PCR sont ajoutés 50 µL de tampon formiate de sodium pH3 (100 mM dans eau pure) et 2 µL de de *méta*-BioPMDAM (100 mM dans DMSO). Le volume total est ajusté à 100 µL. Le mélange est incubé 60 min à 95°C.

Les autres conditions du protocole sont identiques à celles de l'exemple 9.

**Tableau 8 : Marquage et fragmentation des amplicons ADN en présence de la phénanthroline**

| **Conditions** | **Homologie (%)** | **I (rfu)** | **B (rfu)** | **I/B** |
|---|---|---|---|---|
| Phénanthroline FeSO4(5 mM) *méta*-bioPMDAM (2 mM) 60 min à 95°C | >95 | 2236 | 500 | 4,5 |
| Tampon formiate (50mM) pH3 *méta*-bioPMDAM (2 mM) 60 min à 95°C | >95 | 6786 | 565 | 12,0 |

Les deux conditions de fragmentation permettent un résultat satisfaisant comme démontré dans le tableau 8.
Le meilleur résultat est obtenu avec les conditions (b) utilisant la fragmentation à pH acide.

### Exemple 17 : Fragmentation des amplicons PCR marqués par incorporation de d-UTP-fluorescéine :

### Incorporation du nucléotide marqué

Une amplification PCR a été réalisée selon le protocole suivant afin générer des amplicons PCR marqués par la fluorescéine (marquage sur les bases).
A partir de cible ADN génomique *Mycobacterium tuberculosis* 16S (10⁺⁴ copies) en utilisant le kit *Fast Start* de Roche, 0,2 mM des désoxyribonucléotides d-ATP, d-CTP et d-GTP ainsi que 0,14 mM de d-TTP et 0,06 mM de d-UTP-12-fluoresceine, 0,3 µM d'amorces et 0,4 µL d'enzyme. Le pourcentage du nucléotide marqué par rapport à son homologue naturel d-UTP est de 30%. C'est ce rapport qui est généralement utilisé dans les réactions de marquage des amplicons par incorporation de nucléotides marqués.
La d-UTP-12-fluoresceine est disponible commercialement chez Roche Diagnostics référence 1373242, Mannheim, Allemagne).
Les paramètres de la PCR sont ceux de l'exemple 5.

### a. Fragmentation des amplicons PCR marqués à 30% par d-UTP-fluorescéine :

A 10 µL de PCR sont ajoutés 50 µL de tampon formiate de sodium pH 3 (50 mM). Le volume est ajusté à 100 µL. La solution est ensuite incubée 30 min à 60°C.

### b. Marquage pendant la fragmentation des amplicons PCR contenant 30% par d-UTP-fluorescéine :

A 10 µL de PCR sont ajoutés 50 µL de tampon formiate de sodium pH 3 (50 mM) et 2 µL de *méta*-BioPMDAM (100 mM dans du DMSO). Le volume est ajusté à 100 µL. La solution est ensuite incubée 30 min à 60°C. Cet essai correspond à un protocole de référence qui permet de comparer les différentes stratégies de marquage en s'affranchissant de la variabilité due à l'étape d'amplification.

Une étape de purification sur colonne et une étape de dénaturation à 95°C sont effectuées dans tous les cas comme dans l'exemple 9.

### Protocole (a1) :

Les acides nucléiques obtenus par fragmentation des amplicons marqués à la d-UTP-fluorescéine (conditions *a*)) sont hybridés sur puce à ADN et détectés dans un premier temps par lecture directe des signaux fluorescents émis par la fluorescéine comme décrit dans l'exemple 15.

### Protocole (a2) :

Une étape d'amplification du signal a été utilisée pour améliorer la sensibilité de marquage. L'amplification du signal a été réalisée par l'introduction, lors de l'étape d'hybridation, d'un anticorps anti-fluorescéine biotinylé (référence 216-065-084, Jackson ImmunoResearch) puis d'une streptavidine marquée par la phycoérythrine en utilisant les conditions successives suivantes :
- 300 µL d'eau pure,
- 300 µL de tampon Tris 100 mM pH 7 / NaCl 1M / tween 0,05% / Antimousse 0,005% , 2,4 µL de BSA (50 mg/mL), 1,2 µL d'anticorps anti-fluorescéine biotinylé. (1 mg / mL),
- 300 µL d'eau pure, et
- 300 µL de tampon Tris 100 mM pH 7 / NaCl 1M / tween 0,05% / Antimousse 0,005% ; 6 µL de BSA (50 mg/mL) ; 6 µL de streptavidine-PE. (300 µg / mL).
Dans ce protocole, la fluorescéine agit comme un haptène (traceur détectable indirectement par un anticorps marqué) et non pas comme un fluorophore.

### Protocole (b) :

Les fragments biotinylés (condition *b)* hybridés sur puce à ADN sont révélés par l'introduction d'une streptavidine marquée par la phycoérythrine en utilisant les conditions suivantes :
- 300 µL d'eau pure, et
- 300 µL de tampon Tris 100 mM pH 7 / NaCl 1M / tween 0,05% / Antimousse 0,005% ; 6 µL de BSA (50 mg/mL) ; 6 µL de streptavidine marquée. (300 µg / mL).

La lecture de la fluorescence émise à la surface de la puce à ADN après marquage et hybridation ainsi que la génération des données en termes d'intensité du signal et du pourcentage d'homologie sont réalisés par les systèmes de lecture et le logiciel fournis par Affymetrix. A ce propos, il est important de noter que le système de lecture utilisé contient deux filtres permettant de détecter directement :
- la fluorescéine dans le cas où les amplicons sont marqués au d-UTP-fluorescéine uniquement, selon le protocole al, ou bien
- la phycoérythrine dans le cas où les amplicons sont marqués :
   - au d-UTP-fluorescéine avec amplification du signal, selon le protocole a2, ou
   - par le *méta*-bioPMDAM pendant leur fragmentation, selon le protocole *b*.
Dans les deux cas où la révélation s'effectue par la phycoérythrine, l'utilisation d'un filtre permet de s'affranchir du signal généré par la fluorescéine et c'est bien le signal de la phycoérythrine qui est détecté.

### Résultats

**Tableau 9 : Fragmentation des amplicons PCR marqués par incorporation de d-UTP-fluorescéine**

| **Protocole utilisé** | **Marqueur détecté** | **Homologie (%)** | **I (rfu)** | **B (rfu)** | **I/B** |
|---|---|---|---|---|---|
| *a1. Fragmentation des amplicons PCR marqués par la d-UTP-fluorescéine* | Flu* | 81,6 | 595 | 342 | 1,7 |
| *a2. Fragmentation des amplicons PCR marqués par la d-UTP-fluorescéine avec amplification du signal* | PE* | >95 | 22107 | 3461 | 6,4 |
| *b. Fragmentation et marquage sur les mêmes amplicons PCR modifiés par la d-UTP-fluorescéine et marqués par la méta-BioPMDAM* | PE* | >95 | 21700 | 1503 | 14,4 |

| | | | | | |
|---|---|---|---|---|---|
| * Flu =Fluorescéine et PE = Phycoérythrine | | | | | |

Les résultats, du tableau 9 ci-dessus, montrent que la fragmentation chimique utilisant la création de site abasique est compatible avec le marquage enzymatique des amplicons ADN et que le marquage peut avoir lieu avant la fragmentation.
Les niveaux d'intensité ainsi que le pourcentage d'homologie obtenus avec ce protocole d'incorporation enzymatique du fluorophore sont faibles en comparaison de ceux obtenus avec le marquage pendant la fragmentation utilisant le réactif de marquage avec une fonction diazométhyle comme le *méta*-bioPMDAM (conditions (b)).
Pour atteindre le niveau d'intensité obtenue avec le dérivé *méta*-bioPMDAM, une étape d'amplification du signal est nécessaire (conditions a2). Ceci montre bien l'efficacité de la fonction réactive diazométhyle par rapport à l'incorporation traditionnelle de base modifiée comme le d-UTP fluorescéine (protocole de référence (b)).

### Exemple 18 : Fragmentation de l'ADN double brin par sonication :

Des amplicons ADN ont été obtenus en utilisant le protocole décrit dans l'exemple 5. Ces amplicons ont été fragmentés par sonication en présence et en absence du marqueur.

### a. Marquage des amplicons PCR pendant la sonication :

A 10 µL de réaction PCR sont ajoutés 2 µL de *méta*-BioPMDAM (100 mM dans DMSO). Le volume est ajusté à 100 µL par de l'eau pure le pH est ajusté à 6,5. Le mélange est incubé 30 min à 60°C dans un bain d'une cuve à ultrasons (fréquence 35kHz, modèle T460-H, Bioblock, France).

### b. Marquage pendant la fragmentation chimique des amplicons PCR (protocole de référence en une étape) :

A 10 µL de réaction PCR sont ajoutés 50 µL de formiate de sodium pH 3 (50 mM) et 2 µL de *méta*-BioPMDAM (100 mM dans du DMSO). Le volume est ajusté à 100 µL. La solution est incubée 30 min à 60°C.

Les essais sont réalisés en duplicata afin de pouvoir analyser la fragmentation de l'ADN sur gel et l'efficacité de marquage par hybridation et lecture de la puce à ADN comme décrit précédemment (exemple 9 sur la détection de la phycoérythrine).

### Analyse sur gel

L'analyse a été effectuée sur gel dénaturant de polyacrylamide (8 % polyacrylamide, 7 M urée, 1 X TBE) utilisant coloration au bromure d'éthidium.
L'analyse sur gel montre que les amplicons ADN sont fragmentés par sonication à 60°C.

### Résultats

**Tableau 10 : Fragmentation de l'ADN double brin par sonication**

| **Conditions** | **Homologie (%)** | **I (rfu)** | **B (rfu)** | **I/B** |
|---|---|---|---|---|
| *a. Marquage pendant la fragmentation par sonication* | 93,8 | 2271 | 631 | 3,6 |
| *b. Marquage pendant la fragmentation chimique (conditions de référence)* | >95 | 19639 | 1459 | 13,5 |

Les résultats de marquage du tableau 10 pendant la sonication (conditions a) sont satisfaisants. Ceci montre que la fragmentation physique par sonication des cibles ADN est compatible avec la chimie de marquage par des réactifs de marquage portant une fonction diazométhyle.
Les faibles résultats de marquage dans ce cas sont certainement dus au fait que le marqueur se dégrade sous l'effet des ultrasons. Les résultats avec la fragmentation par création de site abasique par action du pH acide sont meilleurs.

### Exemple 19 : Marquage, fragmentation et dénaturation de l'ADN en une étape :

Les amplicons ADN ont été préparés par amplification PCR selon le protocole décrit dans l'exemple 5.
Deux réactions de marquage ont été réalisées :

### a. Marquage, fragmentation et dénaturation à 95°C en une seule étape :

A 10 µL de PCR sont ajoutés 50 µL de tampon formiate de sodium pH3 (50 mM) et 2 µL du marqueur *méta*-BioPMDAM (100mM dans du DMSO anhydre). Le volume final est ajusté à 100 µL. La solution est ensuite incubée 30 min à 95°C. Dans ce cas, le mélange réactionnel a été hybridé sur puce à ADN sans aucune purification préalable.

### b. Marquage et fragmentation à 60°C :

A 10 µL de PCR sont ajoutés 50 µL de tampon formiate de sodium pH3 (50 mM) et 2 µL du marqueur *méta*-BioDPDAM (100mM dans du DMSO anhydre). Le volume final est ajusté à 100 µL. La solution est ensuite incubée 30 min à 60°C. Le mélange réactionnel a été ensuite purifié selon le protocole décrit auparavant. Dans ce protocole et avant hybridation sur puce à ADN, les fragments ont été dénaturés selon le protocole décrit dans l'exemple 12.2.

### Résultats

**Tableau 11 : Marquage, fragmentation et dénaturation de l'ADN en une étape**

| **Protocole utilisé** | **Homologie (%)** | **I (rfu)** | **B (rfu)** | **I/B** |
|---|---|---|---|---|
| *a. Marquage, fragmentation et dénaturation à 95°C* | >95 | 5426 | 506 | 10,7 |
| *b. Marquage et fragmentation à 60°C puis dénaturation à 95°C* | >95 | 7015 | 818 | 6,8 |

L'exemple 12.2 a démontré l'importance de la dénaturation de l'ADN double brin pour la sensibilité de détection. Ces résultats du tableau 11 montrent qu'avec l'approche fragmentation par création de site abasique, le marquage, la fragmentation et la dénaturation de l'ADN peuvent être réalisées en une seule étape, ce qui représente une amélioration notable du point de vue de la simplicité et du temps pour l'utilisateur et cela sans affecter la sensibilité de détection.

### Exemple 20 : Synthèse du para-Bio-EG3-PDAM :

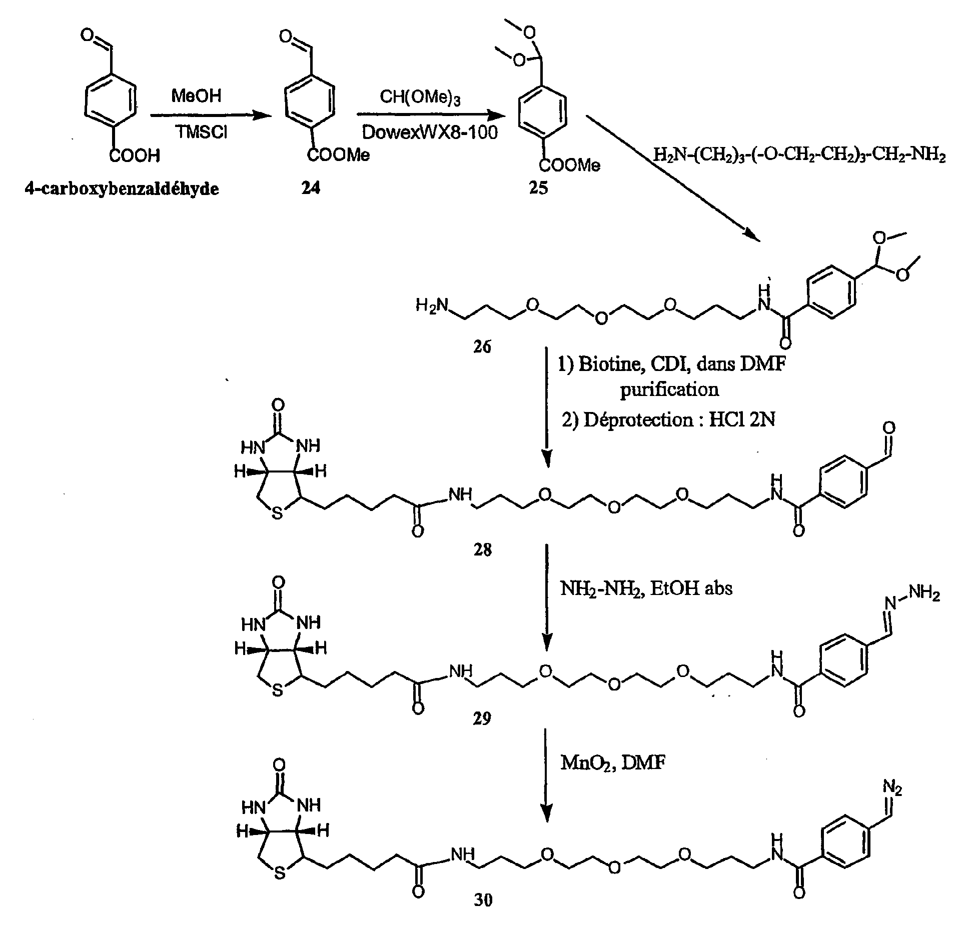

### • Protection du 4-carboxylbenzaldéhyde :

Le 4-carboxybenzaldéhyde est commercial. On le dissout (3 g ; 20 mmol) dans une solution de chlorure de triméthylsilyle (10 g ; 92 mmol) dans 100 mL de MeOH. Le mélange est laissé sous agitation pendant 40 h à température ambiante. Après évaporation, un solide blanc correspondant au 4-méthoxycarbonylbenzaldéhyde **24** est isolé, caractérisé par RMN et utilisé tel quel pour la réaction suivante.

RMN ¹H (200MHz, CDCl₃) δ =10,07 (s, 1H, -CHO) ; 8,17 (d, 2H, *J =* 8 Hz, Ar-H_{2,6}) ; 7,92 (d, 2H, *J=* 8 Hz, Ar-H_{3,5}) ; 3,93 (s, 3H, CO-O-CH₃).

### • Protection du 4'-méthoxycarbonylbenzaldéhyde :

Le 4-méthoxycarbonylbenzaldéhyde (3,35 g; 20 mmol) est mis en solution avec de l'orthoformiate de triméthyle (4,8 g ; 40 mmol) en présence de Dowex 50WX8-100 (1 g). Le mélange est chauffé à reflux pendant 2 h, puis filtré et évaporé. Après un essai de recristallisation, une analyse par RMN montre que la réaction n'est pas complète et celle-ci est relancée dans 30 mL de MeOH, 30 mL de CH(OMe)₃ et 1 g de Dowex 50WX8-100 à température ambiante. On réalise une filtration puis une évaporation, afin d'obtenir 3,55 g (16,89 mmol, 84 %) de produit 25.

RMN ¹H (200MHz, CDCl₃) δ = 8,01 (d, 2H, *J* = 8 Hz, Ar-H_{2,6}) ; 7,50 (d, 2H, *J* = 8 Hz, Ar-H_{3,5}) ; 5,41 (s, 1H, CH) ; 3,93 (s, 3H, -CO-O-CH₃) ; 3,29 (s, 6H, -O-CH₃).

### • Composé 26 :

On solubilise le composé **25** (3,1 g ; 14,8 mmol) dans 16 mL (73 mmol) de 4,7,10-Trioxa-1,13-tridécanediamine. La solution obtenue est chauffée à 140-150°C pendant 2 h. Le mélange est ensuite dissous dans 100 mL de DCM (dichlorométhane ou CH₂Cl₂) et lavé 6 fois avec 10 mL d'eau. La phase organique est séchée avec Mg SO₄ puis évaporée jusqu'à l'obtention d'une huile. Cette huile est lavée avec du pentane 3 fois de suite par décantation, puis une nouvelle extraction avec DCM et H₂O est réalisée. Après séchage sur MgSO₄ et évaporation, le produit **26** est isolé avec un rendement de 63 % (9,27 mmol).

RMN ¹H (200MHz, CDCl₃) δ = 7,78 (d, 2H, *J* = 8 Hz, Ar-H_{2,6}) ; 7,46 (d, 2H, *J* = 8 Hz, Ar-H_{3,5}) ; 5,39 (s, 1H, CH) ; 3,62-3,47 (m, 14H, H_{7',8',10',11'} et H_{5', 13'} et H_{3'}) ; 3,29 (s, 6H, -O-CH₃) ; 2,72 (m, 2H, H_{15'}) 1,87 (m, 2H, H_{4'}) ; 1,64 (m, 2H, H_{14'}) ; 1,30 (s large, 2H, NH₂).

### • Composé biotinylé 27 :

La biotine (500 mg ; 2,05 mmol) est mise en suspension dans 10 mL de DMF puis 365 mg (2,25 mmol) de CDI sont ajoutés. Cette solution est laissée sous agitation pendant 30 min à température ambiante. Le composé 26 (900 mg ; 2,26 mmol) est dissous dans 1 mL de DMF, puis ajouté petit à petit à la solution précédente. Le mélange ainsi obtenu est laissé sous agitation pendant 1 h à température ambiante. Après évaporation, une purification sur colonne par chromatographie-flash (colonne de 20 mm de diamètre) est réalisée avec 250 mL de MeOH-DCM 6 %, puis avec 200 mL de MeOH-DCM 7 % et enfin 200 mL de MeOH-DCM 8 %. Les fractions correspondant au produit 27 sont rassemblées puis évaporées à sec pour donner 1,00 g d'huile avec un rendement estimé à 50 %.

RMN ¹H (200MHz, CDCl₃) δ = 9,50 (s large, 1H, NH_{imidazole}) ; 7,80 (d, 2H, *J* = 8 Hz, Ar-H_{2,6}) ; 7,64 (s, 1H, H_{imidazole}) ; 7,46 (d, 2H, *J* = 8 Hz, Ar-H_{3,5} et 1H, NH_{2'}) ; 7,05 (s, 2H, H_{imidazole}) ; 6,76 (t, 1H, NH_{16'}), 6,20 (s large, 1H, NH_{B1}) ; 5,44 (s large, 1H, NH_{B3}) ; 5,37 (s, 1H, CH) ; 4,42 (m, 1H, H_{B6a}) ; 4,24 (m, 1H, H_{B3a}) ; 3,59-3,44 (m, 14H, H_{7',8',10',11'} et H_{5',13'} et H_{3'}) ; 3,29 (m, 8H, H_{15'} et 2-O-CH₃) ;3,07 (m, 1H, H_{B4}) ; 2,84 et 2,66 (système ABX, 2H, ²*J*_{AB}= 5 Hz, ³*J*_{AX} = 12 Hz, ³*J*_{BX} = 0 Hz, H_{B6}); 2,13 (t, 2H, *J*= 8 Hz, H_{B10}) ;1,85 (m, 2H, H_{4'}) ; 1,66 (m, 2H, H_{14'}) ; 1,40-1,37 (m, 6H, H_{B7, B8, B9}).

### • Composé aldéhydique 28 :

L'acétal **27** est dissous dans 50 mL de chloroforme, puis 20 mL de HCl 2N sont ajoutés . Le mélange diphasique est vigoureusement agité pendant 15 min. On récupère la phase organique qu'on sèche sur NaHCO₃ anhydre. On filtre, on évapore et le composé **28** est obtenu sous forme d'une pâte (495 mg ; 0,855 mmol) avec un rendement global de 42 % à partir de la biotine.

RMN ¹H (300MHz, CDCl₃) δ = 10,05 (s, 1H, CHO) ; 7,98 (d, 2H, *J* = 8 Hz, Ar-H_{2,6}) ; 7,92 (d, 2H, *J* = 8 Hz, Ar-H_{3,5}) ; 7,58 (t, 1H, NH_{2'}) ; 6,46 (t, 1H, NH_{16'}) , 6,02 (s large, 1H, NH_{B1}) ; 5,19 (s large, 1H, NH_{B3}) ; 4,46 (m, 1H, H_{B6a}) ; 4,27 (m, 1H, H_{B3a}) ; 3,66-3,56 (m, 10H, H_{7'}, _{8',10',11'} et H_{5'}) ; 3,50-3,29 (m, 4H, H_{3',13'}) ; 3,28 (m, 2H, H_{15'}) ; 2,95 (m, 1H, H_{B4}) ; 2,84 et 2,71 (système ABX, 2H, ²*J*_{AB}= 5 Hz, ³*J*_{AX} = 12 Hz, ³*J*_{BX} = 0 Hz, H_{B6}) ; 2,15 (t, 2H, *J* = 8 Hz, H_{B10}) ;1,89 (m, 2H, H_{4'}) ; 1,72-1,63 (m, 6H, H_{14'}, H_{B7, B9}) ; 1, 23 (m, 2H, H_{B8}).

### • Composé hydrazone 29 :

L'aldéhyde **28** (495 mg ; 0,855 mmol) est dissous dans 10 mL d'éthanol absolu. L'hydrazine (350 µL; 7,20 mmol) est ajoutée, puis le mélange réactionnel est chauffé à reflux pendant 1 h. L'huile obtenue après évaporation est dissoute dans EtOH abs, pour être de nouveau évaporée. On obtient alors une mousse qu'on triture au pentane. La pâte correspondant au produit **29** (511 mg ; 0,862 mmol) est obtenue avec un rendement de 100 %.

RMN ¹H (300MHz, CDCl₃) δ = 7,76 (d, 2H, *J*= 8 Hz, Ar-H_{2,6}) ; 7,72 (s, 1H, CH) ; 7,56 (d, 2H, *J* = 8 Hz, Ar-H_{3,5}) ; 7,34 (t, 1H, NH_{2'}) ; 6,45 (t, 1H, NH_{16'}), 5,98 (s large, 1H, NH_{B1}) ; 5,78 (s large, 2H, NH₂) ; 5,18 (s large, 1 H, NH_{B3}) ; 4,44 (m, 1 H, H_{B6a}) ; 4,26 (m, 1 H, H_{B3a}) ; 3,62-3,56 (m, 10H, H_{7', 8',10',11'} et H_{5'}) ; 3,48-3,45 (m, 4H, H_{3',13'}) ; 3,27 (m, 2H, H_{15'}) ; 3,07 (m, 1H, H_{B4}) ; 2,84 et 2,68 (système ABX, 2H, ²*J*_{AB}= 5 Hz, ³*J*_{AX} = 12 Hz, ³*J*_{BX} = 0 Hz, H_{B6}) ; 2,11 (t, 2H, *J* = 8 Hz, H_{B10}) ;1,86 (m, 2H, H_{4'}) ; 1,72-1,59 (m, 6H, H_{14'}, H_{B7}, _{B9}); 1, 21 (m, 2H, H_{B8}).

### • Composé diazo 30 :

On solubilise l'hydrazone **29** (357 mg ; 0,602 mmol) dans 17,5 mL de DMF. On ajoute alors MnO₂ (700 mg ; 7,7 mmol). Après 12 min d'agitation à température ambiante, le mélange est filtré sur millipore contenant de la célite (épaisseur : 2 cm) et du tamis moléculaire en poudre 3 Å (0,5 cm). Le mélange réactionnel est évaporé à sec. L'huile résiduelle obtenue est lavée à l'éther, trois fois de suite. Le composé **30** (290 mg, 0,491 mmol) est obtenu sous forme d'un solide légèrement rose avec un rendement de 82 %.

RMN ¹H (300MHz, DMSO-d₆) δ = 8,28 (t, 1H, NH_{2'}) ; 7,77 (d, 2H, *J*= 8 Hz, Ar-H_{2,6}) ; 7,74 (t, 1H, NH_{16'}); 7,00 (d, 2H, *J* = 8 Hz, Ar-H_{3,5}) ; 6,38 (s large, 1H, NH_{B1}) ; 6,32 (s large, 1H, NH_{B3}) ; 5,80 (s, 1H, CH-N₂) ; 4,27 (m, 1H, H_{B6a}) ; 4,11 (m, 1H, H_{B3a}) ; 3,51-3,44 (m, 10H, H_{7', 8', 10', 11'} et H_{5'}) ; 3,37 (m, 2H, H_{15'}) ; 3,32 (m, 4H, H_{3',13'}) ; 3,05 (m, 1H, H_{B4}) ; 2,79 et 2,58 (système ABX, 2H, ²*J*_{AB}= 5 Hz, ³*J*_{AX} = 12 Hz, ³*J*_{BX} = 0 Hz, H_{B6}) ; 2,02 (t, 2H, *J* = 8 Hz, H_{B10}) ;1,69 (m, 2H, H_{4'}) ; 1,59-1,48 (m, 6H, H_{14'}, H_{B7}, _{B9}) ; 1, 25 (m, 2H, H_{B8}).

La réactivité du composé **30** a été testée sur 3'-Uridine-monophosphate et suivie par électrophorèse capillaire. Les conditions d'analyse sont celles de l'exemple 6.1. Les résultats montrent un temps de demi vie de 45 minutes.
Le réactif est stable à -20°C pendant au moins 1 mois.

### Exemple 21 : Marquage et fragmentation des amplicons d'ADN avec le réactif de marquage para-Bio-EG3-PDAM :

Les intérêts principaux de ce genre de molécules, c'est-à-dire de dérivés de PDAM portant un bras de liaison à base de polyéthylène glycol, sont de permettre l'éloignement de la fonction diazo par rapport à la biotine, et d'augmenter la solubilité et, au final, la réactivité de ces molécules.

Le dérivé *para*-Bio-EG3-PDAM **30** a été obtenu selon le schéma réactionnel décrit dans l'exemple 20. Les amplicons ADN ont été préparés par amplification PCR selon le protocole décrit dans l'exemple 5. Deux réactions de marquage ont été réalisées.

### a. Marquage par le réactif para-Bio-EG3-PDAM :

A 10 µL de PCR sont ajoutés 10 µL de *para*-Bio-EG3-PDAM (100 mM dans du DMSO) et 77 µL d'eau Dnase/Rnase free. La solution est homogène et ne présente pas de précipités. Cette solution est incubée 10 min à 95°C, puis 3 µL d'HCl à 0,1 M sont ajoutés et la solution est incubée 10 min à 95°C.

Le reste du protocole est identique à celui de l'exemple 8.

### b. Marquage par le réactif méta-BioPMDAM :

A 10 µL de PCR sont ajoutés 10 µL de *méta*-BioPMDAM (100 mM dans du DMSO) et 77 µL d'eau Dnase/Rnase free. La synthèse de ce produit est évoqué dans l'exemple 1.1. La solution présente un léger précipité. Cette solution est incubée 10 min à 95°C. Puis 3 µL d'HCl 0,1 M sont ajoutés et la solution est incubée 10 min à 95°C.
Le reste du protocole est identique à celui de l'exemple 8.

### Résultats :

**Tableau 12 : Etude comparative du marquage et de la fragmentation d'amplicons d'ADN avec le para-Bio-EG3-PDAM et le méta-BioPMDAM**

| **Protocole utilisé** | **Homologie (%)** | **I (rfu)** | **B (rfu)** | **I/B** |
|---|---|---|---|---|
| a. Marquage par le réactif *para*-Bio-EG3-PDAM | > 95% | 15151 | 621 | 24,4 |
| b. Marquage par le réactif *méta*-BioPMDAM | > 95% | 11226 | 515 | 21,8 |

Les intensités de signal obtenues dans ce tableau 12 sont très satisfaisantes et le pourcentage d'homologie est élevé. Ce résultat montre que l'introduction d'un bras polyéthylène glycol sur la molécule de marquage diazo permet d'augmenter la solubilité en phase aqueuse du réactif. Le test est donc homogène. De plus, l'augmentation de la solubilité permet d'augmenter la réactivité du marqueur.

### Exemple 22 : Synthèse d'autres dérivés PDAM comportant un bras de liaison à base de polyéthylène glycol :

### Exemple 22.1 : Synthèse du méta-Bio-EG3-PMDAM :

### Schéma de synthèse

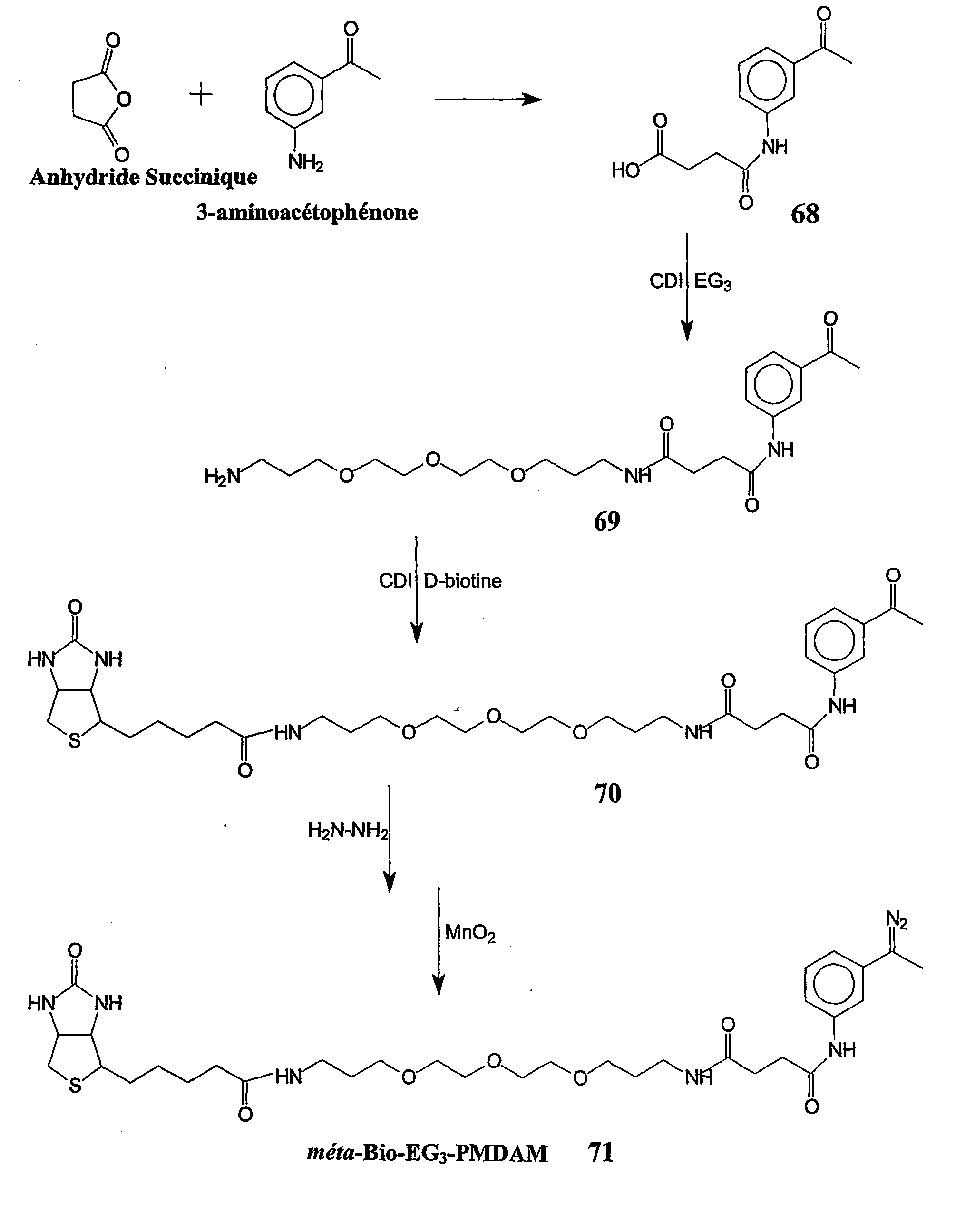

### Composé 68 :

On solubilise la 3-aminoacetophenone (14,5 g, 107 mmol) dans 50 ml de DMF anhydre. On ajoute l'anhydride succinique (10,7 g, 107 mmol) et on laisse sous agitation, sous argon et a température ambiante. Après 6 h, la solution est concentrée sous vide et 50 ml de méthanol sont ajoutés. Le précipité obtenu est filtré et lavé avec méthanol et éther. On obtient ainsi 19,4 g (81 %) de produit **68** sous forme d'une poudre de couleur blanc cassé.
RMN ¹H (200 MHz, DMSO-d₆): d = 2,5-2,6 (m, 7H); 7,45 (t, 1H); 7,64 (d, 1H); 7,83 (d, 1H); 8,19 (s, 1 H); 10,16 (s, 1H); 12,12 (s, 1 H).

### Composé 69 :

On solubilise 5,07 g (22 mmol) du composé **68** dans 10 ml de DMF anhydre, sous argon. On met sur glace et on ajoute 5,00 g (32 mmol) de carbonyldiimidazole. Apres 20 min, on ajoute lentement 20 ml (94,6 mmol) du 4, 7, 10-trioxatridecanediamine (EG₃). Après 3h de réaction à température ambiante, on évapore le DMF et reprend le résidu dans 100 ml de CH₂CL₂. On fait des extractions avec du NaHCO₃ saturé et H₂O, après quoi la phase organique est séchée avec du Na₂SO₄ anhydre et le solvant évaporé. On obtient ainsi 4,34 g (46%) du produit **69.** RMN ¹H (200 MHz, DMSO-d₆): d = 1,59 (m, 2H); 1,87 (m, 2H); 2,16 (s, 3H); 2,40 (m, 2H); 2,55 (m, 2H); 3,08 (m, 2H); 3,45 (m, 16H); 7,30 (t, 1H); 7,42 (d, 1H); 7,70 (d, 1H); 7,83 (t, 1H); 7,97 (s, 1H); 10,00 (s, 1H).

### Composé biotinylé 70 :

On solubilise la D-biotine (1,0 g, 4,1 mmol) dans 10 ml de DMF anhydre, sous argon. On refroidit sur glace et on ajoute le carbonyldiimidazole (CDI) (0,665 g, 4,1 mmol) dans 10 ml de DMF anhydre. Après 15 min, on ajoute le composé **69** (1,8 g, 4,1 mmol) dans 2 ml de DMF anhydre. On laisse réagir 3 h à 35°C, puis on évapore le DMF et on reprend dans 100ml de CH₂CL₂. On fait des extractions avec du NaHCO₃ saturé et H₂O, après quoi la phase organique est séchée avec du Na₂SO₄ anhydre et le solvant évaporé. La caractérisation par RMN du produit ainsi obtenu montre qu'on obtient un mélange du produit **70** et du EG₃ libre. Une autre étape de purification ets effectuée avant de continuer la synthèse.
Le composé final, *méta*-Bio-EG₃-PMDAM, est obtenu après deux étapes de synthèse selon le schéma décrit dans l'exemple 1.

L'intérêt de cette synthèse est double. D'une part, on obtient le produit **69** en seulement deux étapes; ce produit peut être utilisé comme précurseur du diazo avec la possibilité d'y accrocher des molécules détectables de nature différente, grâce au groupement amine terminal. Ce groupement permet aussi de greffer le composé **69** sur des supports solides, avec l'objectif d'immobiliser des acides nucléiques. D'une autre part, le composé **71** possède le même centre réactif que la *méta*-Bio-PMDAM (notre molécule de référence), ce qui facilite l'analyse des avantages liés à l'inclusion du bras éthylèneglycol (EG₃).
Le réactif est stable à -20°C pendant au moins 1 mois.

### Exemple 22.2 : Synthèse du méta-Bio-EG4-PMDAM :

### Schéma de synthèse :

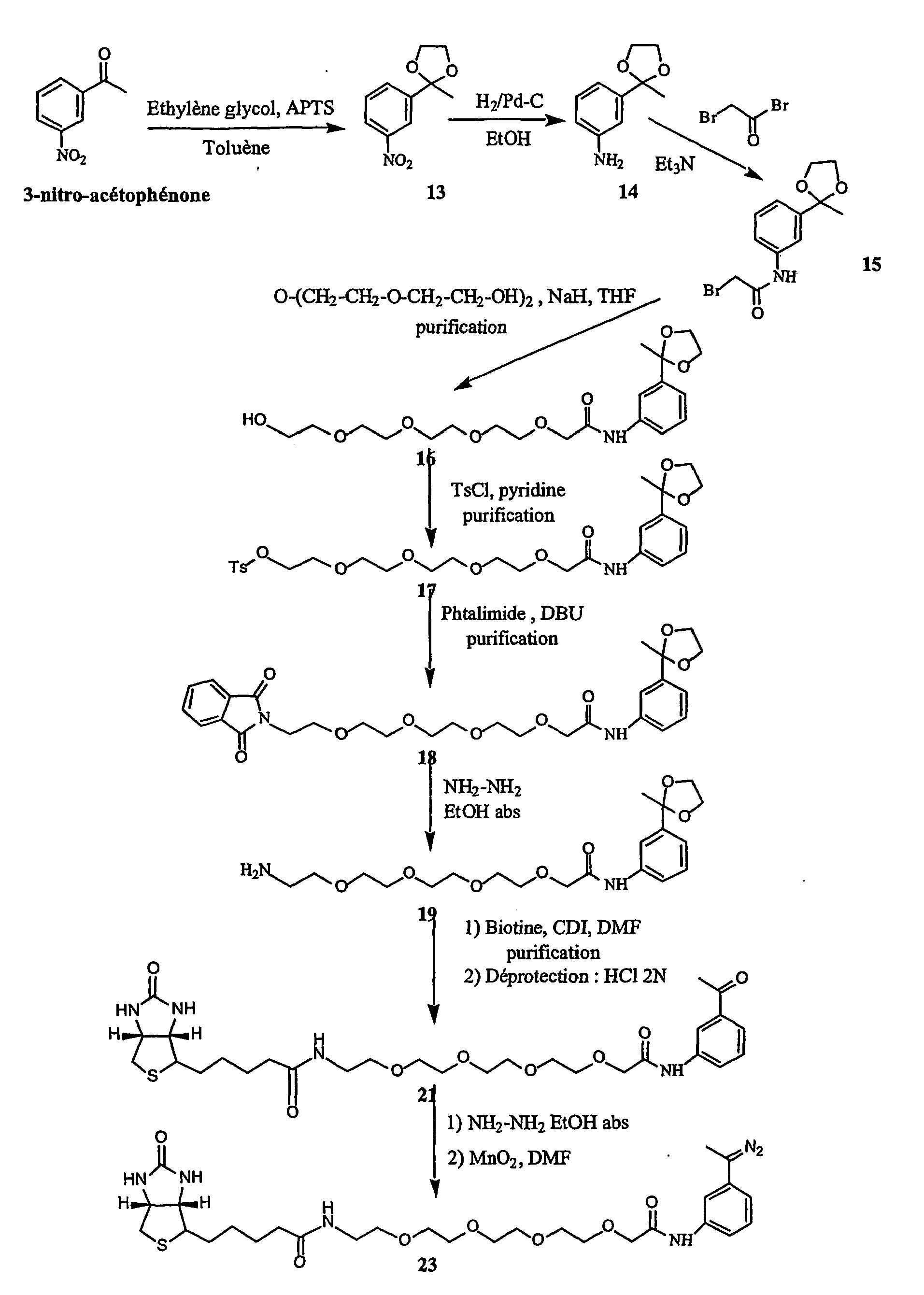

### • Protection de la 3-nitro-acétophenone 13 :

On dissout 33 g (0,20 mol) de 3-nitro-acétophénone dans 400 mL de toluène, puis on ajoute 40 mL (0,717 mol) d'éthylène glycol et 600 mg (3,15 mmol) d'acide para-toluène sulfonique (APTS). On monte un système Dean Stark. On chauffe la solution pendant 3 h à 130°C. Après avoir laissé la solution revenir à température ambiante, on ajoute 400 mL d'acétate d'éthyle, puis on lave celle-ci avec 8 mL d'une solution de NaHCO₃ saturée. On sèche la phase organique sur MgSO₄. Après évaporation, on obtient un solide jaune pâle **13** (39,72 g ; 0,190 mol) avec un rendement de 95 %.

RMN ¹H (200MHz, CDCl₃) δ =7,11 (t, 1H, *J* = 8 Hz, Ar-H); 6,87-6,78 (m, 2H, Ar-H) ; 6,59 (dd, 1H, *J* = 6,5 Hz, Ar-H); 4,00 (m, 2H, H₂C_{acétale}) ; 3,79 (m, 2H, H₂C_{acétale}); 1,61 (s, 3H, CH₃).

### • Préparation de l'amine 14 :

Le composé **13** (39,7 g ; 0,190 mol) est dissous dans 500 mL d'EtOH, puis 1 g de palladium sur charbon 10 % est ajouté. On chauffe pour tout dissoudre, puis on laisse la solution revenir à température ambiante. Après avoir fait le vide et mis la solution sous H₂, on laisse sous forte agitation pendant 5 h. La solution est ensuite filtrée à chaud puis évaporée. Le produit **14** est lavé au pentane, et isolé sous forme d'un solide (34 g ; 0,189 mol) avec un rendement de 99 %.

RMN ¹H (200MHz, CDCl₃) δ =7,14 (t, 1H, *J=* 8 Hz, Ar-H) ; 6,85 (m, 2H, *J=* 7,5 Hz, Ar-H) ; 6,79 (s, 1H, Ar-H) ; 6,59 (dd, 1H, *J* = 6,5 Hz, Ar-H); 4,00 (m, 2H, H₂C_{acétale}); 3,77 (m, 2H, H₂C_{acétale}) ; 1,61 (s, 3H, CH₃).

### • Composé bromé 15 :

L'amine **14** (12,3 g ; 68,7 mmol) et la triéthylamine (7 g ; 69 mmol) sont mises en solution dans 150 mL de DCM sous argon. On ajoute à-5 °C au goutte-à-goutte une solution de 13,8 g (60 mmol) de bromure de bromoacétyle dissous dans 150 mL de DCM. À la fin de l'addition, on ajoute 100 mL de NaHCO₃ aqueux 1 N. On lave la phase organique deux fois de suite avec NaHCO₃ aqueux et on sèche sur MgSO₄. Après évaporation à sec, 22,6 g d'huile marron est obtenue correspondant au composé 15 et utilisée telle quelle pour la réaction suivante.

RMN ¹H (200MHz, CDCl₃) δ = 8,29 (s large, 1H, NH) ; 7,62 (dt, 1H₄, *J* = 5 Hz, Ar-H) ; 7,47 (s, 1H, Ar-H₂); 7,38-7,19 (m, 2H, Ar-H_{5,6}) ; 4,00 (m, 2H, Br-CH₂) ; 3,75 (m, 4H, H₂C-H₂C_{acétale}) ; 1,61 (s, 3H, CH₃).

### • Composé alcool 16 :

L'hydrure de sodium (3,3 g ; 82,5 mmol) est lavé trois fois avec du pentane puis mis en suspension dans 150 mL de THF, le tétra éthylène glycol (50 mL ; 0,29 mol) est alors ajouté à température ambiante. On laisse la réaction sous agitation pendant 15 min, puis on refroidit la solution à -5°C.
L'ajout du composé **15** préalablement dilué dans 25 mL de THF se fait au goutte-à-goutte. On laisse sous agitation pendant 30 min pour laisser revenir à température ambiante. On concentre la solution à 100 mL, puis on dilue celle-ci avec 500 mL de CHCl₃. On lave trois fois de suite cette phase organique avec 250 mL de NaHCO₃ aqueux 1 N, puis on la sèche sur MgSO₄ avant de l'évaporer. On purifie le produit sur colonne de silice par chromatographie-flash (colonne de 65 mm de diamètre) avec 1,5 L de MeOH-DCM 5 %, puis avec 500 mL de MeOH-DCM 7 %, et enfin 500 mL de MeOH-DCM 10 %. Les fractions correspondant au composé **16** sont rassemblées, puis évaporées à sec pour donner 17,4 g (42,1 mmol) de produit, avec un rendement de 61 %.

RMN ¹H (200MHz, CDCl₃) δ = 8,86 (s large, 1H, NH) ; 7,71 (d, 1H, *J=* 7,5 Hz, Ar-H₄) ; 7,51 (s, 1H, Ar-H₂) ; 7,29-7,24 (m, 2H, Ar-H_{5,6}) ; 4,09 (m, 2H, CO-CH₂-O) ; 3,99 (m, 2H, H₂C_{acétale}) ; 3,72-3,53 (m, 20H, O-CH₂-CH₂-O, H₂C_{acétale} et HO-CH₂) ; 1,61 (s, 3H, CH₃).

### • Composé tosylate 17 :

L'alcool **16** (4,13 g ;10,0 mmol) est mis en solution dans 5 mL de pyridine. On ajoute ensuite 2,0 g (10,5 mmol) de chlorure de tosyle à température ambiante. On met sous agitation sous argon pendant 10 h. On dilue avec 100 mL de DCM, on lave trois fois la phase organique avec 20 mL de NaHCO₃ aqueux 1 N, puis on la sèche sur MgSO₄ avant de la co-évaporer avec du toluène. Une purification sur colonne par chromatographie-flash (colonne de 50 mm de diamètre) est réalisée avec 500 mL de MeOH-DCM 2 %, puis 500 mL de MeOH-DCM 3 %, et enfin 500 mL de MeOH-DCM 4 %. Les fractions correspondant au produit **17** sont rassemblées puis évaporées à sec pour donner 3,68 g (6,48 mmol) d'huile avec un rendement estimé à 65 %.

RMN ¹H (300MHz, CDCl₃) δ = 8,86 (s large, 1H, NH) ; 7,76 (d, 4H, *J*= 5,5 Hz, Ar-H_{tosyl}) ; 7,60 (d, 1H, *J*= 7,5 Hz, Ar-H₄); 7,50 (s, 1H, Ar-H₂) ; 7,32-7,22 (m, 2H, Ar-H_{5,6}) ; 4,10 (m, 2H, CO-CH₂-O) ; 4,00 (m, 2H, H₂C_{acétale}) ; 3,73-3,54 (m, 20H, O-CH₂-CH₂-O, H₂C_{acétale} et HO-CH₂) ; 2,42 (s, 3H, Ar-CH₃) ; 1,61 (s, 3H, CH₃).

### • Composé phtalimide 18 :

On met le tosylate **17** (3,68 g ; 6,48 mmol) en solution avec 1,52 g (10,0 mmol) de DBU (1,8-diazobicyclo-[5.4.0]-undecène) puis on ajoute le phtalimide (1,47 g ; 10 mmol). La solution ainsi obtenue est chauffée à 85-90°C pendant 17 h puis évaporée. On purifie le produit sur colonne de silice par chromatographie-flash (colonne de 50 mm de diamètre) avec 1 L d'acétone-DCM 15 %, puis avec 1 L d'acétone-DCM 20 %. Les fractions correspondant au composé **18** sont rassemblées, puis évaporées à sec pour donner 3,15 g (5,8 mmol) de produit, avec un rendement de 90 %.

RMN ¹H (200MHz, CDCl₃) δ = 8,73 (s large, 1H, NH) ; 7,79 (m, 2H, Ar-Hₚₕₜₐ) ; 7,99 (m, 2H, Ar-Hₚₕₜₐ et Ar-H₄) ; 7,49 (s, 1H, Ar-H₂) ; 7,27-7,18 (m, 2H, Ar-H_{5,6}) ; 4,10 (m, 2H, CO-CH₂₋O) ; 4,00 (m, 2H, H₂C_{acétale}) ; 3,69-3,56 (m, 20H, O-CH₂-CH₂-O, H₂C_{acétale} et Nₚₕₜₐ-CH₂); 1,61 (s, 3H, CH₃).

### • Composé amine 19 :

Le produit **18** est dissous dans 20 mL d'EtOH absolu en chauffant à reflux à 75-80°C. L'hydrazine (1,07 mL ; 22,1 mmol) est ensuite ajoutée, et la réaction est laissée sous agitation pendant 1 h 15. Le précipité obtenu est filtré sur fritté et la phase éthanolique évaporée. Le précipité blanc est ensuite lavé au DCM, et la phase DCM est évaporée. L'huile jaune obtenue (2,3 g ; 5,57 mmol) est directement utilisée pour la réaction suivante, même si elle contient de l'imidazole qui pourra être éliminé par la suite, lors de l'étape de déprotection de l'acétal.

RMN ¹H (300MHz, CDCl₃) δ = 8,83 (s large, 1H, NH) ; 7,69 (d, 1H, *J*= 7,5 Hz, Ar-H₄) ; 7,51 (s, 1H, Ar-H₂) ; 7,30-7,19 (m, 2H, Ar-H_{5,6}) ; 4,10 (m, 2H, CO-CH₂-O); 4,00 (m, 2H, H₂C_{acétale}) ; 3,69-3,56 (m, 20H, O-CH₂-CH₂-O, H₂C_{acétale} et H₂N-CH₂) ; 1,61 (s, 3H, CH₃).

### • Composé biotinylé 20 :

On solubilise la D-Biotine (1,05 g ; 4,32 mmol) dans 10 mL de DMF anhydre. On ajoute sous argon 790 mg (4,87 mmol) de carbonyle diimidazole (CDI). Après 10 min d'agitation, on ajoute l'amine **19** diluée dans 5 mL de DMF. La solution est laissée 40 min sous agitation, puis évaporée avant d'être purifiée sur colonne par chromatographie-flash.
Pour ceci on utilise une colonne de 50 mm de diamètre avec comme éluant 500 mL de MeOH-DCM 5 %, puis 500 mL de MeOH-DCM 10 %, et enfin 500 mL de MeOH-DCM 15 %. Les fractions correspondant au produit **20** sont rassemblées puis évaporées à sec pour donner une huile jaune (1,66 g ; 2,6 mmol).
L'huile jaune obtenue (2,4 g) contenant d'après le spectre RMN environ 30 % en poids d'imidazole. On en déduit donc que le rendement de la réaction aboutissant au produit **20** est d'environ 60 % par rapport à la biotine de départ.

RMN ¹H (300MHz, CDCl₃) δ = 8,80 (s large, 1H, NH) ; 7,66 (m, 3H, Ar-H₄ et H_{imidazole}); 7,54 (s, 1H, Ar-H₂); 7,28-7,24 (m, 2H, Ar-H_{5,6}); 7,07 (s, 2H, H_{imidazole}), 6,59 (t, 1H, NH_{15'}); 6,06 (s large, 1H, NH_{B1}); 5,19 (s large, 1H, NH_{B3}); 4,45 (m, 1H, H_{B6a}); 4,27 (m, 1H, H_{B3a}); 4,10 (s, 2H, H_{3'}); 4,00 (m, 2H, H₂C_{acétale}); 3,75-3,49 (m, 18H, O-CH₂-CH₂-O et H₂C_{acétale,}); 3,36 (m, 2H, H_{14'}); 3,09 (m, 1H, H_{B4}); 2,85 et 2,66 (système ABX, 2H, ²*J*_{AB}= 5 Hz, ³*J*_{AX} = 12 Hz, ³*J*_{BX} = 0 Hz, H_{B6}) ; 2,16 (t, 2H, *J* = 8 Hz, H_{B10}) ; 1,61 (s, 3H, CH₃) ; 1,59-1,3 (m, 6H, H_{B9}, _{B8, B7}).

### • Composé cétone 21 :

L'acétal **20** est dissous dans 80 mL de chloroforme, puis 30 mL de HCl 2 N sont ajoutés. On laisse sous forte agitation pendant 45 min. La phase organique est récupérée puis séchée sur NaHCO₃ anhydre. Après filtration, la solution est évaporée et l'huile obtenue est lavée au pentane pour donner le produit **21** (1,48 g ; 2,48 mmol) avec un rendement de 99 %.

RMN ¹H (3,00MW, CDCl₃) δ = 8,99 (s large, 1H, NH) ; 8,07 (s, 1H, Ar-H₂) ; 7,98 (d, 2H, *J*= 8 Hz, Ar-H₄) ; 7,66 (d, 2H, *J* = 8 Hz, Ar-H₆) ; 7,42 (t, 2H, *J* = 8 Hz, Ar-H₅) ; 6,38 (t, 1H, NH_{15'}) ; 5,78 (s large, 1H, NH_{B1}) ; 4,96 (s large, 1H, NH_{B3}) ; 4,47 (m, 1H, H_{B6a}) ; 4,29 (m, 1H, H_{B3a}) ; 4,13 (s, 2H, H_{3'}) ; 3,76-3,37 (m, 16H, O-CH₂-CH₂-O) ; 3,32 (m, 2H, H_{14'}) ; 3,11 (m, 1H, H_{B4}) ; 2,89 et 2,75 (système ABX, 2H, ²*J*_{AB}= 5 Hz, ³*J*_{AX} = 12 Hz, ³*J*_{BX} = 0 Hz, H_{B6}) ; 2,59 (s, 3H, CH₃) ; 2,16 (t, 2H, *J*= 8 Hz, H_{B10}) ; 1,64-1,40 (m, 6H, H_{B9, B8, B7}).

### • Composé hydrazone 22 :

La cétone **21** est dissoute dans 20 mL d'EtOH absolu. On chauffe à reflux à 75 -80°C. On ajoute ensuite l'hydrazine (816 µL ; 16,81 mmol) et on laisse sous agitation pendant 3 h. Après filtration, on évapore à sec, on redissout dans l'éthanol jusqu'à l'obtention d'une mousse blanche collante. Dans un deuxième temps, on dissout cette mousse dans 50 mL de chloroforme puis on ajoute 20 mL d'une solution de NaHCO₃ saturée. On lave bien puis on récupère la phase organique. On la sèche sur Na₂CO₃ anhydre et après filtration, on évapore à sec afin d'obtenir une nouvelle mousse collante. Celle-ci correspond au produit **22** (842 mg g ; 1,38 mmol) et est obtenue avec un rendement de 66 %.

RMN ¹H (300MHz, CDCl₃) δ = 8,81 (s large, 1H, NH) ; 8,82 (s, 1H, Ar-H₂) ; 7,64 (d, 2H, *J* = 8 Hz, Ar-H₄) ; 7,32 (m, 4H, Ar-H_{5,6}) ; 6,43 (t, 1H, NH_{15'}) ; 5,89 (s large, 1H, NH_{B1}) ; 5,46 (s large, 2H, NH₂) ; 4,99 (s large, 1H, NH_{B3}) ; 4,44 (m, 1H, H_{B6a}) ; 4,27 (m, 1H, H_{B3a}) ; 4,11 (s, 2H, H_{3'}) ; 3,70-3,37 (m, 16H, O-CH₂-CH₂-O) ; 3,32 (m, 2H, H_{14'}) ; 3,08 (m, 1H, H_{B4}) ; 2,87 et 2,67 (système ABX, 2H, ²*J*_{AB}= 5 Hz, ³*J*_{AX} = 12 Hz, ³*J*_{BX} = 0 Hz, H_{B6}); 2,11 (m, 5H, CH₃ et H_{B10}) ; 1,64-1,40 (m, 6H, H_{B9, B8, B7}).

### • Composé diazo 23 :

L'hydrazone **22** (100mg ; 0,164 mmol) est dissoute dans 1 mL de DMF sous argon. On ajoute 80 mg de MnO₂ activé et on laisse sous forte agitation pendant 30 min. Le mélange est filtré à travers une couche mixte Célite (3 cm) - tamis moléculaire en poudre (1 cm). La solution est ensuite évaporée à sec. L'huile obtenue en fin d'évaporation est triturée jusqu'à l'obtention d'un poudre rose correspondant au composé 23 (78 mg ; 0,128 mmol ; 78 %).

RMN ¹H (300MHz, DMSO-d₆) δ = 9,60 (s large, 1H, NH) ; 7,89 (s, 1H, Ar-H₂) ; 7,76 (t, 1H, NH_{15'}) ; 7,35-7,25 (m, 4H, Ar-H_{5,6}) ; 6,64 (d, 2H, *J* = 8 Hz, Ar-H₄) ; 6,36 (s large, 1H, NH_{B1}) ; 6,32 (s large, 1H, NH_{B3}) ; 4,28 (m, 1H, H_{B6a}) ; 4,08 (m, 1H, H_{B3a}) ; 4,06 (s, 2H, H_{3'}) ; 3,55-3,31 (m, 16H, O-CH₂-CH₂-O) ; 3,17 (m, 2H, H_{14'}) ; 3,08 (m, 1H, H_{B4}) ; 2,80 et 2,59 (système ABX, 2H, ²*J*_{AB}= 5 Hz, ³*J*_{AX} =12 Hz, ³*J*_{BX} = 0 Hz, H_{B6}) ; 2,13 (m, 5H, CH₃) ; 2,13 (t, 2H, *J*= 8 Hz, H_{B10}) ; 1,99-1,30 (m, 6H, H_{B9, B8, B7}).

La réactivité du composé **23** a été testée sur 3'-Uridine-monophosphate et suivie par électrophorèse capillaire. Les conditions d'analyse sont celles de l'exemple 6.1. Les résultats montrent un temps de demi vie de 30 minutes.
La stabilité du réactif est supérieure à 1 mois à -20°C.

### Exemple 22.3 : Synthèse du para-Bio-EG3-PMDAM :

### Schéma de synthèse :

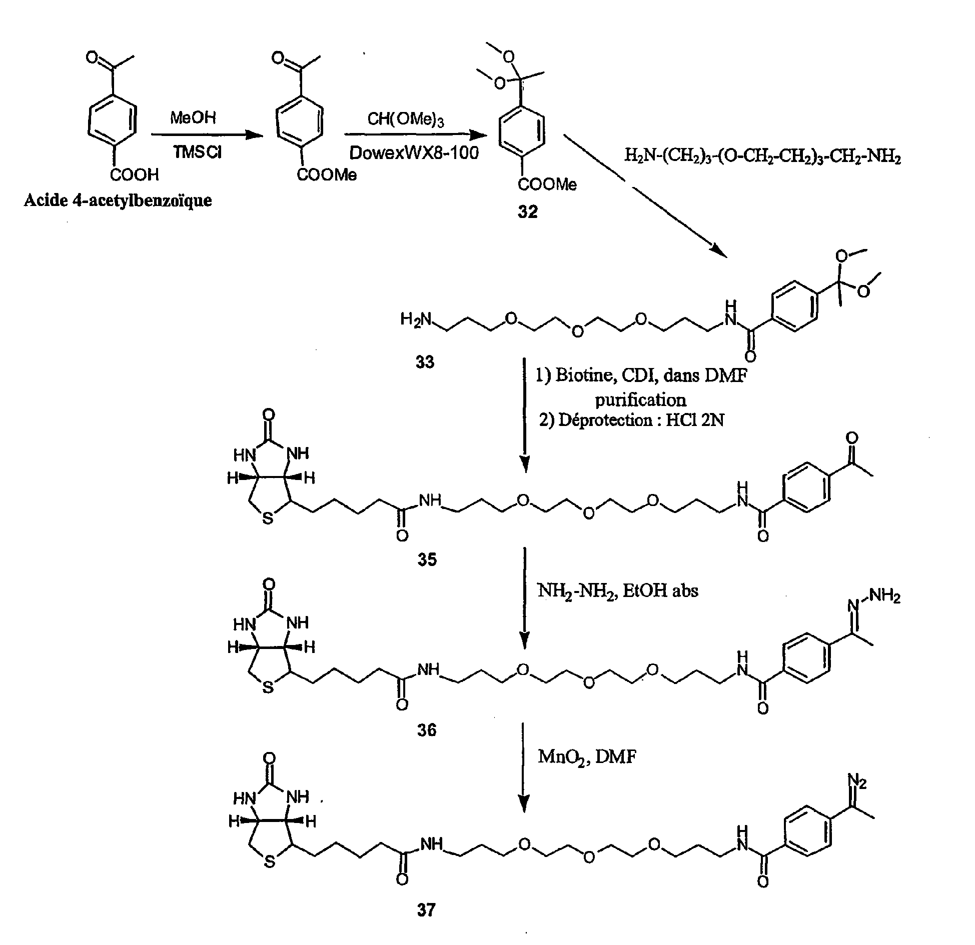

### • Protection de l'acide 4-acétylbenzoïque :

On dissout l'acide 4-acétylbenzoïque (1 g ; 6,1 mmol) dans une solution de chlorure de triméthylsilyle (TMSCl, 10 g ; 92 mmol) dans 5 mL de MeOH. Le mélange est chauffé à 90°C pendant une nuit. Après évaporation, un solide blanc correspondant au composé **31** (1,21 g ; 5,75 mmol) est isolé, caractérisé par RMN et utilisé tel quel pour la réaction suivante.
RMN ¹H (200MHz, CDCl₃) δ = 8,08 (d, 2H, *J* = 8 Hz, Ar-H_{2,6}) ; 7,59 (d, 2H, *J* = 8 Hz, Ar-H_{3,5}) ; 3,18 (s, 6H, -O-CH₃) ; 1,53 (s, 3H, CH₃).

### • Composé 32 :

On dissout le composé **31** (1,21 g ; 5,75 mmol) dans 5 ml d'orthoformiate de triméthyle en présence de Dowex 50WX8-100 (0,3 g). Le mélange est chauffé à 60°C pendant une nuit, puis filtré et évaporé pour donner le composé **32** (1,19 g ; 5,3 mmol) avec un rendement de 87 %.
RMN ¹H (200MHz, CDCl₃) δ = 8,00 (d, 2H, *J* = 8 Hz, Ar-H_{2,6}) ; 7,54 (d, 2H, *J* = 8 Hz, Ar-H_{3,5}) ; 3,89 (s, 1H, CO-O- CH₃) ; 3,16 (s, 6H, -O-CH₃) ; 1,51 (s, 3H, CH₃).

### • Composé 33 :

On solubilise le composé **32** (1,17 g ; 5,22 mmol) dans 5 mL (22,7 mmol) de 4,7,10-trioxa-1,13-tridécanediamine. La solution obtenue est chauffée à 140°C pendant 4 h. Le mélange est ensuite dissous dans 30 mL de DCM et lavé 3 fois avec 10 mL d'eau. La phase organique est séchée avec Mg SO₄, puis évaporée jusqu'à l'obtention d'une huile correspondant au produit 33 (1,44 g ; 3,49 mmol) avec un rendement de 67 %.

RMN ¹H (200MHz, CDCl₃) δ = 7,76 (d, 2H, *J* = 8 Hz, Ar-H_{2,6}) ; 7,51 (d, 2H, *J =* 8 Hz, Ar-H_{3,5}) ; 3,62-3,47 (m, 14H, H_{7',8',10',11'} et H_{5',13'} et H_{3'}) ; 3,15 (s, 6H, -O-CH₃) ; 2,73 (m, 2H, H_{15'}) 1,88 (m, 2H, H_{4'}) ; 1,65 (m, 2H, H_{14'}) ; 1,38 (s large, 2H, NH₂).

### • Composé biotinylé 34 :

La biotine (780 mg ; 3,19 mmol) est mise en suspension dans 13 mL de DMF. On ajoute ensuite 590 mg (3,60 mmol) de CDI. Cette solution est laissée sous agitation pendant 30 min à température ambiante. Le composé 33 est dissous dans 1 mL de DMF, puis ajouté petit à petit à la solution précédente. Le mélange ainsi obtenu est laissé sous agitation pendant 1 h à température ambiante. Après évaporation du DMF, une purification sur colonne par chromatographie-flash (colonne de 35 mm de diamètre) est réalisée avec 500 mL de MeOH-DCM 6 %, puis avec 250 mL de MeOH-DCM 8 %, et enfin 250 mL de MeOH-DCM 8 %. Les fractions correspondant au produit **34** sont rassemblées, puis évaporées à sec pour donner 1,05 g d'huile avec un rendement estimé à 30 %.

RMN ¹H (300MHz, CDCl₃) δ = 8,49 (s large, 1H, NH_{imidazole}) ; 7,79 (d, 2H, *J* = 8 Hz, Ar-H_{2,6}) ; 7,66 (s, 1H, H_{imidazole}) ; 7,50 (d, 2H, *J* = 8 Hz, Ar-H_{3,5}) ; 7,38 (t, 1H, NH_{2'}) ; 7,11 (s, 2H, H_{imidazole}) ; 6,67 (t, 1H, NH_{16'}), 5,99 (s large, 1H, NH_{B1}) ; 5,15 (s large, 1H, NH_{B3}) ; 4,46 (m, 1H, H_{B6a}) ; 4,27 (m, 1H, H_{B3a}) ; 3,61-3,45 (m, 14H, H_{7',8',10',11'} et H_{5',13'} et H_{3'}) ; 3,28 (m, 2H, H_{15'}) ; 3,15 (s, 6H, -OCH₃) ; 2,85 (m, 1H, H_{B4}) ; 2,85 et 2,69 (système ABX, 2H, ²*J*_{AB}= 5 Hz, ³*J*_{AX} = 12 Hz, ³*J*_{BX} = 0 Hz, H_{B6}) ; 2,14 (t, 2H, *J*= 8 Hz, H_{B10}) ;1,86 (m, 2H, H_{4'}) ; 1,69 (m, 2H, H_{14'}) ; 1,49 (s, 3H, CH₃); 1,42-1,39 (m, 6H, H_{B7, B8, B9}).

### • Composé 35 :

L'acétal **34** est dissous dans 45 mL de chloroforme, puis 10 mL de HCl 2N sont ajoutés . Le mélange diphasique est vigoureusement agité pendant 5 min. On récupère la phase organique qu'on sèche sur NaHCO₃ anhydre. On filtre, on évapore, et le composé **35** est obtenu sous forme d'un solide jaune clair (504 mg ; 0,87 mmol) avec un rendement global de 27 % à partir de la biotine.

RMN ¹H (300MHz, CDCl₃) δ = 7,97 (d, 2H, *J* = 8 Hz, Ar-H_{2,6}) ; 7,91 (d, 2H, *J* = 8 Hz, Ar-H_{3,5}) ; 7,51 (t, 1H, NH_{2'}) ; 6,50 (t, 1H, NH_{16'}), 6,05 (s large, 1H, NH_{B1}) ; 5,23 (s large, 1H, NH_{B3}) ; 4,45 (m, 1H, H_{B6a}) ; 4,27 (m, 1H, H_{B3a}) ; 3,62-3,56 (m, 10H, H_{7' 8',10',11'} et H_{5'}) ; 3,48-3,46 (m, 4H, H_{3',13'}) ; 3,27 (m, 2H, H_{15'}) ; 3,10 (m, 1H, H_{B4}) ; 2,85 et 2,71 (système ABX, 2H, ²*J*_{AB}= 5 Hz, ³*J*_{AX} = 12 Hz, ³*J*_{BX} = 0 Hz, H_{B6}); 2,60 (s, 3H, CH₃); 2,14 (t, 2H, *J* = 8 Hz, H_{B10}) ;1,89 (m, 2H, H_{4'}) ; 1,72-1,61 (m, 6H, H_{14'}, H_{B7 B9}) ; 1, 40 (m, 2H, H_{B8}).

### • Composé hydrazone 36 :

La cétone **35** (500 mg ; 0,864 mmol) est dissoute dans 11 mL de EtOH absolu. L'hydrazine (335 µL ; 6,911 mmol) est ajoutée, puis le mélange réactionnel est chauffé à reflux pendant 1 h. L'huile obtenue après évaporation, est dissoute dans EtOH abs, pour être de nouveau évaporée. On obtient alors une mousse collante correspondant au produit 36 (488 mg ; 0,823 mmol) avec un rendement de 95 %.

RMN ¹H (300MHz, CDCl₃) δ = 7,76 (d, 2H, *J* = 8 Hz, Ar-H_{2,6}) ; 7,67 (d, 2H, *J* = 8 Hz, Ar-H_{3,5}) ; 7,29 (t, 1H, NH_{2'}) ; 6,46 (t, 1H, NH_{16'}), 5,98 (s large, 1H, NH_{B1}) ; 5,55 (s large, 2H, NH₂) ; 5,14 (s large, 1H, NH_{B3}) ; 4,45 (m, 1H, H_{B6a}) ; 4,24 (m, 1H, H_{B3a}) ; 3,62-3,51 (m, 10H, H_{7', 8',10',11'} et H_{5'}) ; 3,47-3,45 (m, 4H, H_{3',13'}) ; 3,27 (m, 2H, H_{15'}) ; 3,07 (m, 1H, H_{B4}) ; 2,84 et 2,69 (système ABX, 2H, ²*J*_{AB}= 5 Hz, ³*J*_{AX} = 12 Hz, ³*J*_{BX} = 0 Hz, H_{B6}) ; 2,11 (t, 2H, *J*= 8 Hz, H_{B10} et s, 3H, CH₃) ; 1,86 (m, 2H, H_{4'}) ; 1,72-1,59 (m, 6H, H_{14'}, H_{B7, B9}) ; 1, 21 (m, 2H, H_{B8}).

### • Composé diazo 37 :

On solubilise l'hydrazone **36** (200 mg ; 0,337 mmol) dans 5 mL de DMF. On ajoute alors MnO₂ (450 mg ; 5,17 mmol). Après 15 min d'agitation à température ordinaire, le mélange est filtré sur millipore contenant de la célite (épaisseur : 2 cm) et du tamis moléculaire en poudre 3 Å (0,5 cm). Le mélange réactionnel est évaporé à sec. L'huile résiduelle obtenue est lavée à l'éther trois fois de suite, jusqu'à l'obtention d'une poudre. Le composé **37** (290 mg, 0,491 mmol) est obtenu sous forme d'un solide rose avec un rendement de 93 %.

RMN ¹H (300MHz, DMSO-d₆) δ = 8,33 (t, 1H, NH_{2'}) ; 7,83 (d, 2H, *J*= 8 Hz, Ar-H_{2,6}) ; 7,73 (t, 1H, NH_{16'}) ; 6,98 (d, 2H, *J*= 8 Hz, Ar-H_{3,5}) ; 6,39 (s large, 1H, NH_{B1}) ; 6,33 (s large, 1H, NH_{B3}) ; 4,30 (m, 1H, H_{B6a}) ; 4,12 (m, 1H, H_{B3a}) ; 3,51-3,45 (m, 16H, H_{7',8', 10', 11'} et H_{5'} et H_{15'} et H_{3',13'}) ; 3,07 (m, 1H, H_{B4}) ; 2,79 et 2,58 (système ABX, 2H, ²*J*_{AB}= 5 Hz, ³*J*_{AX} = 12 Hz, ³*J*_{BX} = 0 Hz, H_{B6}) ; 2,14 (s, 3H, CH₃) ; 2,04 (t, 2H, *J =* 8 Hz, H_{B10}) ;1,77 (m, 2H, H_{4'}) ; 1,62-1,48 (m, 6H, H_{14'}, H_{B7, B9}) ; 1, 31 (m, 2H, H_{B8}).

La réactivité du composé **37** a été testée sur 3'-Uridine-monophosphate et suivie par électrophorèse capillaire. Les conditions d'analyse sont celles de l'exemple 6.1. Les résultats montrent un temps de demi vie de 60 minutes.
Le réactif est stable à -20°C pendant au moins 1 mois.

### Exemple 22.4 : Synthèse du méta-Bio-EG3-PDAM :

### Schéma de synthèse :

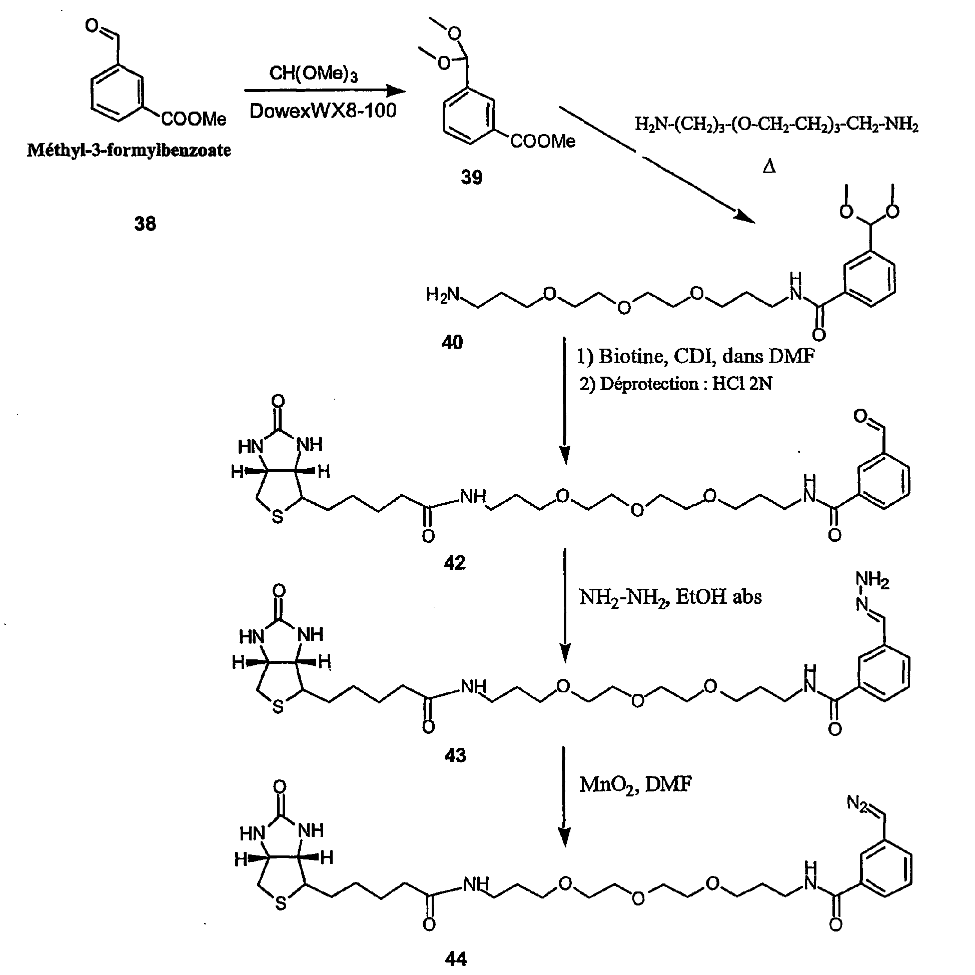

### • Protection du méthyl-3-formylbenzoate 38 :

La résine Dowex 50WX8-100 (2 g) est mise en solution dans 25 mL de MeOH et 25 mL d'orthoformiate de triméthyle puis laissé sous agitation pendant 15 min. Après décantation, on lave la résine deux fois de suite avec 20 mL de MeOH. On met ensuite cette résine dans 100 mL de MeOH, 50 mL de CH(OMe)₃ et on ajoute 7,12 g (43,4 mmol) de méthyl-3-formylbenzoate. La solution est laissée 15 min sous agitation, puis filtrée sur papier plissé avant évaporation. Le produit **39** (9 g ; 43,1 mmol) est isolé sous forme d'un liquide jaune clair avec un rendement de 99 %.

RMN ¹H (200MHz, CDCl₃) δ = 8,10 (s, H, Ar-H₂) ;7,9 (d, H, *J*= 8 Hz, Ar-H₄) ; 7,63 (d, H, *J* = 8 Hz, Ar-H₆); 7,42 (t, H, *J* = 8 Hz, Ar-H₅); 5,40 (s, 1H, CH); 3,90 (s, 3H, -CO-O-CH₃); 3,31 (s, 6H, -O-CH₃).

### • Composé 40 :

On solubilise le composé **39** (2 g ; 9,5 mmol) dans 10,4 mL (47,6 mmol) de 4,7,10-Trioxa-1,13-tridécanediamine. La solution obtenue est chauffée à 165°C pendant 2 h. Le mélange est ensuite dissous dans 80 mL de DCM et lavé 4 fois avec 20 mL d'eau. Après séchage sur MgSO₄ et évaporation, le produit **40** est isolé avec un rendement de 60 % (2,27 g ; 5,69 mmol).

RMN ¹H (200MHz, CDCl₃) δ = 7,84 (s, H, Ar-H₂) ;7,75 (d, H, *J*= 8 Hz, Ar-H₄) ; 7,53 (d, H, *J* = 8 Hz, Ar-H₆) ; 7,39 (t, H, *J* = 8 Hz, Ar-H₅) ; 5,38 (s, 1H, CH) ; 3,64-3,43 (m, 14H, H_{7',8',10',11'} et H_{5', 13'} et H_{3'}) ; 3,29 (s, 6H, -O-CH₃) ; 2,72 (m, 2H, H_{15'}) 1,87 (m, 2H, H_{4'}) ; 1,64 (m, 2H, H_{14'}); 1,30 (s large, 2H, NH₂).

### • Composé biotinylé 41 :

La D-biotine (344 mg ; 1,40 mmol) est mise en suspension dans 4 mL de DMF puis 250 mg (1,54 mmol) de CDI sont ajoutés. Cette solution est laissée sous agitation pendant 30 min à température ambiante. Le composé **40** (616 mg ; 1,54 mmol) est dissous dans 2 mL de DMF, puis ajouté petit à petit à la solution précédente. Le mélange ainsi obtenu est laissé sous agitation pendant 50 min à température ambiante. Après évaporation, une purification sur colonne par chromatographie-flash (colonne de 30 mm de diamètre) est réalisée avec 750 mL de MeOH-DCM 10 %, puis avec 250 mL de MeOH-DCM 15 %. Les fractions correspondant au produit **41** sont rassemblées puis évaporées à sec pour donner 740 mg d'huile avec un rendement estimé à 50 %.

RMN ¹H (200MHz, CDCl₃) δ = 7,87 (s, H, Ar-H₂) ;7,78 (d, H, *J*= 8 Hz, Ar-H₄) ; 7,65 (s, 1H, H_{imidazole}) ; 7,53 (d, H, *J* = 8 Hz, Ar-H₆) ; 7,39 (t, H, *J* = 8 Hz, Ar-H₅) ; 7,07 (s, 2H, H_{imidazole}) ; 6,65 (t, 1H, NH_{16'}), 5,95 (s large, 1H, NH_{B1}); 5,38 (s, 1H, CH) ; 5,15 (s large, 1H, NH_{B3}) ; 4,43 (m, 1H, H_{B6a}) ; 4,27 (m, 1H, H_{B3a}) ; 3,59-3,44 (m, 14H, H_{7',8',10',11'} et H_{5',13'} et H_{3'}) ; 3,29 (m, 8H, H_{15'} et 2-O-CH₃);3,07 (m, 1H, H_{B4}) ; 2,84 et 2,66 (système ABX, 2H, ²*J*_{AB}= 5 Hz, ³*J*_{AX} = 12 Hz, ³*J*_{BX} = 0 Hz, H_{B6}) ; 2,13 (t, 2H, *J*= 8 Hz, H_{B10}) ;1,85 (m, 2H, H_{4'}) ; 1,66 (m, 2H, H_{14'}) ; 1,40-1,37 (m, 6H, H_{B7,B8,B9}).

### • Composé aldéhydique 42 :

L'acétal **41** est dissous dans 20 mL de chloroforme, puis 5 mL de HCl 2N sont ajoutés . Le mélange diphasique est vigoureusement agité pendant 15 min. On récupère la phase organique qu'on sèche sur NaHCO₃ anhydre. On filtre, on évapore et le composé **42** est obtenu sous forme d'une huile jaune (593 mg ; 1,02 mmol) avec un rendement global de 87 % à partir de la biotine.

RMN ¹H (300MHz, CDCl₃) δ = 10,04 (s, 1H, CHO) ;8,34 (s, H, Ar-H₂) ; 8,16 (d, H, *J*= 8 Hz, Ar-H₄) ; 7,96 (d, H, *J*= 8 Hz, Ar-H₆) ; 7,72 (t, 1H, NH_{2'}) ; 7,39 (t, H, *J*= 8 Hz, Ar-H₅) ; 6,51 (t, 1H, NH_{16'}) ; 6,00 (s large, 1H, NH_{B1}) ; 5,30 (s large, 1H, NH_{B3}) ; 4,46 (m, 1H, H_{B6a}) ; 4,27 (m, 1H, H_{B3a}) ; 3,66-3,56 (m, 10H, H_{7', 8',10',11'} et H_{5'}) ; 3,50-3,29 (m, 4H, H_{3',13'}) ; 3,28 (m, 2H, H_{15'}); 2,95 (m, 1H, H_{B4}); 2,84 et 2,71 (système ABX, 2H, ²*J*_{AB}= 5 Hz, ³*J*_{AX} = 12 Hz, ³*J*_{BX} = 0 Hz, H_{B6}) ; 2,15 (t, 2H, *J =* 8 Hz, H_{B10}) ;1,89 (m, 2H, H_{4'}) ; 1,72-1,63 (m, 6H, H_{14',} H_{B7, B9}) ; 1, 23 (m, 2H, H_{B8}).

### • Composé hydrazone 43 :

L'aldéhyde **42** (593 mg ; 1,02 mmol) est dissous dans 10 mL d'éthanol absolu. L'hydrazine (400 µL ; 8,19 mmol) est ajoutée, puis le mélange réactionnel est chauffé à reflux pendant 50 min. L'huile jaune obtenue après évaporation, est triturée avec de l'éther jusqu'à l'obtention d'une poudre beige correspondant au produit **43** (404 mg ; 0,68 mmol) avec un rendement de 66 %. Une purification sur colonne par chromatographie-flash (colonne de 15 mm de diamètre) est réalisée ensuite sur un échantillon de 150 mg (0,253 mmol) avec 200 mL de MeOH-DCM 20% . Les fractions sont rassemblées puis évaporées à sec pour donner 144 mg de produit **43** avec un rendement de 76 %.

RMN ¹H (300MHz, CDCl₃) δ = 7,95 (s, H, Ar-H₂) ; 8,16 (d, H, *J=* 8 Hz, Ar-H₄) ; 7,76 (s, 1H, CH) ; 7,96 (d, H, *J* = 8 Hz, Ar-H₆) ; 7,38 (t, H, *J=* 8 Hz, Ar-H₅) ; 6,45 (t, 1H, NH_{16'}) ; 5,98 (s large, 1H, NH_{B1}) ; 5,72 (s large, 2H, NH₂) ; 5,18 (s large, 1H, NH_{B3}) ; 4,44 (m, 1H, H_{B6a}) ; 4,26 (m, 1H, H_{B3a}) ; 3,62-3,56 (m, 10H, H_{7', 8',10',11'} et H_{5'}) ; 3,48-3,45 (m, 4H, H_{3',13'}) ; 3,27 (m, 2H, H_{15'}) ; 3,07 (m, 1H, H_{B4}) ; 2,84 et 2,68 (système ABX, 2H, ²*J*_{AB}= 5 Hz, ³*J*_{AX} = 12 Hz, ³*J*_{BX} = 0 Hz, H_{B6}) ; 2,11 (t, 2H, *J=* 8 Hz, H_{B10}) ;1,86 (m, 2H, H_{4'}) ; 1,72-1,59 (m, 6H, H_{14'}, H_{B7, B9}) ; 1, 21 (m, 2H, H_{B8}).

### • Composé diazo 44 :

On solubilise l'hydrazone **43** (100 mg; 0,187 mmol) dans 4 mL de DMF. On ajoute alors MnO₂ (200 mg ; 2,3 mmol). Après 13 min d'agitation à température ordinaire, le mélange est filtré sur millipore contenant de la célite (épaisseur : 2 cm) et du tamis moléculaire en poudre 3 Å (0,5 cm). Le mélange réactionnel est évaporé à sec. L'huile résiduelle obtenue est lavée à l'éther, trois fois de suite. Le composé **44** (290 mg, 0,491 mmol) est obtenu sous forme d'un solide orange avec un rendement de 83 %.
RMN ¹H (300MHz, DMSO-d₆) δ = 8,39 (t, 1H, NH_{2'}) ; 7,78 (t, 1H, NH_{16'}) ; 7,39-7,34 (m, Ar-H) ; 7,09 (d, Ar-H) ; 6,38 (s large, 1H, NH_{B1}) ; 6,32 (s large, 1H, NH_{B3}) ; 5,78 (s, 1H, CH-N₂) ; 4,27 (m, 1H, H_{B6a}) ; 4,11 (m, 1H, H_{B3a}) ; 3,51-3,44 (m, 10H, H_{7', 8', 10', 11'} et H_{5'}) ; 3,37 (m, 2H, H_{15'}) ; 3,32 (m, 4H, H_{3',13'}) ; 3,05 (m, 1H, H_{B4}) ; 2,79 et 2,58 (système ABX, 2H, ²*J*_{AB}= 5 Hz, ³*J*_{AX} = 12 Hz, ³*J*_{BX} = 0 Hz, H_{B6}) ; 2,02 (t, 2H, *J* = 8 Hz, H_{B10}) ;1,69 (m, 2H, H_{4'}) ; 1,59-1,48 (m, 6H, H_{14'}, H_{B7, B9}) ; 1, 25 (m, 2H, H_{B8}).

La stabilité du produit est supérieure à 1 mois à -20°C.

### Exemple 23 : Synthèse du para-Cy5-EG3-PDAM :

Comme cela a déjà été évoqué dans l'exemple 2, la biotine peut être remplacée par un autre marqueur tel que le Cy5. Cet exemple montre que l'on peut également relier la fonction diazo, portée par le PDAM, à ce marqueur Cy5 par l'intermédiaire d'un bras de liaison polyéthylène glycol.

Schéma de synthèse : Le contre ion I⁻ n'est pas représenté sur les formules 46, 47, 50' et 51.

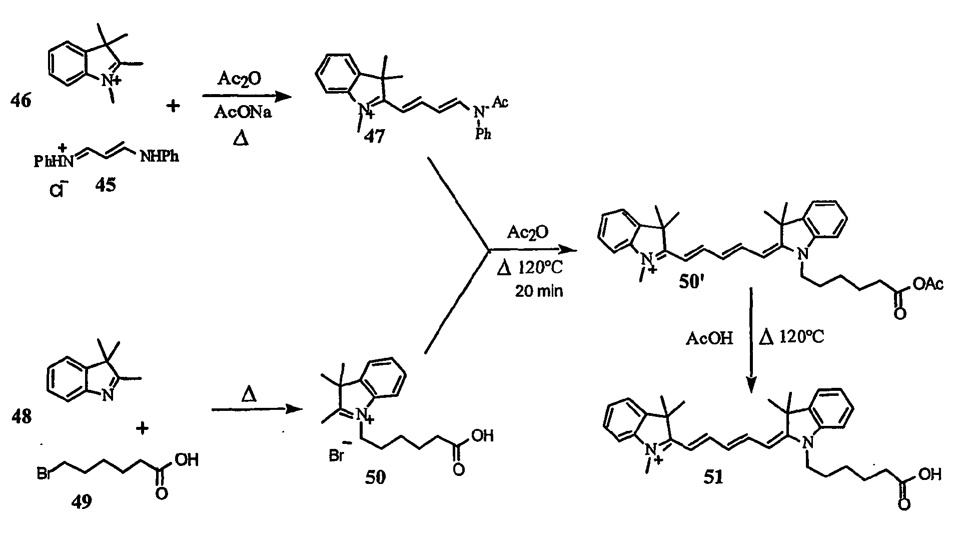

### • Iodure de 2-[4-(N-acétyl-N-phénylamino)buta-1,3-diényl]-1,2,3,3-tétraméthyl[3H]indolium 47 :

Le mélange du monochlorhydrate de malonaldehyde-bis(phénylimine) **45** (13 g ; 50,2 mmol), de NaOAc (6,0 g ; 69,7 mmol) et de l'iodure de 1,2,3,3- tétraméthyl[3H]indolium **46** (3,01 g ; 10 mmol) dans de l'anhydride acétique (50 mL) est chauffé à 100 °C pendant 20 min précisément. Après refroidissement, on ajoute de l'éther (350 mL) et le solide brun précipité est filtré, lavé avec de l'éther (3 × 100 mL). Le solide est redissout dans 150 mL de CH₂Cl₂, filtré (élimination des sels minéraux) puis précipité avec 350 mL d'éther pour donner un solide brun (3,54 g, 54 %).

RMN ¹H (CDCl₃) : δ = 8,64 (d ; 1H ; *J=* 12 Hz ; 1-H) ; 8,14 (t ; 1H ; *J* = 16 ; 12 Hz ; 3-H) ; 7,63-7,19 (m ; 9H) ; 6,90 (d ; 1H ; *J* = 15 Hz ; 4-H) ; 5,82 (t ; 1H ; *J* = 12 ; 13 Hz ; 2-H) ; 4,06 (s ; 3H ; NCH₃) ; (2,16 (s ; 3H ; -COCH₃) ; 1,74 (s ; 6H ; CH₃).

### • Bromure de 1-(5-carboxypentyl)-2,3,3-triméthyl[3H]indolium 50 :

On mélange le 2,3,3-triméthylindole **48** (10,0 g; 62,8 mmole) et l'acide 6-bromohexanoïque **49** (12,3 g ; 62,8 mmole) sans solvant et chauffe à 110 °C pendant 12 h sous argon. Le mélange réactionnel pâteux rouge violet est lavé avec de l'acétate d'éthyle (2 × 60 mL, on triture la pâte avec la spatule et on décante le surnageant), puis avec de l'acétone (50 mL, la pâte solidifie). Le solide rose est filtré puis séché sous vide (16,0 g ; 73 %).

### • Composé Cy5COOH 51 :

Le mélange de l'iodure **47** (2,5 g ; 5,3 mmole), du bromure **50** (1,87 g ; 5,3 mmole) et de NaOAc (1,08 g ; 12,1 mmole) dans de l'anhydride acétique (11 mL) est chauffé à 120 °C pendant 25 min. Après refroidissement, on ajoute de l'éther (200 mL) et le précipité est filtré et lavé avec de l'éther (3 **×** 50 mL). Le solide correspondant au produit **50'** est dissous dans 100 mL de CH₂Cl₂ puis évaporé. On le dissout ensuite dans 15 mL d'acide acétique et l'on agite pendant 30 min à 120 °C. Le précipité correspondant au produit **51** est ainsi obtenu après ajout de 200 mL d'éther et filtration sur fritté, avec un rendement de 84 % (2,71 g ; 4,44 mmol).

RMN ¹H (CDCl₃) : δ = 8,03 (t ; 2H ; *J* = 10 ; 11 Hz, 2-H, 4-H) ; 7,38-6,91 (m ; 9H ; Ar-H, 3-H) ; 6,41 (d ; 1H ; *J*= 14 Hz ; 1-H) ; 6,31 (d ; 1H ; *J*= 13 Hz ; 5-H) ; 4,07 (t ; 2H, *J* = 7 ; 7 Hz ; α-CH₂) ; 3,68 (s ; 3H ; NCH₃) ; 2,47 (t ; 2H, *J*= 7 ; 7 Hz ; ε-CH₂) ; 1,71 (m ; 18H ; CH₃, β,γ et δ-CH₂).

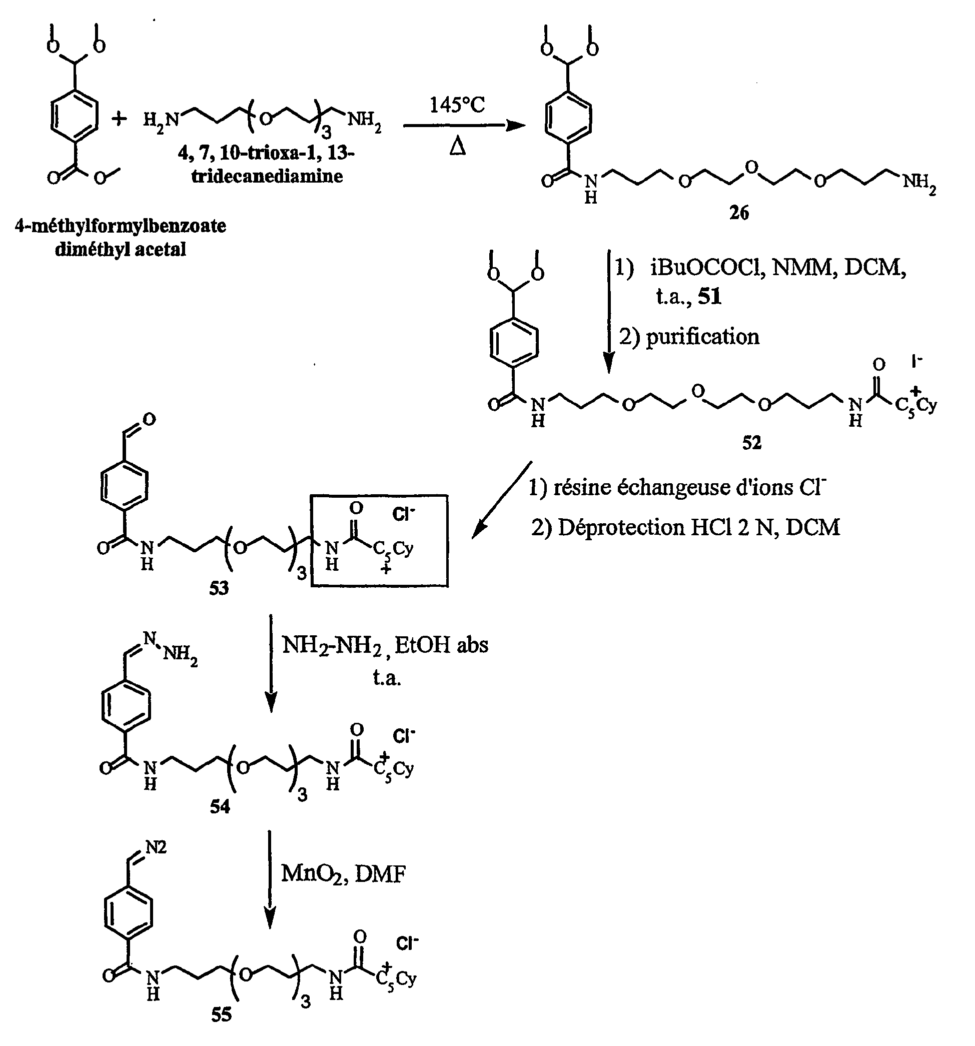

### • Couplage du composé 26 avec le Cy5COOH 51 (produit 52) :

À une solution de Cy5COOH **51** (1,5 g ; 2,46 mmole) dans 15 mL de CH₂Cl₂, on ajoute *N-*méthylmorpholine (NMM, 405 µL; 3,68 mmole). La solution est refroidie avec un bain de glace et mise sous argon, puis on ajoute le chloroformiate d'isobutyle (494 µL ; 3,81 mmole). Après 10 min d'agitation, on additionne l'amine **26** (1,86 mg ; 4,67 mmole) diluée dans 8 mL de CH₂Cl₂. On laisse le mélange sous agitation à température ambiante pendant 1 h 30. On ajoute 20 mL de CH₂Cl₂ et on lave avec 25 mL de NaHCO₃ (1 N) trois fois de suite. Après séchage sur Na₂CO₃, on filtre la solution pour récupérer la phase dichlorométhane que l'on évapore.
Une purification sur colonne par chromatographie-flash (colonne de 45 mm de diamètre, fractions de 20 mL)) est réalisée avec comme éluant du MeOH-DCM 10% . Les fractions correspondant au produit **52** sont rassemblées puis évaporées à sec pour donner un solide bleu qui est dissous dans CH₂Cl₂. Le produit **52** est alors précipité et lavé à l'éther pour donner un produit bleu avec un rendement de 72 % (1,45 g ; 1,77 mmol).
Le produit **52** (iodure) est ensuite dissous dans 54 mL de méthanol puis passé sur colonne d'amberlite IRA900 (Cl⁻ ; 15 g). La solution méthanolique recueillie est évaporée à sec pour donner une huile collante que l'on redissout dans CH₂Cl₂ . L'évaporation permet l'obtention du produit **52'** avec un rendement de 87 %.

### • Aldéhyde 53 :

L'acétal **52'** est dissous dans 10 mL de DCM, puis 10 mL de HCL 2N sont ajoutés. La solution est laissée sous forte agitation pendant 3h 30 min. Après ajout de 20 mL de DCM, la phase dichlorométhane est récupérée puis séchée sur NaHCO₃. Le produit obtenu après évaporation est lavé avec de l'éther pour donner l'aldéhyde **53** avec un rendement de 90 % (1,18g ; 1,46 mmol).

### • Hydrazone 54 :

On dissout l'aldéhyde **53** (200 mg ; 0,247 mmole) dans 1 mL d'éthanol absolu et on ajoute l'hydrazine monohydratée (15,6 µL; 0,321 mmole). La solution est agitée à température ambiante pendant 30 min. On ajoute 8 mL d'éther ; on lave à l'éther par décantation, trois fois de suite puis on sèche sous vide. On obtient 172 mg d'hydrazine **54** (0,209 mmol ; 85 % de rendement) que l'on conserve au congélateur.

### • Diazo 55 :

À une solution de 20 mg (0,243 mmol) d'hydrazone **54** dans 2 mL de DMF, on ajoute 100 mg de MnO₂ et agite vigoureusement pendant 5 min sous argon à température ambiante. On filtre la suspension à travers une couche du célite (épaisseur : 2 cm) et du tamis moléculaire en poudre 3 Å (0,5 cm) et lave avec DMF. On évapore la solution puis on triture à l'éther. Le solide ainsi obtenu est séché. On obtient 18 mg (0,185 mmol ; 76%) de diazo **55.**
La stabilité du réactif est supérieure à 1 mois à -20°C.

### Exemple 24 : Synthèse du méta-Fluo-EG3-PMDAM :

Comme cela a déjà été évoqué dans l'exemple 23, la biotine peut être remplacée par un autre marqueur. Cet exemple montre que l'on peut également relier la fonction diazo, portée par le PMDAM, à ce marqueur fluorescéine par l'intermédiaire d'un bras de liaison polyéthylène glycol.

### Schéma de synthèse :

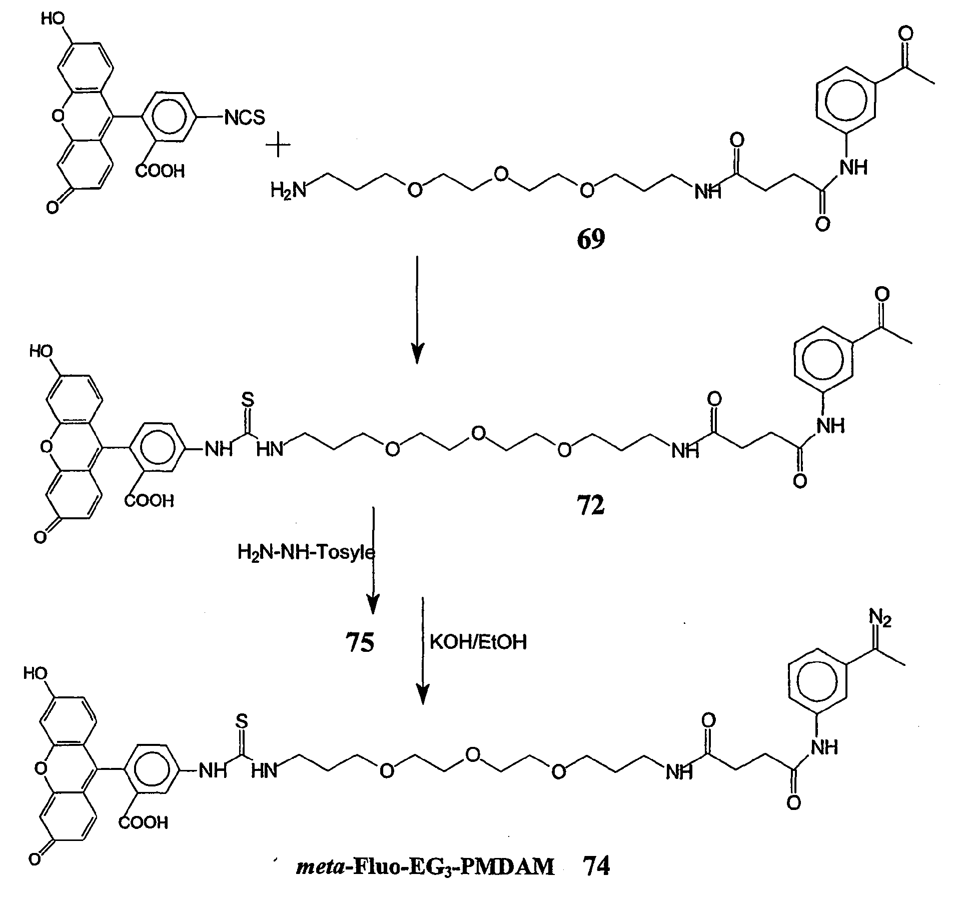

### Composé 72 :

On solubilise l'isothiocyanate de fluorescéine- (250 mg, 0,64 mmol) dans 1,6 ml de DMF anhydre, avec 2% de pyridine, sous argon. On ajoute le produit **69** (0,356 g, 0,81 mmol) dissout dans 1,6 ml de DMF anhydre. On laisse réagir 3,5 h à température ambiante, puis on évapore le DMF et on reprend dans 25 ml de H₂O. On fait trois extractions avec 50 ml de CH₂Cl₂, et la phase aqueuse est évaporée. On obtient 255 mg (48%) du produit **72.**

### Composé méta-Fluo-EG₃-Hydrazone-Tosyle 75 :

On dissout le composé **72** (255 mg, 0,31 mmol) dans 1,5 ml d'éthanol à reflux. On ajoute lap-toluen-sulfonyl-hydrazine (69,2 mg, 0,37 mmol) dans 1,5 ml d'éthanol et on laisse réagir pendant 6 h. On évapore a sec, et on lave le solide avec CH₂Cl₂, H₂O et éther. On obtient 18,5 mg (74%) du produit **75** sous forme d'une poudre orange.
RMN ¹H (200 MHz, DMSO-d₆): d = 1,6-1,8 (m, 4H); 2,13 (s, 1H); 2,28 (s, 1H); 2,36 (s, 1H); 2,80 (m, 1H); 3,07 (m, 2H); 3,46 (m, 12H); 6,5-6,7 (m, 6H); 7,1-8,3 (m, 9H).

### Composé méta-Fluo-EG₃-PMDAM 74 :

On solubilise l'hydrazone **75** (176 mg, 0,18 mmol) dans 720 µl d'une solution de KOH à 10% dans du méthanol anhydre. La solution est laissée a reflux pendant 3 h. On laisse refroidir la solution et un précipité apparaît. La solution est filtrée et évaporée a sec. Le résidu est lavé a l'éther et séché.
L'analyse par RMN montre la disparition des signaux a 2,36 et 2,13 ppm (correspondant aux Méthyles du tosyle et de l'hydrazone) et l'apparition d'un pic à 1,96 ppm (correspondant au méthyle du diazo).

### Exemple 25 : Intermédiaire diazométhyle permettant un marquage ultérieur :

Il peut être intéressant d'avoir non pas un marquage direct par le réactif de marquage diazométhyle portant aussi le marqueur R² mais de procéder en deux étapes avec un marquage indirect. Dans ce cas le réactif de marquage comprenant la fonction diazométhyle est dit préfonctionnalisé c'est à dire qu'il comprend aussi une fonction chimique apte à réagir ultérieurement avec des marqueurs directs ou indirects. La pré-fonctionnalisation peut intervenir par introduction d'une fonction réactive de covalence au sein du réactif de marquage qui peut réagir avec une fonction anti-réactive de covalence du marqueur direct ou indirect. Ces fonctions peuvent être constituées par une fonction chimique organique électrophile et une fonction chimique organique nucléophile, ou inversement.

Un exemple d'une telle stratégie de marquage est illustrée par le schéma ci-dessous 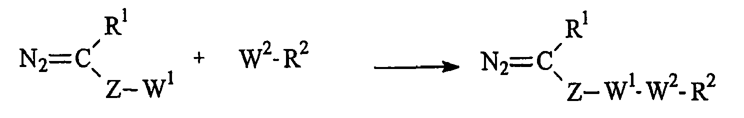 Dans lequel le réactif de marquage comprend, outre une fonction diazométhyle, une fonction W¹ électrophile ou nucléophile susceptible de réagir avec un marqueur R² comprenant une fonction W² complémentaire de W¹.
Par exemple si W¹ est une fonction méthylcétone ou aldéhyde, W² est une fonction alcoxyamine.
Dans un procédé de marquage d'une molécule biologique comme un acide nucléique, l'acide nucléique est mis en contact avec le réactif de marquage coprenant la fonction diazométhyle et dans une étape ultérieure, le marqueur W²- R² réagit avec l'acide nucléique par l'intermédiaire de la fonction W¹.
L'une des utilisations est par exemple constituée par un procédé d'amplification d'une séquence d'un acide nucléique ou par un procédé d'amplification du signal. Des informations complémentaires sur ce type de marquage peuvent être trouvées dans la demande de brevet WO-A-98/05766, sous priorité du 2 août 1996. et dans la demande de brevet WO-A-00/40590 sous priorité du 05 Janvier 1999.

### Exemple 25.1 : Synthèse du MeCO-PMDAM :

### Schéma de synthèse :

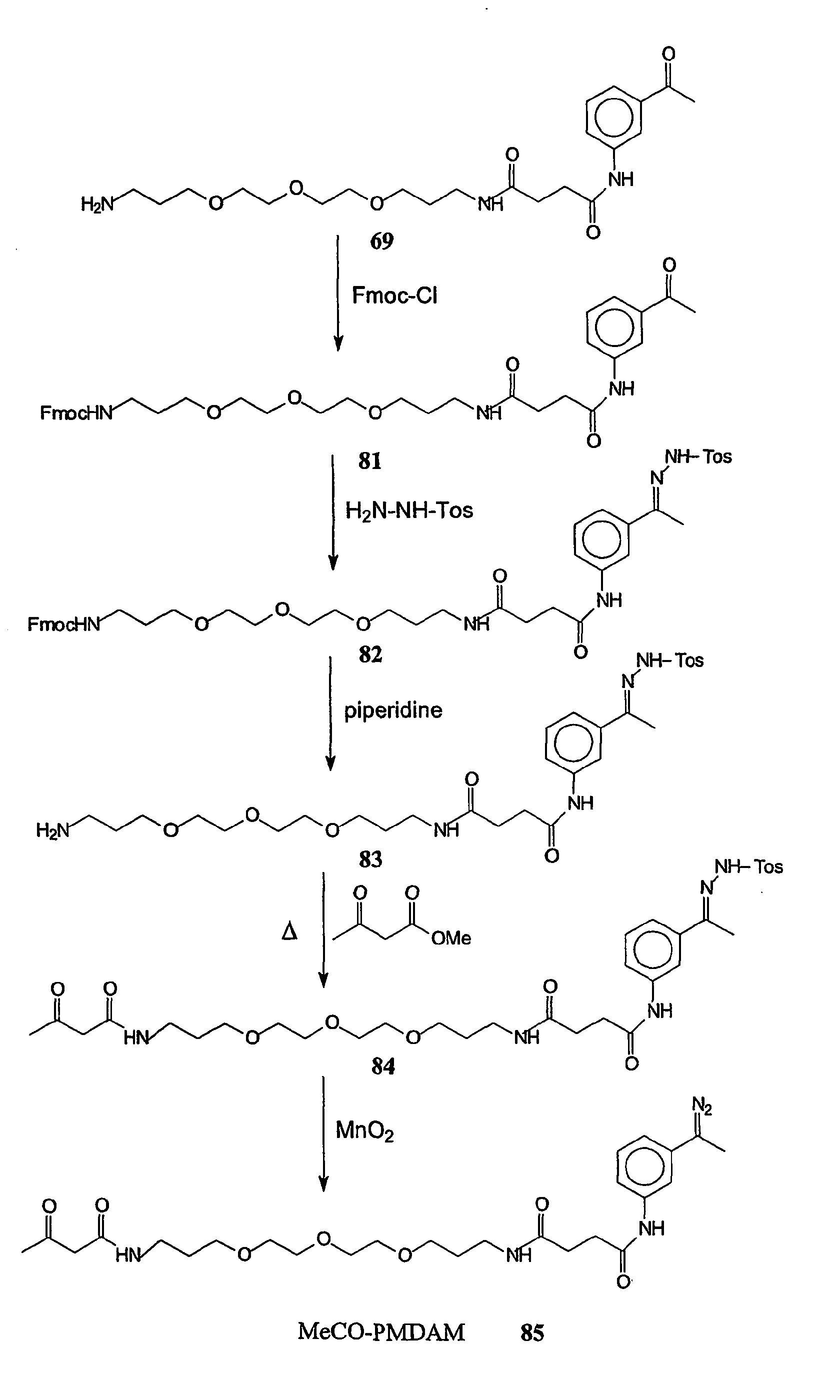

Le produit **85,** dont la synthèse est décrite dans cet exemple, permet de réaliser le marquage des acides nucléiques naturels, grâce à la réactivité de la fonction diazométhyl avec les groupements phosphate, et d'introduire ainsi une fonction méthylcétone, qui peut être utilisée par la suite pour introduire une molécule détectable (fluorescente, biotine) possédant un groupement alcoxyamine.

Cette synthèse est basée sur des méthodes connues et utilisées en routine en chimie. Le produit de départ est le même que pour la synthèse des marqueurs **71** et **74.** La première étape consiste en la protection de l'amine terminale par le Fluorenylmethyl-formate (Fmoc, **99**)**.** Le choix de ce groupement protecteur est basé sur sa stabilité et conditions de clivage.

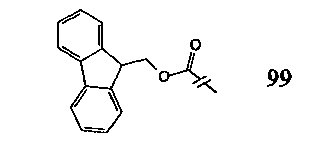

Après formation de l'hydrazone protégée **82** par la méthode utilisée précédemment (exemple *méta*-Fluo-EG₃-PMDAM,), l'amine terminale est déprotégée dans des conditions basiques douces qui garantissent la stabilité de l'hydrazone. L'acétoacétate de méthyle est utilisé pour créer la fonction méthylcetone par une réaction d'acylation de l'amine terminale (voir formation des composés **26** et **36).** La formation du diazométhyl est ensuite réalisée par l'une des méthodes décrites précédemment.

### Exemple 25.2 : Synthèse du H₂NO-PMDAM:

### Schéma de synthèse :

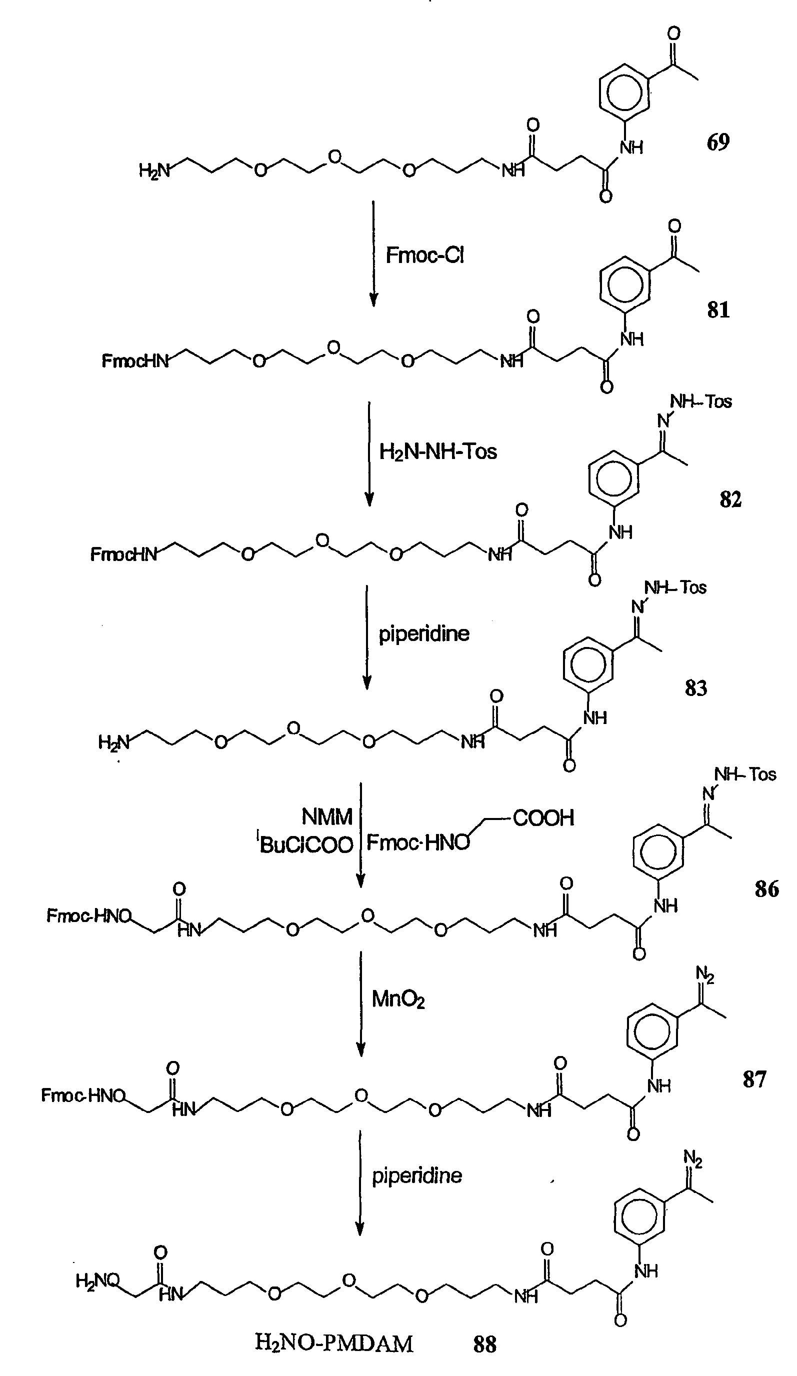

Le produit **88,** dont la synthèse est décrite dans cet exemple, permet de réaliser le marquage des acides nucléiques naturels, grâce a la réactivité de la fonction diazométhyl avec les groupements phosphate, et d'introduire ainsi une fonction alcoxyamine, qui peut être utilisée par la suite pour introduire une molécule détectable (fluorescente, biotine) possédant un groupement méthylcétone.

Cette synthèse est basée sur le modèle de la précédente, c'est-à-dire l'utilisation du précurseur **69,** du Fmoc pour la protection de l'amine et du tosyle pour la protection de l'hydrazone. L'introduction de la fonction alcoxyamine (composé **86**) se fait par l'usage du carboxyméthoxylamine (commercial) protégé par la fonction Fmoc (Thèse E. Trévisiol, LEDSS Grenoble, 1999). Etant donné les conditions douces de la déprotection finale (composé 88), celle ci est réalisée de suite après la formation du diazométhyl.

### Exemple 26 : Préparation de dérivés PDAM permettant l'amplification du signal :

### Exemple 26.1 : Synthèse de marqueurs bis-biotinylés tels que le [Bio-EG3]2-PDAM :

### Schéma de synthèse :

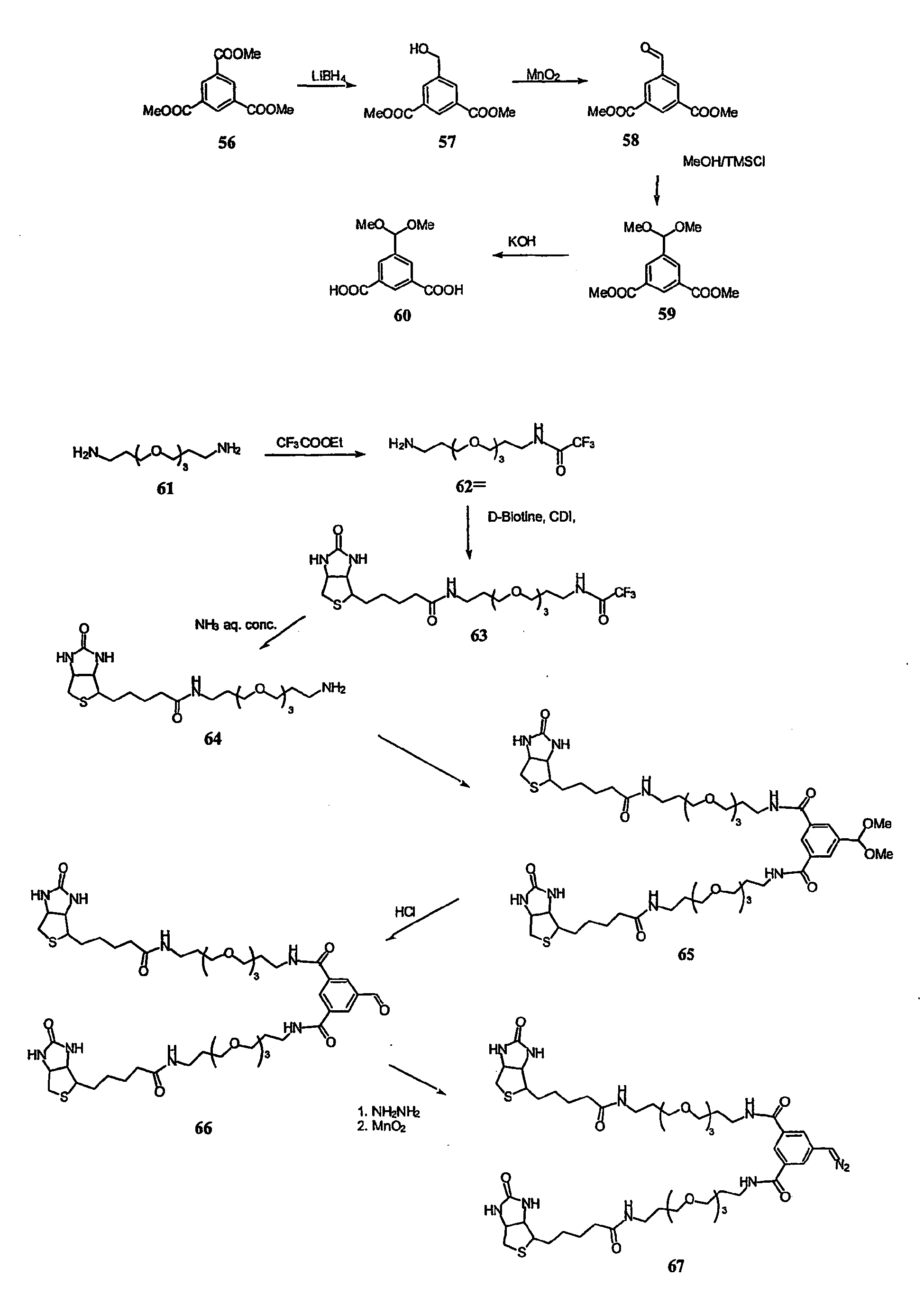

### • Réduction du benzène-1,3,5-tricarboxylate de triméthyle 56 en alcool 57 :

On dissout le triester **56** (12,6 g ; 50,0 mmol) dans 100 mL de THF puis ajoute 1,1 g (50,5 mmol) de LiBH₄ à température ambiante. La solution rouge est chauffée à 40-45°C sous agitation sous argon pendant 1 h. Après refroidissement (glace), on détruit l'hydrure en excès avec précaution (dégagement de H₂) par l'addition d'eau (200 mL) puis de HCl 2N (30 mL). On observe le virage de couleur en jaune claire. On extrait cette solution avec CH₂Cl₂ (100mL puis 3 fois 50 mL), la phase organique est lavée avec NaHCO₃ anhydre, séchée sur MgSO₄ puis le solvant est évaporé jusqu'à l'obtention d'une huile (11,1 g). Par chromatographie flash sur colonne de silice (diamètre = 40 mm, éluant : acétate d'éthyle/cyclohéxane = 1/1), on obtient l'alcool **57** (6,38 g, 57 %).

RMN ¹H (200 MHz, CDCl₃) : δ = 8,53(t, 1H, *J=* 2 Hz) ; 8,18 (d, 2H, *J=* 2 Hz) ; 4,76 (s, 2H); 3,91 (s, 6H) ; 2,30 (s, 1H).

### • Oxydation de l'alcool 57 en aldéhyde 58 :

On dissout l'alcool **57** (5,86 g ; 26,1 mmol) dans 100 mL de THF puis ajoute 40,0 g de MnO₂ petit à petit en 5 min à température ambiante. La solution est laissée une nuit sous agitation sous argon. On filtre la solution à travers un entonnoir Büchner muni d'une couche de Célite 545, on lave avec CH₂Cl₂ puis évapore les solvants. Le solide brut (4,4 g) est purifié par chromatographie flash sur une colonne de silice (diamètre = 50 mm, éluant : acétate d'éthyle/cyclohéxane 3/7). On obtient 3,44 g (59 %) de l'aldéhyde **58.**

RMN ¹H (200 MHz, CDCl₃) : δ = 10,11 (s, 1H) ; 8,89(t, 1H, *J* = 1 Hz) ; 8,69 (d, 2H, J= 1 Hz) ; 3,98 (s, 6H).

### • Formation de l'acétal 59 :

On dissout l'aldéhyde **58** (3,21 g ; 14,4 mmol) dans 30 mL de méthanol puis ajoute 6,0 mL de TMSCI. La solution est laissée à température ambiante sous agitation sous argon pendant 1 h.

On dilue la solution avec 200 mL de CH₂Cl₂, et agite avec 1 M NaHCO₃ (100 mL) (attention dégagement de CO₂). On sépare les deux phases, extrait la phase aqueuse trois fois avec CH₂Cl₂ (25 mL), on rassemble les phases organiques, on sèche sur MgSO₄ puis évapore le solvant. On obtient 3,55 g (92 %) de l'acétal **59.**
RMN ¹H ( 200 MHz, CDCl₃) : δ = 8,63(t, 1H, *J* = 2 Hz) ; 8,29 (d, 2H, *J* = 2 Hz) ; 5,45 (s, 2H) ; 3,93 (s, 6H) ; 3,32 (s, 6H).

### • Hydrolyse du diester 59 en diacide 60 :

On dissout le diester **59** (3,18 g ; 11,9 mmol) dans 10 mL de THF puis ajoute une solution de KOH (2,0 g, pastille 85 %) dans 10 mL de méthanol. Après 15 min à température ambiante, on évapore les solvants. Le résidu est dissous dans H₂O (50 mL). On ajoute H₃PO₄ (environ 2,5 mL, 85 %) jusqu'à pH 3 et le précipité blanc est filtré sur le fritté (#3), lavé avec de l'eau et séché sous vide. On obtient 2, 59 g (91 %) du diacide **60.**

RMN ¹H ( 200 MHz, DMSO-*d*₆) : δ = 8,43 (t, 1H, *J =* 1 Hz) ; 8,15 (d, 2H, *J* = 1 Hz) ; 5,53 (s, 1H) ; 3,27 (s, 6H).

### • Trifluoracétamide 62 :

On dissout la diamine **61** (66 g ; 0,30 mol) dans 250 mL de CH₂Cl₂ puis ajoute le trifluoracétate d'éthyle (11,8 mL, 0,10 mol) goutte à goutte en 5 min à 10 °C sous agitation sous argon. Après 15 min à température ambiante, on transfère la solution dans une ampoule à décanter, on lave avec H₂O (3 × 100 mL), on sèche sur MgSO₄ et évapore le solvant. On obtient 22,4 g (71 %) du monoamide **62** de pureté d'environ 85 % (déterminée par F¹⁹ RMN). Ce composé est conservé à -20 °C et utilisé sans purification.

RMN ¹H ( 200 MHz, CDCl₃) : δ = 3,5-3,6 (m, 12H) ; 3,42 (t, 2H, *J*= 6 Hz) ; 2,75 (t, 2H, *J*= 6 Hz) ; 1,81 (quantiplet, 2H, *J*= 6 Hz) ; 1,67 (quantiplet, 2H, *J*= 6 Hz) ; 1,30 (s. large, 2H).
RMN ¹⁹F ( 190 MHz, CDCl₃) : δ = -76,3.

### • Composé 63 :

A une suspension de D-biotine (6,39 g ; 26,2 mmol) dans 50 mL de DMF, on ajoute le carbonyldiimidazole (CDI, 6,32 g , 80 %, 31,2 mmol). Le mélange est chauffé à 55-60 °C sous agitation sous argon pendant 30 min. On observe initialement une dissolution complète de matériel puis une prise en masse avec une précipitation de solide blanc (CO₂ évolution). On ajoute l'amine (huile) à l'aide de 5 mL de CH₂Cl₂ pour rincer et le mélange est chauffé à 55-60 °C pendant 3 h. On évapore le DMF sous vide (< 1 mmHg) et le résidu est agité avec CH₂Cl₂ (700 mL) et 2N HCl (100 mL). Après une filtration des deux phases à travers une couche de Célite 545, on sépare les phases, la phase aqueuse est extraite avec CH₂Cl₂ **(15 ×** 100 mL), les phases organiques sont rassemblées, séchées sur NaHCO₃ anhydre et MgSO₄, puis le solvant est évaporé. Le résidu huileux est trituré avec 150 mL d'éther pour obtenir une suspension. Le solide pâteux est difficile à filtrer. On décante le surnageant et on répète le lavage à l'éther. Après séchage sous vide, on obtient 9,13 g (64 %) de composé **63.**
RMN ¹H ( 200 MHz, CDCl₃) : δ = 3,5-3,6 (m, 12H); 3,42 (t, 2H, *J=* 6 Hz) ; 2,75 (t, 2H, *J=* 6 Hz) ; 1,81 (quantiplet, 2H, *J=* 6 Hz); 1,67 (quantiplet, 2H, *J=* 6 Hz) ; 1,30 (s. large, 2H).

### • Composé 64 :

On chauffe une solution du composé **63** dans l'ammoniaque (100 mL, 22 % aqueuse) dans un ballon de 250 mL bouché avec un septum à 55-60 °C pendant 2 h. Après refroidissement, on évapore le solvant. Le résidu est dissous dans le méthanol (20 mL) et passé sur une colonne résine échangeuse d'anion Dowex 21K [hauteur 12 cm × diamètre 35 mm, forme OH⁻ obtenu par lavage préalable avec NaOH 1N (1,5 L) puis H₂O (1,5 L) puis méthanol (1,5 L)]. Le composé **64** libre de l'ion trifluoracétate passe dans les premières fractions avec 200 mL du méthanol. Après évaporation, on triture le résidu avec 50 mL d'éther puis on le décante. Le lavage à l'éther est répété cinq fois de suite. Après séchage, on obtient le composé **64** (6,43 g, 86 %).

RMN ¹H ( 300 MHz, CDCl₃) : δ = 6,77 (t, 1H, *J* = 4 Hz) ; 6,32 (s, 1H) ; 5,52 (s, 1H) : 4,45 (m, 1H) ; 4,28 (m, 1H), 3,50-3,68 (m, 12H) ; 3,30 (m, 2H), 3,11 (m, 1H) ; 2,86 (dd, 1H, *J*= 13 et 5 Hz), 2,75 (t, 2H, *J*= 13 Hz), 2,68 (d, 1H, *J=* 13 Hz) ; 2,16 (t, 2H, *J*= 7 Hz) ; 1,60-1,85 (m, 8H) ; 1,41 (m, 2H).

### • [Bio-EG₃]₂- acétal 65 :

A une suspension du diacide **60** (120 mg ; 0,500 mmol) dans le dichloroéthane (5 mL), on ajoute le carbonyldiimidazole (225 mg, 90 %, 1,25 mmol) et on chauffe à 55-60 °C pendant 30 min sous agitation sous argon. On ajoute l'amine **64** (550mg ; 1,23 mmol) et la solution est chauffée à 55-60 °C pendant 6 h. Après évaporation, on passe sur une colonne de silice (diamètre : 25 mm, éluant : méthanol 15-30 % dans CH₂Cl₂). On obtient 413 mg (75 %) de composé **65.**

RMN ¹H ( 300 MHz, CDCl₃) : δ = 8,34 (s, 1H) ; 8,06 (s, 2H) ; 7,87 (m, 2H) ; 6,85 (m, 2H) ; 6,60 (s, 2H) ; 5,93 (s, 2H) : 5,40 (s, 1H) ; 4,45 (m, 2H) ; 4,27 (m, 2H), 3,43-3,68 (m, 24H) ; 3,31 (s, 6H) ; 3,25 (m, 4H), 3,08 (m, 2H) ; 2,83 (dd, 2H, *J=* 13 et 5 Hz), 2,70 (t, 2H, *J* = 13 Hz) ; 2,13 (t, 4H, *J=* 7 Hz) ; 1,89 (quintuplet, 4H, *J=* 7 Hz) ; 1,55-1,70 (m, 12H) ; 1,37 (m, 4H).

### • [Bio-EG₃]₂- aldéhyde 66 :

L'acétal **65** (413 mg ; 0,376 mmol) en solution dans le méthanol est traité avec HCl 2N (0,5 mL). Après évaporation et lavage avec l'éther, on obtient l'aldéhyde **66** (0,37 g, 90 %).
RMN ¹H ( 300 MHz, DMSO-*d*₆) : δ = 10,11 (s,1H) ; 8,82 (t, 2H, *J =* 6 Hz) ; 8,62 (s, 1H) ; 8,47 (s, 2H) ; 7,73 (t, 2H, *J* = 5 Hz) ; 4,30 (m, 2H) ; 4,11 (m, 2H), 3,30-3,60 (m, 24H) ; 3,06 (m, 6H) ; 2,80 (dd, 2H, *J* = 12 et 5 Hz), 2,56 (t, 2H, *J* = 12 Hz) ; 2,03 (t, 4H, *J=* 7 Hz) ; 1,78 (quintuplet, 4H, *J=* 7 Hz) ; 1,35-1,60 (m, 12H) ; 1,28 (m, 4H).

### • [Bio-EG₃]₂-PDAM 67 :

L'aldéhyde 66 est transformé en diazométhane **67** selon la méthode utilisée pour la préparation des diazométhanes (Exemple 1).
La stabilité du réactif est supérieure à 1 mois à -20°C.

### Exemple 26.2 : Synthèse du méta-Bio7-EG3-PMDAM :

### Schéma de synthèse :

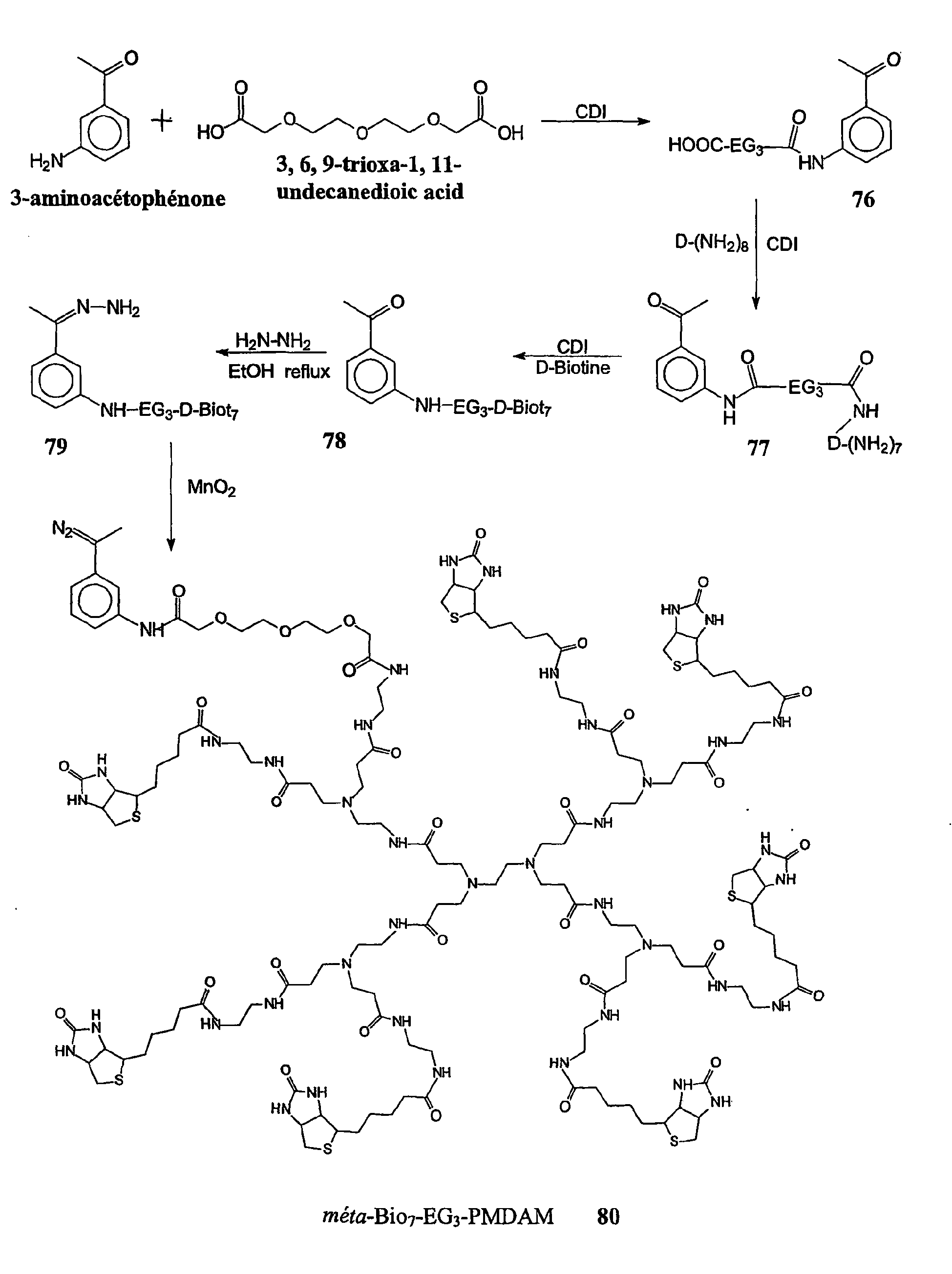

### Composé EG₃-acétophénone 76 :

Le 3, 6, 9-trioxa-1, 11-undecanedioic acid (EG₃, 12,64 ml, 74 mmol) est dissout dans 80 ml de DMF anhydre sous argon et refroidit dans un bain de glace. Le dicyclohexylcarbodiimide (DCC, 11,45 g, 55,5 mmol)) est ensuite dissout dans 20 ml de DMF anhydre et ajouté lentement. Après 30 min, on ajoute le 3-aminoacétophénone (5,0 g, 37 mmol) et on laisse réagir 1 h à température ambiante sous argon. Le DMF est ensuite évaporé sous vide et 70 ml de CH₂CL₂ sont ajoutés. La solution est filtrée et extraite avec 3x25 ml d'acide acétique 1%. Les phases aqueuses sont réunies et lavées avec 25 ml de CH₂CL₂. Les phases organiques sont mélangées, séchées avec du sulfate de sodium anhydre et évaporées à sec. Le produit est recristallisé dans le couple MeOH:H₂O. On obtient ainsi 8,74 g (70%) du produit **76.**

RMN ¹H (200 MHz, DMSO-d₆): d = 2,55 (s, 3H); 3,5-3,7 (m, 8H); 4,0 (s, 2H); 4,1 (s, 2H); 7,45 (t, 1H); 7,65 (d, 1H); 7,90 (d, 2H); 8,2 (s, 1H); 9,8 (s, 1H).

### Composé (NH₂)₇-EG₃-acétophénone 77 :

Le produit **76** (120mg, 0,35 mmol) est dissout dans 15 ml de DMF anhydre sous argon, refroidit sur glace, et le DCC (110 mg, 0,53 mmol) est ensuite ajouté. Après 30 min, on ajoute cette solution sur une solution du dendrimère commercial « Starburst PAMAM Dendrimer, Generation 1 » (Aldrich, St Quentin Fallavier) (1 g, 0,71 mmol, dans 5 ml de méthanol), lentement et sous forte agitation. On laisse réagir 1 h à température ambiante et on évapore. Le résidu est repris dans 10 ml de CH₂CL₂ et extrait deux fois avec 30 ml d'acide acétique 1%.

### Composé Biot₇-EG₃- acétophenone 78 :

La D-Biotine (1,73 g, 7,08 mmol) est solubilisée dans 80 ml de DMF anhydre sous argon, et la solution est refroidie sur glace. On ajoute successivement la N-méthylmorpholine (NMM, 856 µl, 7,7 mmol) et le chloroformiate d'isobutyle (1022 µl, 7,7 mmol). Après 30 min, on ajoute le produit **77** (1,13 g, 0,7 mmol, dans 5 ml de méthanol) et on laisse réagir 3 h sur glace et sous argon. On concentre sous vide jusqu'à 50 ml et on ajoute 100 ml de CH₂Cl₂ . Un précipité se forme, qui est filtré, lavé à l'éther, et séché sous vide. On obtient 1,3 g de **78** sous forme d'une poudre blanche.

### Composé Biot₇-EG₃-Hydrazone 79 :

Le composé **78** (300 mg, 0,09 mmol) est dissout dans 10 ml d'éthanol absolu à reflux. On ajoute l'hydrazine monohydrate (20 ml, 0,40 mmol) et on laisse réagir 3 h à reflux. Après refroidissement, un précipité se forme, qui est filtré, lavé a l'éther et séché sous vide. On obtient ainsi 109 mg (36%) du produit **79,** sous forme d'une poudre blanche.

### Composé Biot₇-EG₃-PMDAM 80 :

On solubilise l'hydrazone **79** (100 mg, 0,03 mmol) dans 5 ml de DMF anhydre à 70°C. On laisse revenir à température ambiante et on ajoute le MnO₂ (31 mg, 0, 36 mmol). On laisse réagir 10 min et on élimine l'oxyde de manganèse par filtration sur un fritté avec de la célite (0,5 cm et du tamis moléculaire en poudre (0,5 cm). Le filtrat est évaporé a sec, lavé a l'éther e séché sous vide. On obtient ainsi 78 mg (78%) du produit **80.**

Les dendrimères sont des molécules arborescentes possédant aux extrémités plusieurs groupements réactifs tels que des amines, carboxyles, hydroxyles ou autres (pour revue, voir Newcome et al, (1996) *Dendritic Molecules: Concept, Syntheses, Perspectives.* VCH Ed., Weinheim, Germany). La synthèse de ces molécules est aujourd'hui bien maîtrisée, et des nombreux dendrimères sont commercialisés par l'industrie chimique. Le choix du PAMAM (Sigma-Aldrich) a été fait sur la base de sa stabilité, solubilité et souplesse, puisque plusieurs versions de cette molécule, avec un nombre et type de terminaisons différentes, sont disponibles. Le "PAMAM Generation 1" permet d'ajouter sept molécules du marqueur (en une seule étape de synthèse) pour chaque groupement diazométhyl.

### Exemple 27 : Marquage et fragmentation en deux étapes d'amplicons ADN avec le méta-BioPMDAM :

Les amplicons ADN ont été préparés par amplification PCR selon le protocole décrit dans l'exemple 5. Deux réactions de marquage ont été réalisées.

### a. Marquage et fragmentation en deux étapes :

A 10 µL de PCR sont ajoutés 10 µL de *méta*-BioPMDAM (100 mM dans du DMSO) et 77 µL d'eau Dnase/Rnase free. La solution est incubée 10 min à 95°C. Puis 3 µL d'HCl à 0,1M sont ajoutés et la solution est incubée 10 min à 95°C.

### b. Marquage et fragmentation en une étape :

A 10 µL de PCR sont ajoutés 10 µL de *méta*-BioPMDAM (100 mM dans du DMSO), 5 µL d'HCl à 0,1 M et 75 µL d'eau Dnase/Rnase free. La solution est incubée 30 min à 60°C. Le reste du protocole est identique à celui de l'exemple 9.

### Résultats :

**Tableau 13 : Etude comparative du marquage et de la fragmentation en deux étapes distinctes et en une seule étape**

| **Protocole utilisé** | **Homologie (%)** | **I (rfu)** | **B (rfu)** | **I/B** |
|---|---|---|---|---|
| a. Marquage et fragmentation en deux étapes | 99,5 | 14129 | 624 | 22,7 |
| b. Marquage et fragmentation en une étape | 98,9 | 4431 | 667 | 6,6 |

Comme le démontre le tableau 13, les résultats obtenus avec le protocole en une étape sont satisfaisants. Ceux obtenus avec un marquage et une fragmentation en deux étapes sont encore meilleurs. Cet exemple montre que les étapes de marquage et clivage peuvent être dissociées pour améliorer le marquage en fonction de la cible utilisée.

### Exemple 28 : Marquage et fragmentation d'amplicons ADN dans différents formats de réaction :

Les amplicons d'ADN ont été préparés par amplification PCR selon le protocole décrit dans l'exemple 5. Trois réactions de marquage ont été réalisées.

### a. Marquage et fragmentation dans un format de 250 µL :

A 50 µL de PCR sont ajoutés 75 µL de *méta*-BioPMDAM (100 mM dans du DMSO) et 102,5 µL d'eau Dnase/Rnase free. La solution est incubée 25 min à 95°C. Puis 22,5 µL d'HCl à 0,1 M sont ajoutés et la solution est incubée 5 min à 95°C.

### b. Marquage et fragmentation dans un format de 200 µL :

A 50 µL de PCR sont ajoutés 75 µL de *méta*-BioPMDAM (100 mM dans du DMSO) et 52,5 µL d'eau Dnase/Rnase free. La solution est incubée 25 min à 95°C. Puis 22,5 µL d'HCl à 0,1 M sont ajoutés et la solution est incubée 5 min à 95°C.

### c. Marquage et fragmentation dans un format de 150 µL :

A 50 µL de PCR sont ajoutés 75 µL de *méta*-BioPMDAM (100 mM dans du DMSO) et 2,5 µL d'eau Dnase/Rnase free. La solution est incubée 25 min à 95°C.
Puis 22,5 µL d'HCl 0,1M sont ajoutés et la solution est incubée 5 min à 95°C.

Le reste du protocole est identique à celui de l'exemple 9.

### Résultats :

**Tableau 14 : Marquage et fragmentation selon différents formats**

| **Protocole utilisé** | **Homologie (%)** | **I (rfu)** | **B (rfu)** | **I/B** |
|---|---|---|---|---|
| *a. Format de 250 µL* | 100,0 | 5606 | 549 | 10,2 |
| *b. Format de 200 µL* | 99,4 | 5886 | 557 | 10,6 |
| *c. Format de 150 µL* | 99,4 | 6800 | 537 | 12,7 |

Les résultats obtenus en terme de signal et de pourcentage d'homologie sont très satisfaisants dans tous les cas de figures. De plus, bien que le format de la réaction varie de 150 à 250 µL, les résultats ont des valeurs similaires.
Cet exemple montre une flexibilité du format réactionnel du protocole de marquage pouvant accepter différents volumes et notamment différents volumes de produits d'amplification.

### Exemple 29 : Comparaison entre un protocole utilisant une étape de purification avant fragmentation et un protocole utilisant une étape de purification après fragmentation :

Les amplicons d'ADN ont été préparés par amplification PCR selon le protocole décrit dans l'exemple 5. Deux réactions de marquage ont été réalisées.

### a. Marquage purification puis fragmentation des amplicons ADN :

A 10 µL de PCR sont ajoutés 10 µL de *méta*-BioPMDAM (100 mM dans du DMSO) et 80 µL d'eau Dnase/Rnase free. La solution est incubée 10 min à 95°C. Puis la purification est réalisée selon le protocole décrit dans l'exemple 9. A la solution d'amplicons marqués purifiés 6 µL d'HCl à 0,1 M sont ajoutés. La solution est incubée 10 min à 95°C. Quatre cents (400) µL de tampon d'hybridation préchauffé à 95°C durant 10 min sont ajoutés.
La composition du tampon d'hybridation ainsi que le reste du protocole sont identiques à celui de l'exemple 9.

### b. Marquage fragmentation puis purification des amplicons ADN :

A 10 µL de PCR sont ajoutés 10 µL de *méta*-BioPMDAM (100 mM dans du DMSO) et 77 µL d'eau Dnase/Rnase free. La solution est incubée 10 min à 95°C.
Puis 3 µL d'HCl à 0,1 M sont ajoutés et la solution est incubée de nouveau 10 min à 95°C. Le reste du protocole est identique à celui de l'exemple 9.

### Résultats :

**Tableau 15 : Comparaison entre protocole utilisant une étape de purification avant fragmentation et protocole utilisant une étape de purification après fragmentation**

| **Protocole utilisé** | **Homologie (%)** | **I (rfu)** | **B (rfu)** | **I/B** |
|---|---|---|---|---|
| a. Purification avant fragmentation | 98,9 | 6256 | 473 | 13 |
| b. Fragmentation avant purification | 96,1 | 6066 | 556 | 11 |

Ce résultat, présenté dans le tableau 15, montre que l'étape de purification peut être introduite entre les étapes de marquage et de fragmentation. De plus l'introduction de la purification entre les étapes de marquage et de fragmentation permet de réaliser la dénaturation pendant le clivage et d'hybrider sur la puce la totalité des fragments d'amplicons marqués.

### Exemple 30 : Synthèse du 2-nitro-para-BioPDAM :

### Schéma de synthèse :

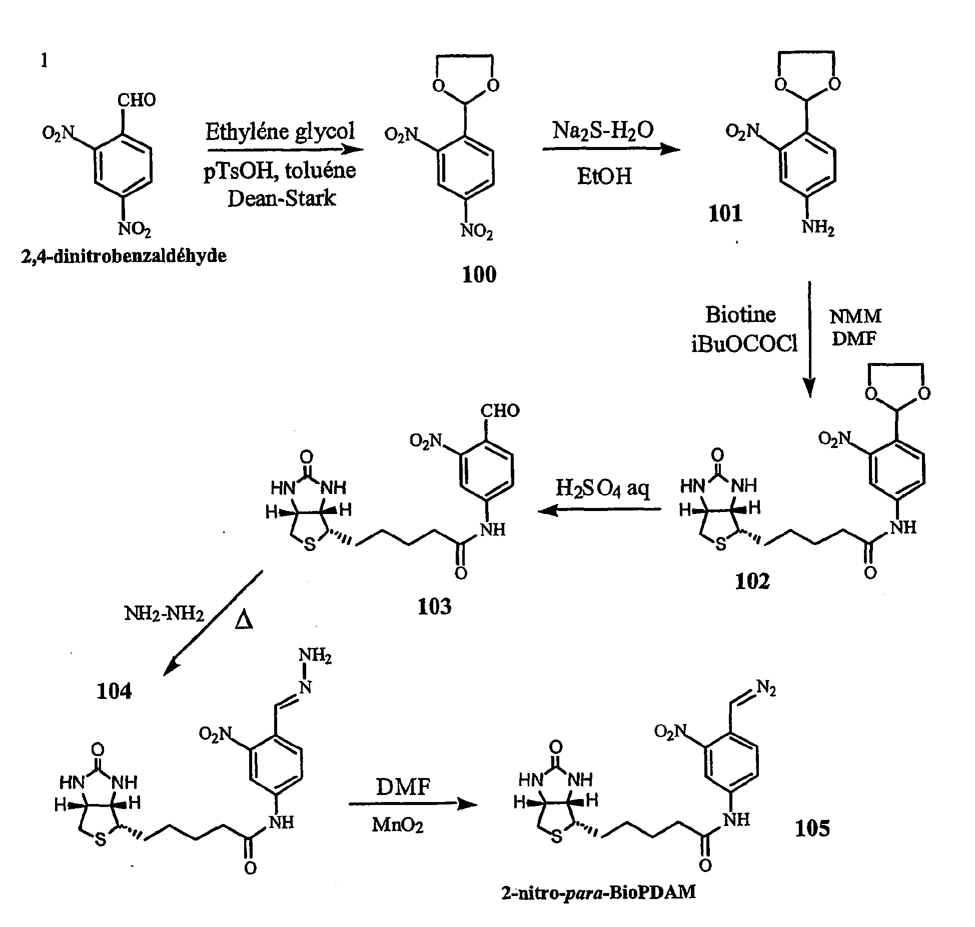

### • Protection de l'aldéhyde :

On dissout 5 g (25,5 mmol) de 2,4-dinitrobenzaldéhyde dans 250 mL de toluène, et l'on ajoute 20 mL d'éthylène glycol et 150 mg d'acide *para*-toluènesulfonique. On chauffe à reflux en récupérant l'eau dans un système Dean-Stark pendant 6 h. On traite avec 150 mL d'EtOAc et 100 mL d'H₂O. On extrait deux fois la solution à l'acétate d'éthyle, on sèche la phase organique avec MgSO₄ puis on l'évapore. L'huile obtenue correspondant au produit 100, est utilisée pour la réaction suivante.

RMN ¹H (200 MHz, CDCl₃) : δ = 8,70 (d, 1Hₐᵣₒ, *J* = 2 Hz, H₃); 8,44 (dd, 1Hₐᵣₒ, *J* = 2 Hz, *J=* 6 Hz, H₅); 8,02 (d, 1Hₐᵣₒ, *J=* 8 Hz, H₆); 6,49 (s, 1H, CH) ; 4,12-4,06 (m, 4H, CH₂-CH₂).

### • Réduction du dérivé dinitro 100 :

Le 2-4-dinitrobenzaldéhyde protégé (6,4 g ; 25,5 mmol) est dissous dans un mélange éthanol-eau (6/1), puis 2 équivalents de Na₂S nonahydraté (12,3 g ; 51,1 mmol) sont ajoutés. Le mélange réactionnel est ensuite chauffé pendant 30 min. Une évaporation puis une extraction au dichlorométhane sont réalisées. Après séchage et filtration, le milieu réactionnel est évaporé afin d'obtenir une huile, qui est directement purifiée sur colonne de silice (cyclohexane-acétate d'éthyle 60/40). Le composé **101** est isolé avec un rendement de 45 %.

Composé **101** : F 58-60°C. - RMN ¹H (200 MHz, CDCl₃) : 7,49 (d, 1Hₐᵣₒ, *J*= 2 Hz, H₃) ; 7,09 (d, 1Hₐᵣₒ, *J*= 2 Hz, H₆) ; 6,80 (dd, 1Hₐᵣₒ, *J*= 2 Hz, *J*= 6 Hz, H₅) ; 6,27 (s, 1H, CH) ; 3,99-3,97 (m, 4H, CH₂-CH₂).

### • Couplage avec la biotine :

On solubilise la D-biotine (1,0 g ; 4,1 mmol) dans 20 mL de DMF anhydre et 600 µL de N-méthylmorpholine. On ajoute sous argon le chloroformiate d'isobutyle (700 µL ; 5,5 mmol) en refroidissant dans un bain glacé. On laisse sous agitation pendant 5 min, puis on ajoute 1 g (4,75 mmol) du composé **101** et 500 µL de N-méthylmorpholine. La solution est maintenue sous agitation à température ambiante pendant 4 h, puis évaporée à sec. L'huile obtenue est passée directement sur colonne de silice avec comme solvant d'élution MeOH-DCM 7 % puis 10 %. Le produit **102** (1,1 g ; 2,52 mmol) est obtenu avec un rendement de 62 %.
RMN ¹H (200MHz, DMSO-d₆) δ =10,40 (s, 1H, NH-CO) ; 8,31 (d, 1Hₐᵣₒ, *J*= 2 Hz, H₃) ; 7,77 (dd, 1Hₐᵣₒ, *J*= 2 Hz, *J*= 6 Hz, H₅) ; 7,68 (d, 1Hₐᵣₒ, *J*= 2 Hz, H₆) ; 6,43 (s large, 1H, NH-CO-NH) ; 6,36 (s large, 1H, NH-CO-NH) ; 6,23 (s, 1H, CH) ; 4,28 (m, 1H, CH₂-CH-NH) ; 4,14 (m, 1H, CH-CH-NH) ; 3,92 (s, 4H, CH₂-CH₂); 3,12 (m, 1H, CH-S) ; 2,85 et 2,76 (système ABX, 2H, ²*J*_{AB}= 5 Hz, ³*J*_{AX} = 12 Hz, ³*J*_{BX} = 0 Hz, CH₂-S) ; 2,29 (t, 2H, *J* = 8 Hz, CH₂-CO) ; 1,61-1,39 (m, 6H, (CH₂)₃).

### • Déprotection de l'acétal :

On met le produit **102** (768 mg ; 1,76 mmol) en suspension dans 25 mL de THF. Tout se dissout après ajout de 4 mL de H₂SO₄ 2N. On laisse sous agitation pendant 2 h. On évapore puis on rince et lave à l'eau sur fritté. Le composé **103** (694 mg) est obtenu sous forme d'une poudre jaune avec un rendement de 90 %.

F 165°C. - RMN ¹H (200MHz, DMSO-d₆) δ =10,69 (s, 1H, NH-CO) ;10,09 (s, H, CHO) ; 8,43 (d, 1Hₐᵣₒ, J= 2 Hz, H₃) ; 7,91 (s, 2Hₐᵣₒ, H₅ et H₆) ; 6,42 (s large, 1H, NH-CO-NH) ; 6,35 (s large, 1H, NH-CO-NH) ; δ = 6,23 (s, 1H, CH) ; 4,29 (m, 1H, CH₂-CH-NH) ; 4,13 (m, 1H, CH-CH-NH) ; 3,12 (m, 1H, CH-S) ; 2,84 et 2,78 (système ABX, 2H, ²*J*_{AB}= 5 Hz, ³*J*_{AX} = 12 Hz, ³*J*_{BX} = 0 Hz, CH₂-S) ; 2,29 (t, 2H, *J* = 8 Hz, CH₂-CO) ; 1,61-1,39 (m, 6H, (CH₂)₃).

### • Formation de l'hydrazone 104 :

On met l'aldéhyde **103** en suspension dans l'éthanol et l'on chauffe à 80°C. Quand on ajoute l'hydrazine, tout se dissout et la solution se colore immédiatement en orange. Un précipité se forme après 5 min. On chauffe sous agitation pendant 1 h. On filtre sur fritté puis on sèche le précipité. Le produit **104** (700 mg ; 690 mmol) est obtenu avec un rendement de 98 %.

F 169°C. - RMN ¹H (200MHz, DMSO-d₆) δ =10,31 (s, 1H, NH-CO) ; 8,31 (d, 1H_{aro,} *J* = 2 Hz, H₃); 7,96 (s, H, CHO) ; 7,87 (d, 1Hₐᵣₒ, *J= 2* Hz, H₆); 7,68 (dd, 1Hₐᵣₒ, *J =* 2 Hz, *J*= 6 Hz, H₅) ; 7,31 (s, 2H, NH₂); 6,42 (s large, 1H, NH-CO-NH) ; 6,34 (s large, 1H, NH-CO-NH); 4,29 (m, 1H, CH₂-CH-NH); 4,13 (m, 1H, CH-CH-NH); 3,12 (m, 1H, CH-S) ; 2,84 et 2,78 (système ABX, 2H, ²*J*_{AB}= 5 Hz, ³*J*_{AX} = 12 Hz, ³*J*_{BX} = 0 Hz, CH₂-S) ; 2,29 (t, 2H, *J =* 8 Hz, CH₂-CO); 1,61-1,39 (m, 6H, (CH₂)₃).

### • Formation du diazo 105 :

On dissout le composé **104** (200 mg ; 0,492 mmol) dans 8 mL de DMF. On ajoute 400 mg de MnO₂. On agite vigoureusement pendant 10 min. On filtre sur millipore contenant de la célite (épaisseur : 2 cm) et du tamis moléculaire en poudre 3 Å (0,5 cm). On évapore à sec puis on lave à l'éther. On filtre de nouveau sur millipore. Le composé **105** (180 mg ; 0,445 mmol) est obtenu sous forme d'une poudre orange avec un rendement de 98 %.

F 155°C. - RMN ¹H (200MHz, DMSO-d₆) δ =10,21 (s, 1H, NH-CO) ; 8,60 (d, 1Hₐᵣₒ, *J* = 2 Hz, H₃); 7,77 (d, 1Hₐᵣₒ, *J* = 6 Hz, H₅); 7,22 (d, 1Hₐᵣₒ, *J* = 6 Hz, H₆); 6,60 (s, H, CH-N); 6,41 (s large, 1H, NH-CO-NH); 6,33 (s large, 1H, NH-CO-NH); 4,29 (m, 1H, CH₂-CH-NH); 4,13 (m, 1H, CH-CH-NH); 3,12 (m, 1H, CH-S) ; 2,84 et 2,78 (système ABX, 2H, ²*J*_{AB}= 5 Hz, ³*J*_{AX} = 12 Hz, ³*J*_{BX} = 0 Hz, CH₂-S); 2,29 (t, 2H, *J* = 8 Hz, CH₂-CO); 1,61-1,39 (m, 6H, (CH₂)₃).

La réactivité du composé **105** a été testée sur 3'-Uridine-monophosphate suivie par électrophorèse capillaire. Les conditions d'analyse sont celles de l'exemple 6.1. Les résultats montrent un temps de demi réaction de 45 minutes. La stabilité du réactif est supérieure à 1 mois à -20°C

### Exemple 31: Marquage et fragmentation des amplicons d'ADN avec le réactif de marquage 2-nitro-para-BioPDAM:

Le dérivé 2-nitro-*para*-BioPDAM a été obtenu selon le schéma réactionnel décrit dans l'exemple 30. Les amplicons ADN ont été préparés par amplification PCR selon le protocole décrit dans l'exemple 5. Deux réactions de marquage ont été réalisées.

### a. Marquage par le réactif 2-nitro-para-BioPDAM :

A 10 µL de PCR sont ajoutés 2 µL de 2-nitro-*para*-BioPDAM (100 mM dans du DMSO), 5µL d'HCl à 0,1 M et 83 µL d'eau Dnase/Rnase free. Cette solution est incubée 30 min à 60°C.

### b. Marquage par le réactif méta-bioPMDAM :

A 10 µL de PCR sont ajoutés 2 µL de *méta*-BioPMDAM (100 mM dans du DMSO), 5µL d'HCl à 0,1 M et 83 µL d'eau Dnase/Rnase free. Cette solution est incubée 30 min à 60°C.

Le reste du protocole est identique à celui de l'exemple 9.

### Résultat :

**Tableau 16 : Etude comparative du marquage d'ADN par le dérivé 2-nitro-para-BioPDAM vis-à-vis du dérivé méta-BioPMDAM**

| **Protocole utilisé** | **Homologie (%)** | **I (rfu)** | **B (rfu)** | **I/B** |
|---|---|---|---|---|
| *a. Marquage par le réactif* 2-nitro-*para*-BioPDAM | 100,0 | 24392 | 899 | 27,1 |
| *b. Marquage par le réactif méta-bioPMDAM* | 98,9 | 21883 | 774 | 28,3 |

Le réactif 2-nitro-*para*-BioPDAM utilisé pour le marquage de l'ADN donne des résultats intéressants en terme d'intensité de marquage et de pourcentage d'homologie.

### Exemple 32 : Insertion d'une double liaison entre la fonction diazométhyle et le noyau phenyle, éloignement du DAM et synthèse d'une molécule particulièrement adaptée à cet éloignement :

L'objectif visé est l'éloignement de la fonction diazométhyle (DAM) de la structure aromatique pour minimiser l'effet de l'encombrement stérique lors de l'alkyaltion des phosphates et aussi lors de l'hybridation de l'acide nucléique marqué avec sa séquence complémentaire.

### Schéma de synthèse :

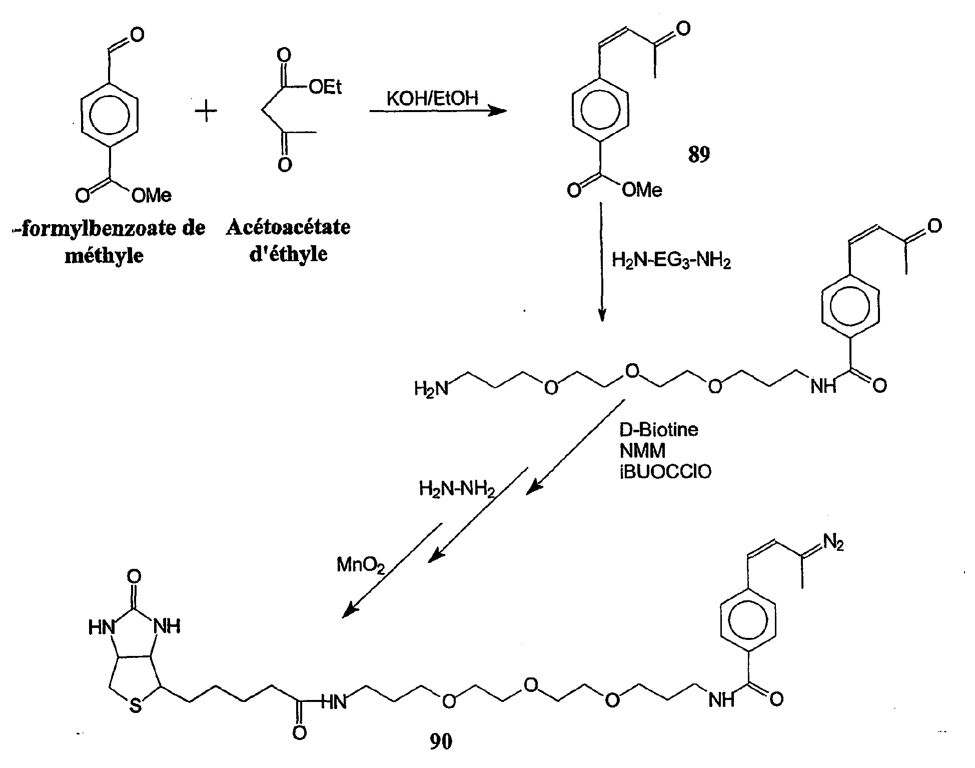

Pour la réaction aldolique de formation du (*para*-méthoxycarbonyl)-styrylméthylcétone **89,** on utilise l'acétoacétate d'éthyle pour la forte acidité des protons du méthylène, ce qui facilite l'attaque du groupement formyl, avec élimination postérieure de H₂O (favorisée par la conjugaison de la double liaison avec l'anneau aromatique) et décarboxylation par hydrolyse due au milieu basique. Le reste de la synthèse est similaire a ce qui a été montré dans les autres exemples.
Le produit final *para*-Bio-EG₃-SMDAM **90,** possède deux carbones de plus entre le diazométhyle et l'anneau aromatique, ce qui limite les possibles problèmes stériques, tout en conservant la stabilisation du diazométhyle par le système aromatique par conjugaison.

### Exemple 33 : Capture et détection d'un acide nucléique sur un support solide portant des groupements diazométhyles :

La réactivité d'une résine portant des groupements diazométhyles a été étudiée pour déterminer sa capacité à lier des acides nucléiques.

La 4-(Diazomethyl)phenoxymethyl-polystyrene (référence 17338, Fluka) est une résine décrite pour son pouvoir à lier les groupements carboxyliques, en particuliers ceux présents dans les protéines (G. Bhalay, A.R. Dunstan, Tetrahedron Lett. *39*, 7803 - 1998), mais elle n'est pas décrite pour sa capacité à lier les molécules d'ADN. Nous avons testé la possibilité de capturer des acides nucléiques avec ce réactif, et de les révéler par un test colorimétrique.

L'expérience est réalisée avec une partie des réactifs présents dans le kit HLA-DR oligo-détection (référence 33 202, bioMérieux, France principe de base décrit dans le brevet EP 549 776-B1), permettant la détection d'acides nucléiques amplifiés par PCR dans des microplaques, par lecture colorimétrique. Dans le cadre de l'expérience décrite, on fait réagir simultanément des acides nucléiques produits par PCR avec la résine testée, ainsi qu'avec une molécule de *para*-Bio-EG3-PDAM, dont la synthèse est évoquée dans l'exemple 20. Si l'ADN réagit avec les fonctions diazométhyle présentes sur les deux composés, il sera possible de le révéler, après lavage et élimination des molécules fixées de manière non covalente, grâce à une réaction colorimétrique faisant intervenir une enzyme couplée à la streptavidine. La streptavidine est associée à une péroxydase de raifort, cet enzyme pouvant décomposer une molécule d'OrthoPhénylène Diamine (réactif Color 1 du kit) en un composé détectable à une longueur d'onde de 492 nm.

### Exemple 33.1 : Capture et détection de l'ADN :

On incube 10 mg de résine pendant 30 minutes à 60°C avec 50 µL de PCR réalisées comme décrit dans l'exemple 5, dans 400 µL d'eau pure (Sigma) additionnée de 5 µL de *para*-Bio-EG3-PDAM. On lave ensuite cette résine par 500 µL de tampon PBS tween (Réactif Color 0 HLA du kit, PBS pH 7,0 ; Tween 1 %, BND 0,2 g/L ; Ciproflaxacine 0,01 g/L). On remet en suspension alors la résine dans 100 µL de PBS Tween, et 250 µL de tampon d'hybridation streptavidine (BPS pH 7,0 TWEEN 0,5 %) additionné de streptavidine HRP (S-911, MOLECULAR PROBES, EUGENE, OR, USA) dilué au 1/10000. On incube le mélange réactionnel 30 min à température ambiante. On lave ensuite la résine trois (3) fois par 500 µL de tampon PBS tween, et on l'incube à température ambiante en présence de réactif chromogénique (1 Comprimé Color 1, Chlorhydrate d'orthophénylène diamine, dilué dans 5 mL de tampon Color 2, Phosphate de sodium 100 mM, acide citrique 50 mM, H₂O₂ 0,03% ). Après une incubation de 20 min à l'obscurité, la réaction est ensuite bloquée par 50 µL de H₂SO₄ (1, 8 N réactif Color 3). Le surnageant est alors pipeté et placé dans une microplaque pour lire l'absorbance du milieu réactionnel à 492 nm.

### Exemple 33.2 : Témoin sans acide nucléique :

On incube 10 mg de résine pendant 30 minutes à 60°C dans 425 µL d'eau pure (Sigma) additionnée de 5 µL de *para*-Bio-EG3-PDAM. On lave ensuite cette résine par 500 µL de tampon PBS. On traite alors l'échantillon de façon identique à processus décrit à l'exemple 33.1.

### Exemple 33.3 : Témoin avec PCR réalisée sans cibles :

On incube 10 mg de résine dans 400 µL d'eau pure additionnée de 5 µL de *para*-Bio-EG3-PDAM pendant 30 minutes à 60°C, avec 50 µL de PCR réalisée avec un volume de 25 µL d'eau pure à la place du volume l'ADN génomique décrit. On lave ensuite cette résine par 500 µL de tampon PBS. On traite alors l'échantillon de façon identique au processus décrit à l'exemple 33.1.

### Exemple 33.4 : Témoin avec PCR sans molécule de révélation :

On incube 10 mg de résine avec 50 µL de PCR dans 400 µL d'eau pure pendant 30 minutes à 60°C. On lave ensuite cette résine par 500 µL de tampon PBS. On traite alors l'échantillon de façon identique au processus décrit à l'exemple 33.1.

### Exemple 33.5 : Témoin avec acide nucléique non capturé :

On incube 10 mg de résine pendant 30 minutes à 60°C avec 400 µL d'eau pure additionnée de 5 µL de *para*-Bio-EG3-PDAM. On lave ensuite cette résine par 500 µL de tampon PBS tween (Réactif Color 0 HLA du kit, PBS pH 7,0 ; Tween 1 %, BND 0,2 g/L ; Ciproflaxacine 0,01 g/L). On resuspend alors la résine dans 100 µL de PBS Tween, et 250 µL de tampon d'hybridation streptavidine additionné de streptavidine HRP dilué au 1/10000 ème. A cette préparation est rajoutée 50µL d'une préparation d'ADN préparée comme suit :

On rajoute 5 µL de *para*-Bio-EG3-PDAM et 70 µL d'eau pure à 25 µL d'ADN provenant d'une PCR préparé comme décrit dans l'exemple 5. Ce mélange est incubé 30 min à 60°C, puis l'excès de marqueur est éliminé en soumettant la préparation à une purification sur colonne QIAquick (Nucleotide Removal Kit, Qiagen, Hilden, Allemagne) suivant le protocole recommandé par le fournisseur, en effectuant une élution finale dans un volume de 50 µL.

Le mélange réactionnel est incubé 30 min à température ambiante, puis il est traité suivant le processus décrit à l'exemple 33.1.

### Résultats

**Tableau 17 : Etude de la réactivité d'une résine portant des groupements diazométhyle**

| **Conditions** | **Absorbance à 492 nm** |
|---|---|
| **Ex. 33.1 :** ADN capturé sur Résine | 527 |
| **Ex. 33.2 :** Sans acide nucléique | 249 |
| **Ex. 33.3 :** PCR sans cible | 261 |
| **Ex. 33.4 :** Témoin sans marqueur | 264 |
| **Ex. 33.5 :** Acide nucléique non capturé | 249 |

Dans le tableau 17, une forte valeur colorimétrique indique une forte concentration d'enzymes dans le milieu réactionnel, correspondant à une présence importante d'acide nucléique portant des dérivés biotines. Les témoins indiquent que le signal n'est pas dû à une adsorption non spécifique de l'ADN sur la bille, à une réaction du *para*-Bio-EG3-PDAM sur la résine, ou à une adsorption de la streptavidine HRP sur la résine, mais bien à la présence d'ADN capturé de façon covalente et marqué par le *para*-Bio-EG3-PDAM.

### Exemple 34 : Marquage d'un produit PCR permettant sa capture et sa détection dans une microplaque :

Il est montré dans cet exemple la possibilité de marquer une molécule d'ADN avec un seul type de molécule portant une fonction diazométhyle, de façon à capturer et à détecter cet acide nucléique en une seule étape, sur une microplaque.

L'expérience est réalisée avec une partie des réactifs présent dans le kit HLA-DR oligo-détection (référence 33 202, bioMérieux), permettant la détection d'acides nucléiques amplifiés par PCR dans des microplaques, par lecture colorimétrique. Dans le cadre de l'expérience décrite, on fait réagir du *para*-Bio-EG3-PDAM, dont la synthèse est décrite dans l'exemple 20, sur des acides nucléiques produits par PCR. L'ADN réagit avec les groupements diazométhyle de la molécule, et se trouve ainsi doté de biotines greffé sur ses phosphate. Il sera alors possible de capturer l'acide nucléique par incubation sur une microplaque où des molécule de streptavidine sont adsorbées, et de le révéler, grâce à une réaction colorimétrique. On utilise un réactif de détection qui est aussi une molécule de streptavidine, associée à une peroxydase de raifort (Horse Radish Peroxidase, HRP). Dans les condition utilisées, la péroxydase peut décomposer une molécule d'OrthoPhénylène Diamine (réactif Color 1 du kit) en un composé détectable à une longueur d'onde de 492 nm.

### Exemple 34.1 : Capture et détection de l'ADN issu de PCR, sur microplaque :

On marque 10 µL d'ADN obtenu par amplification PCR comme décrit dans l'exemple 5 en l'incubant 30 min à 60°C dans 80 µL d'eau pure (Sigma) additionnée de 10 µL de *para-*Bio-EG3-PDAM. Après marquage, l'ADN est purifié sur colonne QIAquick (Nucleotide Removal Kit, Qiagen, Hilden, Allemagne) suivant le protocole recommandé par le fournisseur, et l'éluat final est recueilli dans 50 µL de tampon EB (Tris EDTA 10 mM, pH 8,5). Vingt (20) µL de cet éluat est dilué dans 180 µL de tampon PEG (0,1 M Na PO3 ; 0,5 M NaCl ; 0,65% Tween 20 ; 0,14 mg/mL ADN de sperme de Saumon (Gibco) ; 2 % PEG 4000) additionné de streptavidine HRP (S-911, Molecular Probes, Eugene, OR, USA) diluée au 1/10 000. Cent (100) µL de cette préparation sont ensuite incubés 1 heure à 37°C, soit dans un puits de Combiplate 8, streptavidine coated, (référence 95029263, Labsystem, Helsinki, Finlande) soit dans un puits témoin, provenant d'une barrette Maxisorb. (Nunc, Danemark).

### Exemple 34.2 : Réalisation de témoins :

Des témoins sont simultanément réalisés de la manière suivante :

### A - Témoin de marquage sans ADN :

Quatre-vingt-dix (90) µL d'eau pure additionnés de 10 µL de *para*-Bio-EG3-PDAM sont incubés 30 min à 60°C. Le mélange réactionnel est ensuite traité de façon similaire au processus précédemment décrit à l'exemple 34.1.

### B - Témoin de marquage sans marqueurs :

Dix (10) µL d'ADN obtenus par amplification PCR, comme décrit dans l'exemple 5, sont incubés 30 min à 60°C dans 90 µL d'eau pure. Le mélange réactionnel est ensuite traité de façon similaire au processus précédemment décrit à l'exemple 34.1.

Toutes les barrettes sont ensuite lavées par trois fois 100 µl de tampon PBS Tween (Réactif Color 0 HLA du kit, PBS pH 7,0 ; Tween 1 %, BND 0,2 g/L ; Ciproflaxacine 0,01 g/L) puis la présence de streptavidine HRP est révélée par addition de 100 µL du réactif chromogénique (1 Comprimé Color 1, Chlorydrate d'orthophénylène diamine, dilué dans 5 mL de tampon Color 2, Phosphate de sodium 100 mM, acide citrique 50 mM, H₂O₂ 0,03%), incubé 20 min dans l'obscurité, la réaction étant ensuite bloquée par 50 µL de H₂SO₄ (1,8 N Réactif Color3). L'absorbance du milieu réactionnel est alors mesurée à 492 nm.

### Résultats :

**Tableau 18 : Détection d'ADN capturé et marqué par du para-Bio-EG3-PDAM**

| **Conditions** | **Combiplate** | **Témoin maxisorp** |
|---|---|---|
| A - ADN marqué | 1382 | 152 |
| B - ADN non marqué | 178 | 136 |
| C - Sans ADN | 140 | 192 |

L'expérience, exposée dans le tableau 18, montre donc que l'ADN marqué par du *para*-Bio-EG3-PDAM peut être capturé et détecté en une seule étape dans un puits de microplaque. Comme l'indique les témoins de réaction, le signal généré est uniquement dû à l'ADN et ne résulte pas d'une adsorption non spécifique de l'acide nucléique sur la paroi de la microplaque, ou sur la streptavidine, ou bien à une réaction non spécifique de la streptavidine HRP sur l'ADN non marqué, ou sur le plastique de la microplaque.

### Exemple 35 : Double marquage d'un produit PCR permettant sa capture et sa détection sur un support solide de type microplaque :

Il est montré dans cet exemple la possibilité de marquer avec deux molécules, porteuses de fonctions diazométhyles, et en une seule étape, une molécule d'ADN, de façon la capturer et à la détecter sur une microplaque.

L'expérience est réalisée avec une partie des réactifs présent dans le kit HLA-DR oligo-détection, permettant la détection d'acides nucléiques amplifié par PCR dans des microplaques, par simple lecture colorimétrique. Dans le cadre de l'expérience décrite, on fait réagir simultanément sur des acides nucléiques produits par PCR du :
- 1-Pyrényldiazométhane (PDAM), et
- *para*-Bio-EG3-PDAM.
Si l'ADN réagit avec les fonctions diazométhyle présentes sur les deux composés, il pourra se fixer sur un support portant des anticorps anti-pyrène, et il sera possible de le révéler avec une molécule de streptavidine associée à une péroxydase de raifort. Cette enzyme peut décomposer une molécule d'OrthoPhénylène Diamine, faisant office de réactif de révélation, en un composé détectable à une longueur d'onde de 492 nm.

### Exemple 35.1 : Double marquage de l'ADN et détection sur microplaque :

On a adsorbé sur des barrettes Maxisorp huit (8) puits, des anticorps anti-pyrène, en incubant une nuit à température ambiante 100 µL d'une solution de 1,1 µL d'anticorps anti-pyrène dilués dans 100 µL de tampon bicarbonate (0,05M pH 9,6). De tels anticorps, appelés Rabbit anti-pyrène (Réf.: YSRT-AHP236) sont disponibles chez Accurate Chemical & Scientific (Westbury, New York, Etats-Unis d'Amérique). Bien entendu, ceci aurait pu être réalisé avec d'autres anticorps commercialement disponibles, sans qu'il y ait de résultats divergents par rapport à ceux que nous avons obtenus dans cet exemple.

On a ensuite marqué 10 µL d'ADN obtenu par amplification PCR, comme décrit dans l'exemple 5, en l'incubant 30 min à 60°C dans 40 µL d'eau pure (Sigma), 10 µL de *para-*Bio-EG3-PDAM, 2 µL de PDAM (P-1405, 1-pyrenyldiazomethane, Molecular Probes, Eugene, OR, USA) et 38 µL de DMSO.
Après marquage, l'ADN a été purifié par utilisation d'un kit QIAquick (QIAGEN) et l'éluat final à été recueilli dans 50 µL de tampon EB (Tris EDTA 10 mM, pH 8,5). Vingt (20) µL de cet éluat a été dilué dans 180 µL de tampon PEG (0,1 M Na PO3 ; 0,5 M NaCl ; 0,65 % Tween 20 ; 0,14 mg/mL ADN de sperme de Saumon (Gibco) ; 2 % PEG 4000) additionné de streptavidine HRP (S-911, MOLECULAR PROBES, EUGENE, OR, USA) diluée au 1/10 000. Cent (100) µL de cette préparation ont été ensuite incubés 1 heure à 37°C, soit dans un puits de barrette Maxisorp adsorbé, soit dans un puits témoin, non adsorbé.

### Exemple 35.2 : Réalisation de témoins :

Des témoins ont été simultanément réalisés de la manière suivante :

### A - Témoin de marquage avec para-Bio-EG3-PDAM seul :

Dix (10) µL d'ADN obtenus par amplification PCR, comme décrit dans l'exemple 5, sont marqués par incubation de 30 min à 60°C dans 90 µL d'eau pure additionnée de 10 µL de *para*-Bio-EG3-PDAM. Après marquage, l'ADN est purifié par utilisation d'un kit QIAquick et l'éluat final est recueilli dans 50 µL de tampon EB. Vingt (20) µL de cet éluat est dilué dans 180 µL de tampon PEG additionné de streptavidine HRP diluée au 1/10 000. Cent (100) µL de cette préparation sont ensuite incubés 1 heure à 37°C, soit dans un puits de barrette Maxisorp adsorbé, soit dans un puits non adsorbé.

### B - Témoin de marquage avec PDAM seul :

Dix (10) µL d'ADN obtenus par amplification PCR, comme décrit dans l'exemple 5, sont marqués par incubation de 30 min à 60°C dans 90 µL d'eau pure additionnée de 2 µL de PDAM et 38 µL de DMSO. Après marquage, l'ADN est purifié par utilisation d'un kit QIAquick et l'éluat final est recueilli dans 50 µL de tampon EB. Vingt (20) µL de cet éluat est dilué dans 180 µL de tampon PEG additionné de streptavidine HRP diluée au 1/10 000. Cent (100) µL de cette préparation sont ensuite incubés 1 heure à 37°C, soit dans un puits de barrette Maxisorp adsorbé, soit dans un puits non adsorbé.

### C - Témoin sans marquage :

Dix (10) µL d'ADN obtenus par amplification PCR, comme décrit dans l'exemple 5, sont incubés 30 min à 60°C dans 100 µL d'eau pure. Après marquage, l'ADN est purifié par utilisation d'un kit QIAquick et l'éluat final est recueilli dans 50 µL de tampon EB. Vingt (20) µL de cet éluat est dilué dans 180 µL de tampon PEG additionné de streptavidine HRP diluée au 1/10 000. Cent (100) µL de cette préparation sont ensuite incubés 1 heure à 37°C, soit dans un puits de barrette Maxisorp adsorbé, soit dans un puits non adsorbé.

Les barrettes sont ensuite lavées par trois fois 100 µL de tampon PBS Tween (color 0) puis la présence de streptavidine HRP est révélée par addition de 100 µL du réactif chromogénique (Color 2), incubé 20 min dans l'obscurité, la réaction étant ensuite bloquée par 50 µL de H₂SO₄ (Color3). L'absorbance du milieu réactionnel est alors mesurée à 492 nm.

### Résultats :

**Tableau 19 : Double marquage de l'ADN par le PDAM et le para-Bio-EG3-PDAM**

| **Conditions** | **Anticorps anti-pyrène adsorbés sur plaque (rfu)** | **Témoin plaque non adsorbée (rfu)** |
|---|---|---|
| **Ex. 35.1:** ADN marqué PDAM + *para*-Bio-EG3-PDAM | 348 | 16 |
| **Ex. 35.2.A :** ADN marqué *para*-Bio-EG3PDAM | 44 | 19 |
| **Ex. 35.2.B :** ADN marqué PDAM | 68 | 12 |
| **Ex. 35.2.C :** ADN non marqué | 75 | 19 |

Le résultat du tableau 19 montre clairement un signal important résultant de la capture de l'ADN dans les puits par les anticorps anti-pyrène, ainsi que le marquage simultané de celui-ci par la streptavidine HRP, qui s'est fixée. Comme le montre l'absence de signal au niveau des témoins, cette détection est spécifique de l'ADN marqué, et n'est pas dû à l'adsorption non spécifique de l'ADN ou de la streptavidine HRP sur le plastique, ou à une liaison non spécifique de l'enzyme sur l'ADN capturé. Cet exemple montre donc qu'il est possible de réaliser un double marquage de l'ADN en une seule étape, ce double marquage pouvant être utilisé pour le capturer et le détecter simultanément.

### Exemple 36 : Marquage d'un produit PCR permettant simultanément sa capture et sa détection par sondes nucléiques complémentaires : :

Cette expérience permet de démontrer qu'il est possible de détecter spécifiquement un ADN, ledit ADN étant capturé sur une surface solide en utilisant la réactivité de la fonction diazométhyle vis-à-vis d'un groupement phosphate de l'ADN.

L'expérience est réalisée avec une partie des réactifs présents dans le kit HLA-DR oligo-détection (référence 33 202, bioMérieux, France), permettant la détection d'acides nucléiques amplifié par PCR dans des microplaques, par lecture colorimétrique. Dans le cadre de l'expérience décrite, on fait réagir du *para*-Bio-EG3-PDAM sur des acides nucléiques produit par PCR. L'ADN réagit avec les fonctions diazométhyle de la molécule, et se trouve ainsi doté de biotines greffées sur ses phosphates. Il sera alors possible de capturer l'acide nucléique par incubation sur une microplaque où des molécule de streptavidine sont adsorbées, et de le révéler, grâce à une sonde constitué par un oligonucléotide complémentaire de la séquence capturée, associée à une péroxydase de raifort, cette enzyme pouvant décomposer des molécules incolores d'OrthoPhénylène Diamine (réactif Color 1 du kit) en un composé détectable à une longueur d'onde de 492 nm.

### Exemple 36.1 : Capture et détection spécifique de l'ADN sur microplaque :

On réalise en double le marquage de 10 µL d'ADN obtenu par amplification PCR, en l'incubant 30 min à 60°C avec 20 µL de *para*-Bio-EG3-PDAM. Après marquage, l'ADN est purifié sur colonne QIAquick (Nucleotide Removal Kit, Qiagen, Hilden, Allemagne) suivant le protocole recommandé par le fournisseur et l'éluat final est recueuilli dans 50 µL de tampon EB (Tris-HCl 10 mM, pH 8,5). Quatre-vingt-cinq (85) µL du mélange de ces éluats sont dénaturés par 8,5 µL de réactif R4 (NaOH 2N) durant 5 min à température ambiante, puis la solution est ensuite neutralisée par 8,5 µL de réactif R5 (acide acétique 2 N). On rajoute au mélange 850 µL de tampon d'hybridation (R6 - Tris-HCL 10 mM, pH 7,0 BND 0,2 g/L, Ciproflaxacine 0,01 g/L.) et 85 µL d'oligonucléotide de détection (R7 - Phosphate de sodium 4 mM, Phosphate de potassium 1 mM, pH 7,0, Sérum albumine bovine 0,1 %, Phénol 0,5%). Cent (100) µL de cette préparation sont déposés soit sur le contrôle positif d'une barrette R1 fournie avec le kit, (capture par une séquence consensus du gène amplifié), soit sur une plaque Combiplate 8 streptavidine coated (référence 95029263, Labsystem, Helsinki, Finlande), soit sur une plaque témoin Maxisorp (Nunc, Danemark).

Parallèlement, la même réaction d'hybridation est réalisée sur une dilution, dans du tampon EB, au dixième et au centième, de la préparation d'ADN, afin de tester la sensibilité de la technique.

### Exemple 36.2 : Réalisation des témoins :

Des témoins ont été simultanément réalisés de la manière suivante :

### A - Comparaison au kit HLA-DR :

Dix (10) µL d'ADN obtenus par amplification PCR, comme décrit à l'exemple 5, sont incubés 30 min à 60°C avec 20 µL de *para*-Bio-EG3-PDAM. Après marquage, l'ADN est purifié sur colonne QIAquick, et l'éluat final est recueilli dans un volume de 50 µL de tampon EB. Les 45 µL d'éluat sont dénaturés par 4,5 µL de réactif R4 durant 5 min à température ambiante, puis la solution est neutralisée par 4,5 µL de réactif R5. On rajoute au mélange 450 µL de tampon d'hybridation R6 et 45 µL d'oligonucléotide de détection. Cent (100) µL de cette préparation sont déposés soit sur le contrôle positif d'une barrette R1 fournie avec le kit (hybridation avec une séquence consensus du gène amplifié), soit sur une plaque Combiplate 8 streptavidine, soit sur une plaque témoin Maxisorp.

### B - Hybridation réalisée sur un ADN ne s'hybridant pas à la sonde spécifique :

Dix (10) µL d'ADN obtenu par amplification PCR, comme décrit dans l'exemple 5, sont incubés 30 min à 60°C avec 20 µL de *para*-Bio-EG3-PDAM. On traite alors l'échantillon de façon identique à processus décrit précédemment dans l'exemple A.

### C - Témoin sans ADN :

Dix (10) µL de réactif R6 (tampon d'hybridation) et 100 µL de réactif R7 (oligonucléotide de détection) sont déposés soit sur le contrôle positif d'une barrette R1 fournie avec le kit, sur une plaque streptavidine, soit sur une plaque témoin de type Maxisorp.

Toutes les barrettes des protocoles ci-dessus sont incubées une heure et demie à 37°C, puis lavées par trois fois 100 µL de tampon PBS Tween (réactif color 0 HLA) puis la présence de la sonde de détection spécifique est révélée par addition de 100 µL du réactif chromogénique (Réactif Color 2, , PBS pH 7,0 ; Tween 1 %, BND 0,2 g/L ; Ciproflaxacine 0,01 g/L), incubé 20 min dans l'obscurité, la réaction étant ensuite bloquée par 50 µL de H₂SO₄ (1,8 N réactif Color 3). L'absorbance du milieu réactionnel est alors mesurée à 492 nm.

### Résultats :

**Tableau 20 : Détection spécifique sur microplaque d'un ADN issus de PCR**

| **Conditions** | **Barette R1 Kit HLA DR (Capture spécifique)** | **Combiplate (Streptavidine)** | **Maxisorp (Témoin)** |
|---|---|---|---|
| **A - ADN marqué - Ex 36.1** | **1215** | **2160** | **16** |
| **A - ADN marqué (dilué au 1/10ème)** | NA | **900** | NA |
| **A - ADN marqué (dilué au 1/100ème)** | NA | **53** | NA |
| **B - Témoin ADN non marqué - Ex 36.2 A** | **1153** | **40** | **17** |
| **C- Témoin ADN bactérien - Ex 36.2 B** | **24** | **15** | **N**A |
| **D - Témoin sans ADN - Ex 36.2 C** | **13** | **12** | **17** |

Les résultats du tableau 20 indiquent une excellente amplification de la cible permettant d'envisager une utilisation dans un cadre diagnostic. Cet exemple montre que le marquage au niveau des groupements phosphate permet la capture de l'ADN et n'empêche par l'hybridation spécifique sur celui-ci.

### Exemple 37 : Capture et amplification d'ADN issu d'un lysat bactérien et marqué par para-Bio-EG3-PDAM :

Cet exemple montre qu'il est possible de capturer et d'amplifier un ADN bactérien en utilisant une capture basée sur la réactivité de la fonction diazométhyle sur le phosphate de l'acide nucléique.

Dans le cas présent, les acides nucléiques contenus dans un lysat bactérien et marqués par du *para*-Bio-EG3-PDAM, sont capturés sur des billes magnétiques recouvertes de streptavidine. L'utilisation de billes magnétiques permet d'immobiliser celles-ci par aimantation lors des lavages successifs visant à éliminer les résidus cellulaires, présents dans le milieu réactionnel, ces résidus devant être éliminés car ils peuvent inhiber l'amplification par PCR qui est pratiqué par la suite.
Les produits d'amplification ont pu être analysés par passage sur puces à ADN.

L'ADN bactérien est obtenu par lyse des cellules contenues dans une culture de *Mycobacterium tuberculosis.* La lyse s'effectue par lyse mécanique. Plus précisément, elle est réalisée par sonication, l'échantillon liquide traité contenant des billes de verre. Un tel procédé est déjà bien décrit par la Demanderesse dans sa demande de brevet WO-A-99/15621, en ce qui concerne les billes, et dans sa demande de brevet WO-A-00/60049, en ce qui concerne la sonication. La sonication peut également être réalisée à l'aide d'un bain liquide.
Toutefois d'autres techniques, connues de l'homme du métier, peuvent être utilisées comme celles décrites dans le brevet US-A-5,902,746 et les demandes de brevet WO-A-98/54306 et WO-A-00/05338. Tous ces titres de Propriété Industrielle appartiennent à la Demanderesse.

L'ADN bactérien a été quantifié par Picogreen (P-7589 ; Molecular Probes, Eugene, OR, USA)suivant le protocole décrit par le fournisseur, à une concentration de 10⁷ copies par µL.

Dix (10) µL de lysat sont incubés en présence de 20 µL de *para*-Bio-EG3-PDAM durant 30 minutes à 60°C. Parallèlement, 10 µL de lysat sont incubés dans 20 µL d'eau pure (Sigma) dans les mêmes conditions.

Le milieu réactionnel est ensuite purifié sur colonne QIAquick (Nucleotide Removal Kit, Qiagen, Hilden, Allemagne). Le protocole de purification utilisé est celui recommandé par le fournisseur. Le volume final d'élution est de 50 µL.

Les fragments d'ADN marqués sont ensuite capturés sur des billes magnétiques Dynal (Dynabeads M-280 streptavidin ; référence 112.05 ; Dynal Biotech ASA, Oslo, Norway), qui sont préparées selon le protocole suivant :
Quatre-vingt-dix (90) µL de billes Dynal sont lavés deux fois par 200 µL d'eau pure Free (Sigma), puis sont repris par 200 µL de tampon PEG (0,1 M Na PO₄, pH 7, 0,5 M NaCl ; 0,65 % Tween 20 ; 0,14 mL ADN de sperme de Hareng (Référence 15634-017, GibcoBRL) ; 2 % PEG 4000) et incubés 30 min à 37°C. Elles sont ensuite lavées deux fois par 200 µL de tampon PBS 1X Tween 20 0,5%, puis finalement reprises par 90 µL du même tampon.

Dix (10) µL des éluats d'ADN marqués ou non marqués sont incubés 5 min à température ambiante avec 40 µL de tampon PEG et 2,5 µL de la préparation de billes magnétiques décrite précédemment.

Les billes sont ensuite lavées trois fois par 200 µL de tampon PBS 1 X tween 0,5%, reprises dans 200 µL d'eau et incubées 20 min à 60°C, puis lavées de nouveau quatre fois par 200 µL de PBS tween. Les billes sont finalement reprises par 25 µL d'eau et une PCR est réalisée en suivant le protocole décrit à l'exemple 5. Deux contrôles réactionnels sont effectués l'un avec 25 µL d'eau pure et l'autre avec 2,5 µL de billes préparées et lavées dans les mêmes conditions que les échantillons biologiques, et reprises dans 25 µL d'eau.

### Résultat :

Les produits de PCR sont ensuite quantifiés par Picogreen (P-7589 ; Molecular Probes, Eugene, OR, USA) suivant le protocole décrit par le fournisseur. Cette méthode repose sur l'utilisation d'une molécule (Picogreen) ayant la particularité de devenir fluorescente uniquement lorsqu'elle se positionne à l'intérieure d'une molécule d'ADN (en s'intercalant entre les bases). En raison du caractère très spécifique de cette intercalation, et du fait que le signal fluorescent produit est directement proportionnel à la quantité d'ADN présent dans le milieu, il est possible de doser de cette manière, de façon très précise, la concentration d'acide nucléique présente dans un échantillon. Le signal est alors exprimé en rfu (relative fluorescent unit).
L'analyse des résultats de PCR sur gel montre la présence d'une seule bande spécifique à la taille attendue dans les échantillons réalisé à partir d'ADN génomique marqué par *para-Bio-*EG3-PDAM. Les bandes ne sont pas détectées lorsque la PCR a été effectuée à partir d'ADN génomique non marqué. Une quantification de l'ADN par Picogreen permet de confirmer la production d'ADN à partir d'ADN génomique capturé sur billes.

**Tableau 21 : Quantification de l'ADN produit par PCR, à partir d'un lysat bactérien, capturé et purifié par le para-Bio-EG3-PDAM**

| **Conditions** | **rfu** |
|---|---|
| Témoins non Marqués | 51 |
| ADN marqués | 170 |
| Bruit de fond | 20 |

Une analyse sur puce à ADN, suivant le protocole décrit à l'exemple 8 permet de confirmer la spécificité de l'amplification comme le montre le taleau 34 ci-dessous.

**Tableau 22 : Détection spécifique de la cible capturée et purifiée par le para-Bio-EG3-PDAM**

| | **Homologie %** | **I (rfu)** | **B(rfu)** | **I/B** |
|---|---|---|---|---|
| Echantillon | 98 | 11531 | 723 | 16 |

Cet exemple montre qu'il est possible de préparer un échantillon biologique de façon à amplifier l'acide nucléique qu'il contient, en utilisant une technique de capture basée sur la réactivité de la fonction diazométhyle sur les groupements phosphate de celui-ci.

### Exemple 38 : Amplification successive de deux gènes à partir d'un ADN bactérien capturé sur un support solide :

Cet exemple montre qu'il est possible d'amplifier à plusieurs reprises et sur des cibles différentes un ADN capturé grâce à la réactivité de la fonction diazométhyle sur les groupements phosphates de celui-ci.

Dans le cas présent, les acides nucléiques, contenus dans un lysat bactérien et marqués par du *para*-Bio-EG3-PDAM, sont capturés sur des billes magnétiques recouvertes de streptavidine. L'utilisation de billes magnétiques permet de conserver celles-ci par aimantation lors des lavages successifs visant à éliminer les résidus cellulaires présents dans le milieu réactionnel, ces résidus devant être éliminés car ils peuvent inhiber les amplifications par PCR, qui seront pratiquées par la suite. Ces amplifications vont avoir lieu sur deux gènes différents présents dans l'ADN génomiques, respectivement désignés sous les noms de 16S et rpoB. Ces deux gênes sont ensuite analysés par utilisation de puces à ADN.

L'ADN bactérien est obtenu par lyse des cellules contenues dans une culture de *Mycobacterium tuberculosis,* suivant le protocole déjà décrit dans l'exemple 37. L'ADN bactérien a été quantifié par Picogreen suivant le protocole décrit par le fournisseur, à une concentration de 10⁷ copies par µL.

Dix (10) µL de lysat sont incubés en présence de 20 µL de *para*-Bio-EG3-PDAM durant 30 minutes à 60°C. Parallèlement, 10 µL de lysat sont incubés dans 20 µL d'eau pure (Sigma) dans les mêmes conditions.

Le milieu réactionnel est ensuite purifié sur colonne QIAquick (Nucleotide Removal Kit, Qiagen, Hilden, Allemagne). Le protocole de purification utilisé est celui recommandé par le fournisseur. Le volume final d'élution est de 50 µL.

Les fragments d'ADN marqués sont ensuite capturés sur des billes magnétiques Dynal, qui sont préparées suivant le protocole suivant :
Quatre-vingt-dix (90) µL de billes Dynal sont lavées deux fois par 200 µL d'eau pure Free (Sigma), puis sont reprises par 200 µL de tampon PEG (0,1 M Na PO₄, pH 7, 0,5 M NaCl ; 0,65 % Tween 20 ; 0,14 mL ADN de sperme de saumon (Gibco) ; 2 % PEG 4000) et incubées 30 min à 37°C. Elles sont ensuite lavées deux fois par 200 µL de tampon PBS 1X Tween 20 0.5%, puis finalement reprises par 90 µL du même tampon.

Dix (10) µL des éluats d'ADN marqués ou non marqués sont incubés 5 min à température ambiante avec 40 µL de tampon PEG et 2,5 µL de la préparation de billes magnétiques décrite précédemment.

Les billes sont ensuite lavées trois fois par 200 µL de tampon PBS 1 X tween 0,5 %, reprise dans 200 µL d'eau et incubées 20 min à 60°C, puis lavées de nouveau quatre fois par 200 µL de PBS tween. Les billes sont finalement reprises par 25 µL d'eau et une PCR est réalisée en suivant le protocole décrit dans l'exemple 5. Deux contrôles réactionnels sont effectués l'un avec 25 µL d'eau pure (Sigma) et l'autre avec 2,5 µL de billes préparées et lavées dans les même, conditions que les échantillons biologiques, et reprise, dans 25 µL d'eau.

Après amplification, le milieu réactionnel est recueilli, et les billes sont séparées et lavées par 150 µL de PBS 1 X Tween 0,5%, puis resuspendues dans 25 µL d'eau pure (Sigma). Une nouvelle amplification est réalisée sur les billes, mais en présence d'amorces destinées à amplifier le gène rpoB.

Des amplifications témoins réalisés à partir d'ADN génomique non capturé sont réalisées en parrallèle sur les deux systèmes d'amplification (rpoB et 16S).

### Résultat :

Les produits de PCR obtenus sont ensuite analysés par puces à ADN suivant le protocole décrit à l'exemple 8.

**Tableau 23 : Analyse sur puces à ADN des amplicons ADN issus des PCR amplifiées successivement ou non**

| **Conditions** | | **Homologie (%)** | **I (rfu)** | **B (rfu)** | **I/B** |
|---|---|---|---|---|---|
| PCR témoin 16S | 16S | 96 | 4662 | 387 | 12 |
| Résultats sur séquence rpoB | RpoB | 23 | 237 | 358 | 1 |
| PCR témoin rpoB | RpoB | 98 | 5438 | 397 | 14 |
| Résultats sur séquence 16S | 16S | 17 | 183 | 391 | 1 |
| PCR 16S sur billes | 16S | 98 | 2726 | 534 | 5 |
| Résultats sur séquence rpoB | RpoB | 8 | 161 | 480 | <1 |
| PCR rpoB sur billes lavées | RpoB | 97 | 3205 | 349 | 9 |
| Résultats sur séquence 16S | 16S | 14 | 84 | 358 | <1 |

L'analyse des résultats de PCR sur gel montre la présence d'une seule bande spécifique à la taille attendue dans les échantillons réalisés à partir d'ADN génomique marqué par *para-*Bio-EG3-PDAM. Les bandes ne sont pas détectées lorsque la PCR a été effectuée à partir d'ADN génomique non marqué.
Une analyse par puce à ADN, telle que présentée sur le tableau 23 ci-dessus, permet de confirmer la spécificité des deux amplifications successives, et donc la capacité de réaliser une amplification successive de plusieurs gènes à partir d'ADN immobilisé sur un support solide, ceci permettant d'éviter la mise au point de systèmes multiplex, qui réduisent souvent de façon importante la sensibilité et l'efficacité des amplifications d'acides nucléiques.

### Exemple 39 : Capture et Amplification d'ADN sur une membrane de Nylon portant des groupements diazométhyles :

Une membrane de nylon activée de façon à porter des groupements diazométhyles a été utilisée afin de capturer de l'ADN bactérien, dans le but de l'amplifier par PCR.

### Exemple 39.1 : Modification du filtre Biodyne C :

### Schéma de synthèse :

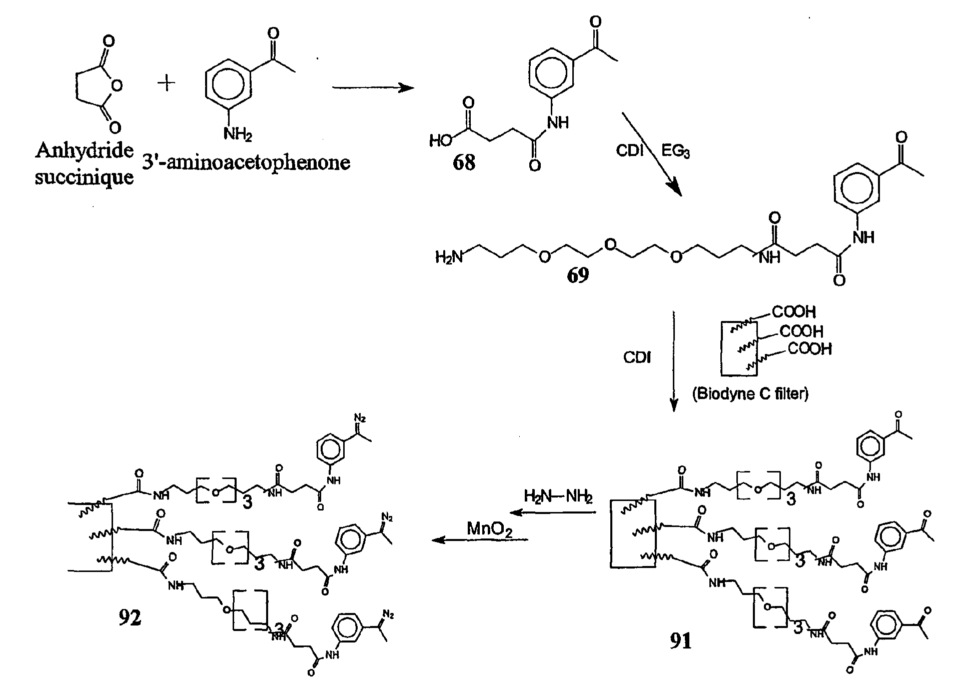

### Composé 68 :

On solubilise la 3'-aminoacetophenone (14,5 g, 107 mmol) dans 50 ml de DMF anhydre. On ajoute l'anhydride succinique (10,7 g, 107 mmol) et on laisse sous agitation, sous argon et à température ambiante. Après 6 h, la solution est concentrée sous vide et 50 ml de méthanol sont ajoutés. Le précipité obtenu est filtré et lavé avec méthanol et éther. On obtient ainsi 19,4 g (81 %) de produit **68** sous forme d'une poudre de couleur blanc cassé.

RMN ¹H (200 MHz, DMSO-d₆): d= 2,5-2,6 (m, 7H); 7,45 (t, 1H); 7,64 (d, 1H); 7,83 (d, 1H); 8,19 (s, 1H); 10,16 (s, 1H); 12,12 (s, 1H).

### Composé 69 :

On solubilise 5,07 g (22 mmol) du composé **68** dans 10 ml de DMF anhydre, sous argon. On met sur glace et on ajoute 5,00 g (32 mmol) de carbonyldiimidazole. Apres 20 min, on ajoute lentement 20 ml (94,6 mmol) du 4,7,10-trioxatridecanediamine (EG₃). Après 3h de réaction à température ambiante, on évapore le DMF et on reprend le résidu dans 100 ml de CH₂Cl₂. On fait des extractions avec du NaHCO₃ saturé et H₂O, après quoi la phase organique est séchée avec du Na₂SO₄ anhydre et le solvant évaporé. On obtient ainsi 4,34 g (46 %) du produit **69.**

RMN ¹H (200 MHz, DMSO-d₆): d= 1,59 (m, 2H); 1,87 (m, 2H); 2,16 (s, 3H); 2,40 (m, 2H); 2,55 (m, 2H); 3,08 (m, 2H); 3,45 (m, 16H); 7,30 (t, 1H); 7,42 (d, 1H); 7,70 (d, 1H); 7,83 (t, 1H); 7,97 (s, 1H); 10,00 (s, 1H).

### Composé 91:

Un rectangle de 4 cm² est découpé sur une feuille de filtre Biodyne C (référence 60314 ; Pall Gelman Laboratory ; Ann Arbor ; Michigan ; USA), introduit dans un flacon et mis en contact avec 0,97 g (6 mmol) de carbonyldiimidazole (CDI) dans 3 ml de DMF anhydre, sur glace, sous argon et sous forte agitation. Après 20 min, la solution est éliminée et le filtre lavé avec du DMF. Une quantité de 0,53 g du produit **68** (1 mmol) dans 3 ml de DMF anhydre est ensuite ajoutée, et la réaction se réalise pendant la nuit à température ambiante. La solution est ensuite enlevée et le filtre est rincé avec de l'éthanol, séché sous vide et gardé sous argon.

### Composé 92 :

Le filtre modifié **91** est mis dans une solution de 97 ml d'hydrazine hydrate (2 mmol) dans 4 ml d'éthanol absolu. La solution est mise à reflux pendant 5 h. Après avoir laissé refroidir, le filtre est lavé avec H₂O, éthanol et éther, séché sous vide et mis sous argon. Ensuite, on ajoute 4 ml de DMF anhydre et 86 mg de MnO₂ (1 mmol), et on laisse réagir sous forte agitation. Après 20 min, la solution est rejetée, et le filtre est rincé avec du DMF et de l'éther. Le filtre modifié-diazométhyl **92** est conservé sous argon, à une température de -19 à -31°C.

### Exemple 39.2 : Essais biologiques :

La membrane activée est découpée en petits fragments de 2 mm² qui vont être incubés pendant 30 minutes à température ambiante dans 25 µl de lysat bactérien de *Mycobacterium tuberculosis,* préparé par lyse mécanique, suivant la même technique et la même concentration finale que dans l'exemple 37, et 375 µl d'eau pure (Sigma).

La membrane est ensuite placée à 65°C pendant 60 min dans 100 mL de tampon de lavage (5 % Formamide (Sigma), 1X SSPE (Perbio), 0.01 % triton X-100) afin d'éliminer les acides nucléiques non spécifiques adsorbés sur la membrane, puis celle-ci est stockée dans 1 mL d'eau pure avant amplification.

La PCR est pratiquée comme décrite dans le paragraphe 5.1, le volume réactionnel étant complété par une quantité suffisante d'eau pure.

Parallèlement, des contrôles sont effectués suivant le même processus avec des membranes ne pouvant pas lier de façon covalente les acides nucléiques :
- Membrane Biodyne C non modifiée (membrane A),
- Membrane Biodyne modifiée chimiquement suivant le processus décrit, mais non traitée par du DMF anhydre et du MnO₂ ; ce contrôle permet de vérifier le comportement de la membrane en l'absence de groupements diazométhyles (membrane B), et
- Membrane Biodyne C non modifiée, mais traitée 20 min sous forte agitation par du DMF anhydre et du MnO₂, ce contrôle permettant de vérifier que cette dernière étape ne modifie pas l'adsorption de l'ADN sur la membrane (membrane C).

Afin de contrôler l'absence d'inhibition de la PCR provoquée par le traitement des membranes, un autre fragment des membranes A, B, C est amplifié simultanément avec 25 µl de lysat bactériens.(tubes A', B', C')

Les produits de PCR sont ensuite quantifiés par Picogreen suivant le protocole décrit par le fournisseur.

### Résultat :

**Tableau 24 : Quantification de l'ADN obtenu par PCR à partir d'ADN de lysat bactérien capturé sur support solide**

| **Tests effectués** | **Signal (rfu)** |
|---|---|
| Membrane modifiée | 111 |
| Membrane non modifiée (A) | 18 |
| Membrane non modifiée, co-amplifiée avec 25 µL de lysat bactérien (A') | 260 |
| Membrane modifiée non activée (B) | 26 |
| Membrane modifiée non activée, co-amplifiée avec 25 µL de lysat bactérien (B') | 264 |
| Membrane non modifiée ayant subit une activation (C) | 21 |
| Membrane non modifiée ayant subit une activation, co-amplifiée avec 25 µL de lysat bactérien (C') | 268 |

Ces résultats du tableau 24 indiquent qu'il est possible de capturer de façon covalente sur un support solide, des acides nucléiques, issus de lysat, grâce à la chimie diazométhyle. L'amplification observée n'est pas due à une adsorption non spécifique de l'ADN sur la membrane. D'autre part, on observe avec les contrôles effectués avec les PCR que les membranes ne provoquent pas d'inhibition de la réaction d'amplification.

Afin de contrôler la nature du produit amplifié sur la membrane, le produit d'amplification a été analysé par passage sur puce à ADN, suivant le protocole décrit auparavant.

## Revendications

1. Procédé de marquage et de fragmentation d'un acide désoxyribonucléique (ADN) simple ou double brin comprenant:
- fragmenter l'ADN par l'intermédiaire de la création d'au moins un site abasique sur ledit ADN,
- attacher un marqueur sur au moins un des fragments par l'intermédiaire d'un réactif de marquage, ledit réactif se couplant de manière covalente et majoritaire sur au moins un phosphate dudit fragment, la fragmentation et le marquage étant réalisés en une étape.

2. Procédé pour la mise à disposition de sondes de détection d'un acide nucléique cible comprenant le marquage et la fragmentation d'un acide désoxyribonucléique (ADN) simple ou double brin comprenant les étapes suivantes :
- fragmenter l'ADN par l'intermédiaire de la création d'au moins un site abasique sur ledit ADN,
- attacher un marqueur sur au moins un des fragments par l'intermédiaire d'un réactif de marquage, ledit réactif se couplant de manière covalente et majoritaire sur au moins un phosphate dudit fragment.

3. Procédé, selon la revendication 2, **caractérisé par le fait que** la fragmentation et le marquage sont effectués en deux étapes.

4. Procédé, selon la revendication 2, **caractérisé par le fait que** la fragmentation et le marquage sont réalisés en une étape.

5. Procédé, selon l'une quelconque des revendications 1 à 4, **caractérisé par le fait que** le site abasique est généré par action d'un milieu aqueux acide.

6. Procédé, selon la revendication 5, **caractérisé par le fait que** le pH du milieu acide est inférieur à 5, de préférence inférieur à 4.

7. Procédé, selon la revendication 6, **caractérisé par le fait que** le milieu aqueux acide est un tampon formiate de sodium à pH d'environ 3.

8. Procédé, selon l'une quelconque des revendications 1 à 7, **caractérisé par le fait que** l'ADN contient au moins une base modifiée susceptible de générer un site abasique.

9. Procédé, selon la revendication 8, **caractérisé par le fait que** la base modifiée susceptible de générer un site abasique est choisi parmi les dérivés de la 8-bromo purine.

10. Procédé, selon l'une quelconque des revendications 1 à 4, **caractérisé par le fait que** le site abasique est généré par un agent akylant ou un agent oxydant.

11. Procédé selon l'une quelconque des revendications 1 à 10, **caractérisé par le fait que** le réactif de marquage comprend, comme fonction réactive, un motif choisi parmi les composés suivants : diazométhyle ; halogénure d'alkyle ; nitrosourée ; spirocyclopropane ; aziridine ; époxyde ; trifuorosulfonates.

12. Procédé, selon la revendication 11 **caractérisé par le fait que** ledit réactif de marquage est la 5-(bromométhyl)fluorescéine.

13. Procédé, selon la revendication 11, **caractérisé par le fait que** le réactif de marquage est choisi parmi les composés de formule (1): 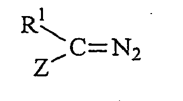 dans laquelle :
• R¹ représente H ou un groupe alkyle, alkyle substitué, aryle ou aryle substitué, et
• Z comprend un marqueur détectable.

14. Procédé, selon la revendication 13, **caractérisé par le fait que** Z est :

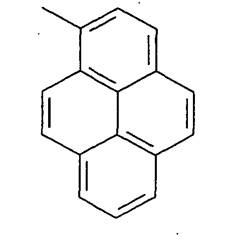

15. Procédé, selon la revendication 14, **caractérisé par le fait que** le réactif de marquage est choisi panni les composés de formule (2) : 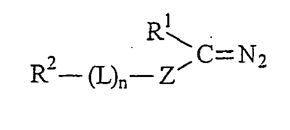 dans laquelle
• R¹ représente H ou un groupe alkyle, aryle ou aryle substitué,
• R² est un marqueur détectable,
• L est un bras de liaison comportant un enchaînement linéaire d'au moins deux liaisons covalentes et n est égal à 0 ou 1, et
• Z est choisi parmi : 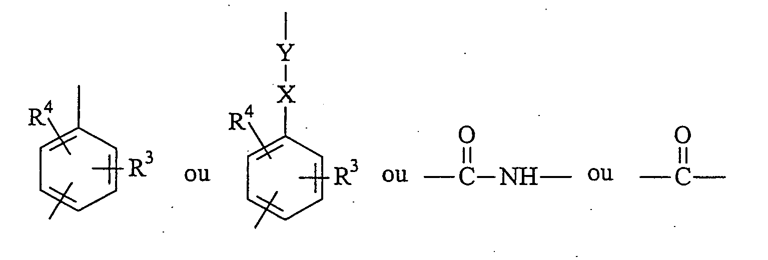 dans lequel :
■ R³ et R⁴ représentent indépendamment l'un de l'autre : H, NO₂, Cl, Br, F, I, OR, SR, NR₂, R, NHCOR, CONHR, COOR avec R = alkyle ou aryle, et
■ -Y-X- représente -CONH-, -NHCO-, -CH₂O-, -CH₂S-.

16. Procédé, selon la revendication 15, **caractérisé par le fait que** le réactif de marquage a la formule (3) : 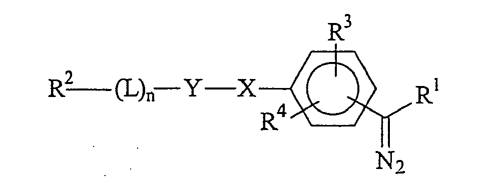 dans laquelle :
• R¹ représente H ou un groupe alkyle, aryle ou aryle substitué,
• R² représente un marqueur détectable,
• L est un bras de liaison comportant un enchaînement linéaire d'au moins 2 liaisons covalentes et n un nombre entier égal à 0 ou 1,
• R³ et R⁴ représentent indépendamment l'un de l'autre : H, NO₂, Cl, Br, F, I, OR, SR, NR₂, R, NHCOR, CONHR, COOR avec R = alkyle ou aryle, et
• -Y-X- représente -CONH-, -NHCO-, -CH₂O-, -CH₂S-.

17. Procédé, selon l'une quelconque des revendications 15 ou 16, **caractérisé par le fait que** R² est un composé fluorescent ou un haptène.

18. Procédé, selon l'une quelconque des revendications 11 à 17, **caractérisé par le fait que** ledit réactif de marquage est soluble dans un solvant miscible à l'eau.

19. Procédé de détection d'un acide désoxyribonucléique cible (ADN) simple ou double brin comportant les étapes suivantes :
• fragmenter et marquer lesdits ADN, selon l'un quelconque des procédés selon les revendications 1 à 18,
• hybrider les fragments marqués sur au moins une sonde nucléique suffisamment spécifique de l'acide nucléique cible, et
• détecter l'hybride formé par l'intermédiaire du marqueur.

20. Procédé de détection d'un acide désoxyribonucléique cible (ADN) double brin, selon la revendication 19 comportant en outre une étape de dénaturation après la fragmentation et le marquage.

21. Procédé, selon la revendication 20, **caractérisé par le fait que** la fragmentation, le marquage et la dénaturation sont réalisés en une seule étape.

22. Procédé de détection d'un acide nucléique cible comportant les étapes suivantes :
• amplifier enzymatiquement l'acide nucléique cible pour générer une multitude d'amplicons ADN double brin,
• fragmenter et marquer lesdits amplicons ADN, selon l'un quelconque des procédés selon les revendications 1 à 18,
• hybrider les fragments marqués sur au moins une sonde nucléique suffisamment spécifique de l'acide nucléique cible, et
• détecter l'hybride formé par l'intermédiaire du marqueur.

23. Procédé, selon la revendication 22, comportant en outre une étape de dénaturation après l'étape de fragmentation et marquage.

24. Procédé, selon la revendication 23, **caractérisé par le fait que** la fragmentation, le marquage et la dénaturation sont réalisés en une seule étape.

25. Procédé de mise en évidence d'un polymorphisme réparti en des positions prédéterminées ou non d'un acide nucléique cible par la présence d'une pluralité de délétions et/ou insertions et/ou mutations dans la séquence dudit acide nucléique cible par rapport à une séquence dite de référence comportant les étapes suivantes :
• disposer d'un ADN cible comportant l'ensemble du polymorphisme à étudier, ledit ADN étant éventuellement généré par une technique d'amplification enzymatique.
• fragmenter et marquer ledit ADN par un procédé, selon l'une quelconque des revendications 1 à 18.
• hybrider lesdits fragments sur une pluralité de sondes nucléiques dites sondes de capture, la pluralité de sondes de capture étant fixé sur un support solide et la pluralité de sondes de capture couvrant dans son ensemble au moins le polymorphisme à étudier.
• détecter les hybrides formés entre les fragments marqués et au moins une partie des sondes nucléiques par l'intermédiaire du marqueur et en déduire le polymorphisme de l'ADN cible.

26. Procédé, selon la revendication 25, comportant en outre une étape de dénaturation après l'étape de fragmentation et marquage.

27. Procédé, selon la revendication 26, **caractérisé par le fait que** la fragmentation, le marquage et la dénaturation sont réalisés en une seule étape.

28. Procédé, selon l'une quelconque des revendications 25 à 27, **caractérisé par le fait que** le support solide comporte au moins dix (10) sondes nucléiques de séquences différentes, avantageusement au moins quatre cents (400), de préférence au moins mille (1000).

## Claims

1. Method for labelling and fragmenting a single-stranded or double-stranded deoxyribonucleic acid (DNA), comprising:
- fragmenting the DNA by means of the creation of at least one abasic site on said DNA,
- attaching a label to at least one of the fragments by means of a labelling reagent, said reagent coupling covalently and predominantly to at least one phosphate of said fragment, the fragmentation and the labelling being carried out in one step.

2. Method for the provision of probes for detecting a target nucleic acid, comprising the labelling and the fragmentation of a single-stranded or double-stranded deoxyribonucleic acid (DNA), comprising the following steps:
- fragmenting the DNA by means of the creation of at least one abasic site on said DNA,
- attaching a label to at least one of the fragments by means of a labelling reagent, said reagent coupling covalently and predominantly to at least one phosphate of said fragment.

3. Method according to Claim 2, **characterized in that** the fragmentation and the labelling are carried out in two steps.

4. Method according to Claim 2, **characterized in that** the fragmentation and the labelling are carried out in one step.

5. Method according to any one of Claims 1 to 4, **characterized in that** the abasic site is generated by the action of an acidic aqueous medium.

6. Method according to Claim 5, **characterized in that** the pH of the acidic medium is less than 5, preferably less than 4.

7. Method according to Claim 6, **characterized in that** the acidic aqueous medium is a sodium formate buffer having a pH of approximately 3.

8. Method according to any one of Claims 1 to 7, **characterized in that** the DNA contains at least one modified base capable of generating an abasic site.

9. Method according to Claim 8, **characterized in that** the modified base capable of generating an abasic site is chosen from 8-bromopurine derivatives.

10. Method according to any one of Claims 1 to 4, **characterized in that** the abasic site is generated by an alkylating agent or an oxidizing agent.

11. Method according to any one of Claims 1 to 10, **characterized in that** the labelling reagent comprises, as reactive function, a unit chosen from the following compounds: diazomethyl; alkyl halide; nitrosourea; spirocyclopropane; aziridine; epoxide; trifluorosulfonates.

12. Method according to Claim 11, **characterized in that** the labelling reagent is 5-(bromomethyl)-fluorescein.

13. Method according to Claim 11, **characterized in that** the labelling reagent is chosen from the compounds of formula (1): 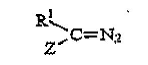 in which:
• R¹ represents H or an alkyl, substituted alkyl, aryl or substituted aryl group, and
• Z comprises a detectable label.

14. Method according to Claim 13, **characterized in that** Z is:

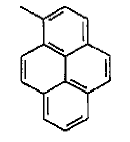

15. Method according to Claim 14, **characterized in that** the labelling reagent is chosen from the compounds of formula (2): 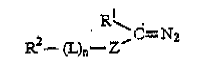 in which
• R¹ represents H or an alkyl, aryl or substituted aryl group,
• R² is a detectable label,
• L is a linker arm containing a linear chain of at least two covalent bonds and n is equal to 0 or 1, and
• Z is chosen from: 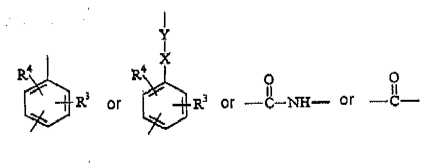 in which:
■ R³ and R⁴ represent, independently of one anqther: H, NO₂, Cl, Br, F, I, OR, SR, NR₂, R, NHCOR, CONHR or COOR with R = alkyl or aryl, and
■ -Y-X- represents -CONH-, -NHCO-, -CH₂O-, -CH₂S-.

16. Method according to Claim 15, **characterized in that** the labelling agent has the formula (3): 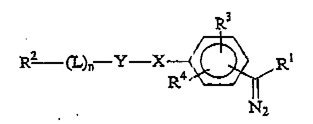 in which:
• R¹ represents H or an alkyl, aryl or substituted aryl group,
• R² represents a detectable label,
• L is a linker arm containing a linear chain of at least 2 covalent bonds and n is an integer equal to 0 or 1,
• R³ and R⁴ represent, independently of one another: H, NO₂, Cl, Br, F, I, OR, SR, NR₂, R, NHCOR, CONHR or COOR with R = alkyl or aryl, and
• -Y-X- represents -CONH-, -NHCO-, -CH₂O- or -CH₂S-.

17. Method according to either one of Claims 15 and 16, **characterized in that** R² is a fluorescent compound or a hapten.

18. Method according to either one of Claims 16 and 17, **characterized in that** said labelling reagent is soluble in a water-miscible solvent.

19. Method for detecting a single-stranded or double-stranded target deoxyribonucleic acid (DNA), comprising the following steps:
• fragmenting and labelling said DNAs, according to any one of the methods according to Claims 1 to 18,
• hybridizing the labelled fragments to at least one nucleic acid probe sufficiently specific for the target nucleic acid, and
• detecting the hybrid formed, by means of the label.

20. Method for detecting a double-stranded target deoxyribonucleic acid (DNA), according to Claim 19, also comprising a denaturation step after the fragmentation and the labelling.

21. Method according to Claim 20, **characterized in that** the fragmentation, the labelling and the denaturation are carried out in a single step.

22. Method for detecting a target nucleic acid, comprising the following steps:
• enzymatically amplifying the target nucleic acid so as to generate a multitude of double-stranded DNA amplicons,
• fragmenting and labelling said DNA amplicons, according to any one of the methods according to Claims 1 to 18,
• hybridizing the labelled fragments to at least one nucleic acid probe sufficiently specific for the target nucleic acid, and
• detecting the hybrid formed, by means of the label.

23. Method according to Claim 22, also comprising a denaturation step after the fragmentation and labelling step.

24. Method according to Claim 23, **characterized in that** the fragmentation, the labelling and the denaturation are carried out in a single step.

25. Method for demonstrating a polymorphism distributed in possibly predetermined positions of a target nucleic acid, by means of the presence of a plurality of deletions and/or insertions and/or mutations in the sequence of said target nucleic acid compared with a "reference" sequence, comprising the following steps:
• providing a target DNA containing the entire polymorphism to be studied, said DNA being optionally generated by means of an enzymatic amplification technique,
• fragmenting and labelling said DNA by means of a method according to any one of Claims 1 to 18,
• hybridizing said fragments to a plurality of nucleic acid probes referred to as capture probes, the plurality of capture probes being attached to a solid support and the plurality of capture probes covering, in its entirety, at least the polymorphism to be studied,
• detecting the hybrids formed between the labelled fragments and at least some of the nucleic acid probes, by means of the label, and deducing therefrom the polymorphism of the target DNA.

26. Method according to Claim 25, also comprising a denaturation step after the fragmentation and labelling step.

27. Method according to Claim 26, **characterized in that** the fragmentation, the labelling and the denaturation are carried out in a single step.

28. Method according to any one of Claims 25 to 27, **characterized in that** the solid support comprises at least ten (10) nucleic acid probes of different sequences, advantageously at least four hundred (400), preferably at least a thousand (1000).

## Patentansprüche

1. Verfahren zur Markierung und Fragmentierung einer einzel- oder doppelsträngigen Desoxyribonukleinsäure (DNA), umfassend:
- Fragmentieren der DNA durch Erzeugen mindestens einer abasischen Stelle auf der DNA,
- Binden eines Markers an mindestens eines der Fragmente durch ein Markierungsreagens, wobei das Reagens kovalent und größtenteils an mindestens ein Phosphat des Fragments kuppelt, wobei Fragmentierung und Markierung in einem Schritt erfolgen.

2. Verfahren zur Bereitstellung von Sonden zum Nachweis einer Zielnukleinsäure, umfassend die Markierung und Fragmentierung einer einzel- oder doppelsträngigen Desoxyribonukleinsäure (DNA), umfassend die folgenden Schritte:
- Fragmentieren der DNA durch Erzeugen mindestens einer abasischen Stelle auf der DNA,
- Binden eines Markers an mindestens eines der Fragmente durch ein Markierungsreagens, wobei das Reagens kovalent und größtenteils an mindestens ein Phosphat des Fragments kuppelt.

3. Verfahren nach Anspruch 2, **dadurch gekennzeichnet, dass** Fragmentierung und Markierung in zwei Schritten erfolgen.

4. Verfahren nach Anspruch 2, **dadurch gekennzeichnet, dass** Fragmentierung und Markierung in einem Schritt erfolgen.

5. Verfahren nach einem der Ansprüche 1 bis 4, **dadurch gekennzeichnet, dass** die abasische Stelle durch Einwirkung eines wässrigen sauren Mediums erzeugt wird.

6. Verfahren nach Anspruch 5, **dadurch gekennzeichnet, dass** der pH des sauren Mediums kleiner als 5, vorzugsweise kleiner als 4 ist.

7. Verfahren nach Anspruch 6, **dadurch gekennzeichnet, dass** das wässrige saure Medium ein Natriumformiatpuffer mit einem pH von etwa 3 ist.

8. Verfahren nach einem der Ansprüche 1 bis 7, **dadurch gekennzeichnet, dass** die DNA mindestens eine modifizierte Base enthält, die eine abasische Stelle bilden kann.

9. Verfahren nach Anspruch 8, **dadurch gekennzeichnet, dass** die modifizierte Base, die eine abasische Stelle bilden kann, aus 8-Brompurinderivaten ausgewählt ist.

10. Verfahren nach einem der Ansprüche 1 bis 4, **dadurch gekennzeichnet, dass** die abasische Stelle durch ein Alkylierungsmittel oder ein Oxidationsmittel erzeugt wird.

11. Verfahren nach einem der Ansprüche 1 bis 10, **dadurch gekennzeichnet, dass** das Markierungsreagens als reaktionsfähige Funktion eine Einheit enthält, die aus den folgenden Verbindungen ausgewählt ist: Diazomethyl; Alkylhalogenid; Nitrosoharnstoff; Spirocyclopropan; Aziridin; Epoxid; Trifluorsulfonaten.

12. Verfahren nach Anspruch 11, **dadurch gekennzeichnet, dass** das Markierungsreagens 5-(Brommethyl)fluorescein ist.

13. Verfahren nach Anspruch 11, **dadurch gekennzeichnet, dass** das Markierungsreagens aus Verbindungen der Formel (1): 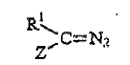 worin:
• R¹ für H oder eine Alkyl-, substituierte Alkyl-, Aryl- oder substituierte Arylgruppe steht und
• Z einen nachweisbaren Marker umfasst,
ausgewählt ist.

14. Verfahren nach Anspruch 13, **dadurch gekennzeichnet, dass** Z Folgendes ist:

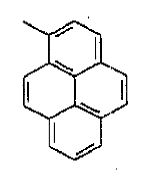

15. Verfahren nach Anspruch 14, **dadurch gekennzeichnet, dass** das Markierungsreagens ausgewählt ist aus Verbindungen der Formel (2): 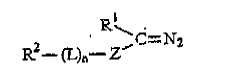 worin:
• R¹ für H oder eine Alkyl-, Aryl- oder substituierte Arylgruppe steht,
• R² ein nachweisbarer Marker ist,
• L ein Bindungsarm ist, der eine gerade Aneinanderreihung von mindestens zwei kovalenten Bindungen enthält, und n gleich 0 oder 1 ist und
• Z ausgewählt ist aus: 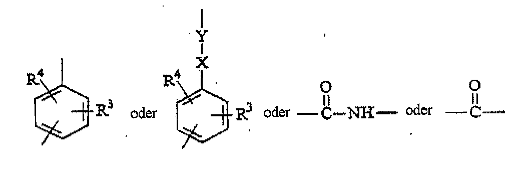 worin:
■ R³ und R⁴ unabhängig voneinander für; H, NO₂, Cl, Br, F, I, OR, SR, NR₂, R, NHCOR, CONHR, COOR mit R = Alkyl oder Aryl stehen und
■ -Y-X- für -CONH-, -NHCO-, -CH₂O-, CH₂S- steht.

16. Verfahren nach Anspruch 15, **dadurch gekennzeichnet, dass** das Markierungreagens die Formel (3) hat: 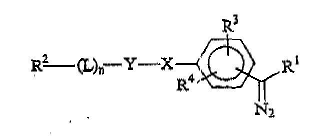 worin:
• R¹ für H oder eine Alkyl-, Aryl- oder substituierte Arylgruppe steht,
• R² einen nachweisbaren Marker darstellt,
• L ein Bindungsarm ist, der eine gerade Aneinanderreihung von mindestens 2 kovalenten Bindungen enthält, und n eine ganze Zahl gleich 0 oder 1 ist,
• R³ und R⁴ unabhängig voneinander für: H, NO₂, Cl, Br, F, I, OR, SR, NR₂, R, NHCOR, CONHR, COOR mit R = Alkyl oder Aryl stehen und
• -Y-X- für -CONH-, -NHCO-, -CH₂O-, CH₂S- steht.

17. Verfahren nach einem der Ansprüche 15 oder 16, **dadurch gekennzeichnet, dass** R² eine fluoreszierende Verbindung oder ein Hapten ist.

18. Verfahren nach einem der Ansprüche 11 bis 17, **dadurch gekennzeichnet, dass** das Markierungsreagens in einem mit Wasser mischbaren Lösungsmittel löslich ist.

19. Verfahren zum Nachweis einer einzel- oder doppelsträngigen Ziel-Desoxyribonukleinsäure (DNA), umfassend die folgenden Schritte:
- Fragmentieren und Markieren der DNAs durch eines der Verfahren der Ansprüche 1 bis 18,
- Hybridisieren der markierten Fragmente mit mindestens einer für die Zielnukleinsäure ausreichend spezifischen Nukleinsonde und
- Nachweisen des gebildeten Hybrids mithilfe eines Markers.

20. Verfahren zum Nachweis einer doppelsträngigen Ziel-Desoxyribonukleinsäure (DNA) nach Anspruch 19, das ferner einen Denaturierungsschritt nach der Fragmentierung und Markierung enthält.

21. Verfahren nach Anspruch 20, **dadurch gekennzeichnet, dass** Fragmentierung, Markierung und Denaturierung in einem einzigen Schritt durchgeführt werden.

22. Verfahren zum Nachweis einer Zielnukleinsäure, umfassend die folgenden Schritte:
- enzymatische Amplifizierung der Zielnukleinsäure, um eine Vielzahl an doppelsträngigen DNA-Amplikons zu erzeugen,
- Fragmentieren und Markieren der DNA-Amplikons nach einem der Verfahren der Ansprüche 1 bis 18,
- Hybridisieren der markierten Fragmente mit mindestens einer für die Zielnukleinsäure ausreichend spezifischen Nukleinsonde und
- Nachweisen des gebildeten Hybrids mithilfe eines Markers.

23. Verfahren nach Anspruch 22, das ferner einen Denaturierungsschritt nach dem Fragmentierungs- und Markierungsschritt enthält.

24. Verfahren nach Anspruch 23, **dadurch gekennzeichnet, dass** Fragmentierung, Markierung und Denaturierung in einem einzigen Schritt durchgeführt werden.

25. Verfahren zum Nachweis eines an zuvor bestimmten oder nicht bestimmten Positionen einer Zielnukleinsäure vorliegenden Polymorphismus durch Vorhandensein einer Mehrzahl an Deletionen und/oder Insertionen und/oder Mutationen in der Sequenz der Zielnukleinsäure im Vergleich zu einer so genannten Bezugssequenz, umfassend die folgenden Schritte:
• Gewinnen einer Ziel-DNA mit der Gesamtheit des zu untersuchenden Polymorphismus, wobei die DNA gegebenenfalls durch eine enzymatische Amplifikationstechnik erzeugt wird;
• Fragmentieren und Markieren der DNA durch ein Verfahren nach einem der Ansprüche 1 bis 18;
• Hybridisieren der Fragmente mit einer Mehrzahl als Fangsonden bezeichneter Nukleinsonden, wobei die Mehrzahl Fangsonden an einem festen Träger fixiert ist und die Mehrzahl Fangsonden in ihrer Gesamtheit mindestens den zu untersuchenden Polymorphismus abdeckt;
• Nachweisen der zwischen den markierten Fragmenten und mindestens einem Teil der Nukleinsonden gebildeten Hybride mithilfe eines Markers und Rückschließen auf den Polymorphismus der Ziel-DNA.

26. Verfahren nach Anspruch 25, das ferner einen Denaturierungsschritt nach dem Fragmentierungs- und Markierungsschritt enthält.

27. Verfahren nach Anspruch 26, **dadurch gekennzeichnet, dass** Fragmentierung, Markierung und Denaturierung in einem einzigen Schritt durchgeführt werden.

28. Verfahren nach einem der Ansprüche 25 bis 27, **dadurch gekennzeichnet, dass** der feste Träger mindestens zehn (10), vorteilhafterweise mindestens vierhundert (400), vorzugsweise mindestens tausend (1000), Nukleinsonden mit unterschiedlichen Sequenzen trägt.
